(19) Europäisches Patentamt
European Patent Office
Office européen des brevets

(11) EP 1 562 907 B1

# (12) EUROPEAN PATENT SPECIFICATION

(45) Date of publication and mention of the grant of the patent: **15.11.2006 Bulletin 2006/46**

(21) Application number: **03798193.3**

(22) Date of filing: **25.09.2003**

(51) Int Cl.:
*C07D 213/82* (2006.01) *C07D 401/12* (2006.01)
*C07D 413/12* (2006.01) *A61K 31/44* (2006.01)
*A61K 31/444* (2006.01) *A61K 31/4439* (2006.01)
*A61P 29/00* (2006.01)

(86) International application number:
**PCT/EP2003/010935**

(87) International publication number:
**WO 2004/029027 (08.04.2004 Gazette 2004/15)**

## (54) PYRIDINE DERIVATIVES AS CB2 RECEPTOR MODULATORS

PYRIDINDERIVATE ALS MODULATOREN DES CB2-REZEPTORS

DERIVES DE PYRIDINE EN TANT QUE MODULATEURS DU RECEPTEUR CB2

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **27.09.2002 GB 0222495**

(43) Date of publication of application:
**17.08.2005 Bulletin 2005/33**

(73) Proprietor: **GLAXO GROUP LIMITED**
**Greenford, Middlesex UB6 ONN (GB)**

(72) Inventors:
- **EATHERTON, Andrew John**
  **Harlow, Essex CM19 5AW (GB)**
- **GIBLIN, Gerard Martin Paul**
  **Harlow, Essex CM19 5AW (GB)**
- **GREEN, Richard, Howard deceased**
  **(GB)**
- **JANDU, Karamjit Singh**
  **Harlow, Essex CM19 5AW (GB)**
- **MITCHELL, William Leonard**
  **Harlow, Essex CM19 5AW (GB)**
- **NAYLOR, Alan**
  **Harlow, Essex CM19 5AW (GB)**
- **PALOMBI, Giovanni,**
  **C/o NiKem Research S.r.L.**
  **I-20021 Milan (IT)**
- **RAWLINGS, Derek Anthony**
  **Harlow, Essex CM19 5AW (GB)**
- **SLINGSBY, Brian Peter**
  **Harlow, Essex CM19 5AW (GB)**
- **WHITTINGTON, Andrew Richard**
  **Stevenage, Hertfordshire SG1 2NY (GB)**

(56) References cited:
**WO-A-02/062750**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 562 907 B1

## Description

The present invention relates to novel pyridine derivatives, pharmaceutical compositions containing these compounds and their use in the treatment of diseases, particularly pain, which diseases are caused directly or indirectly by an increase or decrease in activity of the cannabinoid receptor.

Cannabinoids are a specific class of psychoactive compounds present in Indian cannabis *(Cannabis sativa),* including about sixty different molecules, the most representative being cannabinol, cannabidiol and several isomers of tetrahydrocannabinol. Knowledge of the therapeutic activity of cannabis dates back to the ancient dynasties of China, where, 5,000 years ago, cannabis was used for the treatment of asthma, migraine and some gynaecological disorders. These uses later became so established that, around 1850, cannabis extracts were included in the US Pharmacopaeia and remained there until 1947.

Cannabinoids are known to cause different effects on various systems and/or organs, the most important being on the central nervous system and on the cardiovascular system. These effects include alterations in memory and cognition, euphoria, and sedation. Cannabinoids also increase heart rate and vary systemic arterial pressure. Peripheral effects related to bronchial constriction, immunomodulation, and inflammation have also been observed. The capability of cannabinoids to reduce intraocular pressure and to affect respiratory and endocrine systems is also well documented. See e.g. L.E. Hollister, Health Aspects of Cannabis, Pharmacological Reviews, Vol. 38, pp. 1-20, (1986). More recently, it was found that cannabinoids suppress the cellular and humoral immune responses and exhibit antiinflammatory properties. Wirth et al., Antiinflammatory Properties of Cannabichrome, Life Science, Vol. 26, pp. 1991-1995, (1980).

In spite of the foregoing benefits, the therapeutic use of cannabis is controversial, both due to its relevant psychoactive effects (causing dependence and addiction), and due to manifold side effects that have not yet been completely clarified. Although work in this field has been ongoing since the 1940's, evidence indicating that the peripheral effects of cannabinoids are directly mediated, and not secondary to a CNS effect, has been limited by the lack of receptor characterization, the lack of information concerning an endogenous cannabinoid ligand and, until recently, the lack of receptor subtype selective compounds.

The first cannabinoid receptor was found to be mainly located in the brain, in neural cell lines, and, only to a lesser extent, at the peripheral level. In view of its location, it was called the central receptor ("CB1"). See Matsuda et al., "Structure of a Cannabinoid Receptor and Functional Expression of the Cloned cDNA," Nature, Vol. 346, pp. 561-564 (1990. The second cannabinoid receptor ("CB2") was identified in the spleen, and was assumed to modulate the non psychoactive effects of the cannabinoids. See Munro et el., "Molecular Characterization of a Peripheral Receptor for Cannabinoids," Nature, Vol. 365, pp. 61-65 (1993).

Recently, some compounds have been prepared which are capable of acting as agonists on both the cannabinoid receptors. For example, use of derivatives of dihydroxypyrrole-(1,2,3-d,e)-1,4-benzoxazine in the treatment of glaucoma and the use of derivatives of 1,5-diphenyl-pyrazole as immunomodulators or psychotropic agents in the treatment of various neuropathologies, migraine, epilepsy, glaucoma, etc are known. See U.S. Patent No. 5,112,820 and EP 576357, respectively. However, because these compounds are active on both the CB1 and CB2 receptor, they can lead to serious psychoactive effects.

The foregoing indications and the preferential localization of the CB2 receptor in the immune system confirms a specific role of CB2 in modulating the immune and antiinflammatory response to stimuli of different sources.

The total size of the patient population suffering from pain is vast (almost 300 million), dominated by those suffering from back pain, osteo-arthritic pain and postoperative pain. Neuropathic pain (associated with neuronal lesions such as those induced by diabetes, HIV, herpes infection, or stroke) occurs with lower, but still substantial prevalence, as does cancer pain.

The pathogenic mechanisms that give rise to pain symptoms can be grouped into two main categories:

- those that are components of inflammatory tissue responses (Inflammatory Pain):
- those that result from a neuronal lesion of some form (Neuropathic Pain).

Chronic inflammatory pain consists predominantly of osteoarthritis, chronic low back pain and rheumatoid arthritis. The pain results from acute and on-going injury and/or inflammation. There may be both spontaneous and provoked pain.

There is an underlying pathological hypersensitivity as a result of physiological hyperexcitability and the release of inflammatory mediators which further potentiate this hyperexcitability. CB2 receptors are expressed on inflammatory cells (T cells, B cells, macrophages, mast cells) and mediate immune suppression through inhibition of cellular interaction/ inflammatory mediator release. CB2 receptors may also be expressed on sensory nerve terminals and therefore directly inhibit hyperalgesia.

The role of CB2 in immunomodulation, inflammation, osteoporosis, cardiovascular, renal and other disease conditions is now being examined. In light of the fact that cannabinoids act on receptors capable of modulating different

functional effects, and in view of the low homology between CB2 and CB1, the importance of developing a class of drugs selective for the specific receptor sub-type is evident. The natural or synthetic cannabinoids currently available do not fulfil this function because they are active on both receptors.

Based on the foregoing, there is a need for compounds which are capable of selectively modulating the receptor for cannabinoids and, therefore, the pathologies associated with such receptors. Thus, CB2 modulators offer a unique approach toward the pharmacotherapy of immune disorders, inflammation, osteoporosis, renal ischemia and other pathophysiological conditions.

International Patent Application WO 02/062750 (Schering Corp) describes compounds of the following formula as being cannabinoid receptor ligands exhibiting anti-inflammatory and/or immunomodulatory activity:

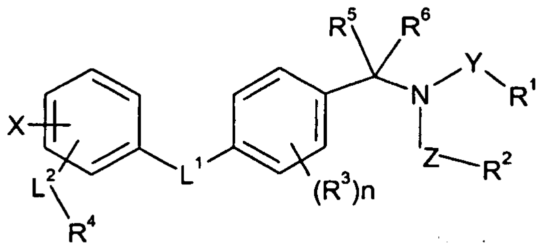

WO O2/062750

The present invention provides novel pyridine derivatives of formula (I) and pharmaceutically acceptable derivatives thereof, pharmaceutical compositions containing these compounds or derivatives, and their use as CB2 receptor modulators, which are useful in the treatment of a variety of disorders.

The present invention has application in the treatment of diseases mediated by CB2 receptors in an animal, including humans, which comprise administering to an animal in need thereof an effective amount of a compound of formula (I) or a pharmaceutically acceptable derivative thereof.

The invention provides compounds of formula (I):

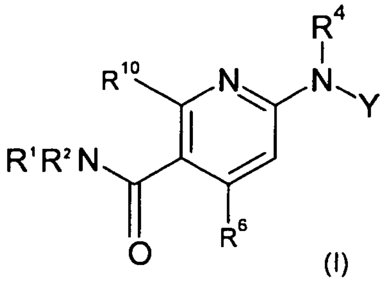

(I)

wherein:

Y is phenyl, substituted with one, two or three substituents;

$R^1$ is selected from hydrogen, $C_{1-6}$ alkyl, $C_{3-7}$ cycloalkyl, or halosubstituted$C_{1-6}$ alkyl;

$R^2$ is $(CH_2)mR^3$;

$R^3$ is an unsubstituted or substituted 5- to 6- membered aromatic heterocyclyl group, or group A:

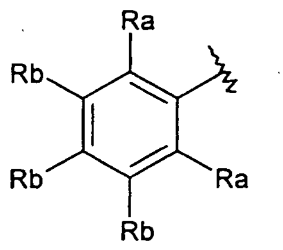

(A)

$R^4$ is selected from hydrogen, $C_{1-6}$ alkyl, $C_{3-7}$ cycloalkyl, or halosubstituted$C_{1-6}$ alkyl, $COCH_3$, and $SO_2Me$;
$R^6$ is unsubstituted or substituted $(C_{1-6})$alkyl or chloro and $R^{10}$ is hydrogen or $R^{10}$ is unsubstituted or substituted $(C_{1-6})$alkyl or chloro and $R^6$ is hydrogen;
Ra can be independently selected from hydrogen, fluoro, chloro or trifluoromethyl;
Rb can independently be selected from hydrogen, $C_{1-6}$ alkyl, $C_{1-6}$ alkoxy, halo substituted $C_{1-6}$ alkoxy, hydroxy, cyano, halo, sulfonyl, $CONH_2$, COOH, $SO_2CH_3$, $NHCOCH_3$, $NHSO_2CH_3$ and $CONHCH_3$;
m is 1 or 2;
and pharmaceutically acceptable derivatives thereof.

In one particular embodiment Y is substituted by 1 or 2 substituents. If monosubstituted, in one particular embodiment, the substituent is in the 3 position.

Substituents for Y are selected from: $C_{1-6}$ alkyl, halosubstituted$C_{1-6}$ alkyl, $C_{1-6}$ alkoxy, hydroxy, cyano, halo, $C_{1-6}$alkylsulfonyl, and COOH. Additional substituents can be selected from halosubstituted$C_{1-6}$ alkoxy, $CONH_2$, $NHCOCH_3$, $C_{1-6}$alkynyl, $C_{1-6}$alkyenyl $SO_2NR^{8a}R^{8b}$ wherein $R^{8a}$ and $R^{8b}$ are independently selected from H and $C_{1-6}$alkyl.

In one particular embodiment Y is substituted by halo, cyano, methoxy, methyl, trifluoromethyl or trifluoromethoxy.

In one particular embodiment $R^2$ is $CH_2R^3$.

A further aspect of the invention are compounds of formula (Ia):

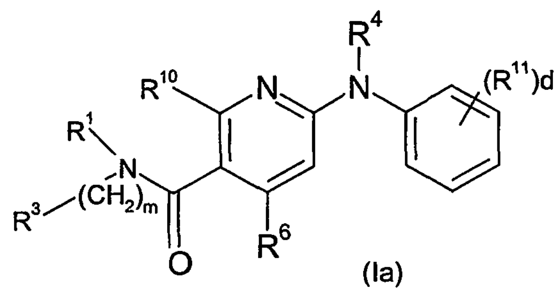

(Ia)

wherein:

$R^1$ is selected from hydrogen, $C_{1-6}$ alkyl, $C_{3-7}$ cycloalkyl, or halosubstituted$C_{1-6}$ alkyl;
$R^3$ is furanyl, dioxalanyl, pyrrolyl, oxazolyl, thiazolyl, imidazolyl, oxadiazolyl, thiadiazolyl, triazolyl, triazinyl, isothiazolyl, isoxazolyl, thienyl, pyrazolyl, tetrazolyl, pyridyl, pyrizinyl, pyrimidinyl, pyrazinyl, triazinyl, or tetrazinyl which can be unsubstituted or substituted with 1, 2 or 3 substitutents selected from $C_{1-6}$ alkyl, $C_{1-6}$ alkoxy, halosubstituted$C_{1-6}$ alkoxy, halosubstituted$C_{1-6}$ alkyl, hydroxy, cyano, halo, sulfonyl, $CONH_2$ and COOH, or $R^3$ is group A:

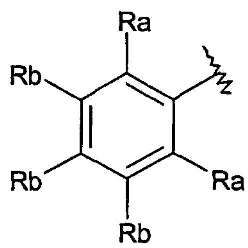

(A)

$R^4$ is selected from hydrogen, $C_{1-6}$ alkyl, $C_{3-7}$ cycloalkyl, or halosubstituted$C_{1-6}$ alkyl, $COCH_3$, and $SO_2Me$;
$R^6$ is unsubstituted or substituted ($C_{1-6}$)alkyl, chloro and $R^{10}$ is hydrogen or $R^{10}$ is unsubstituted or substituted ($C_{1-6}$) alkyl or chloro and $R^6$ is hydrogen;
Ra can be independently selected from hydrogen, fluoro, chloro or trifluoromethyl;
Rb can independently be selected from hydrogen, $C_{1-6}$ alkyl, $C_{1-6}$ alkoxy, halosubstituted$C_{1-6}$ alkoxy, hydroxy, cyano, halo, sulfonyl, $CONH_2$, COOH, $SO_2CH_3$, $NHCOCH_3$, $NHSO_2CH_3$ and $CONHCH_3$;
$R^{11}$ is $C_{1-6}$ alkyl, halosubstituted$C_{1-6}$ alkyl, $C_{1-6}$ alkoxy, hydroxy, cyano, halo, $C_{1-6}$alkylsulfonyl, $CONH_2$, $NHCOCH_3$, COOH, halosubstituted$C_{1-6}$ alkoxy, $C_{1-6}$alkynyl, $C_{1-6}$alkynyl, $SO_2NR^{8a}R^{8b}$;
d is 1,2,or3:
m is 1 or 2;
$R^{8a}$ and $R^{8b}$ are independently selected from hydrogen or $C_{1-6}$alkyl;
and pharmaceutically acceptable derivatives thereof.

In one particular embodiment $R^1$ is hydrogen or $C_{1-6}$alkyl, more particularly hydrogen.

In one particular embodiment $R^4$ is hydrogen or methyl, more particularly hydrogen.

In one particular embodiment $R^3$ is pyridinyl, pyrimidinyl, imidazoyl, oxadiazoyl, triazolyl or pyrazinyl any of which can be unsubstituted or substituted or is group A, In one particular embodiment $R^3$ is group A, pyridinyl or pyrimidinyl. In a further particular embodiment $R^3$ is group A or pyridinyl

When $R^3$ is a substituted 5- to 6- membered aromatic heterocyclyl group, the substituent or substituents is/are preferably selected from: $C_{1-6}$ alkyl, $C_{1-6}$ alkoxy, halosubstituted $C_{1-6}$ alkoxy, hydroxy, cyano, halo, sulfonyl, $CONH_2$, and COOH. Preferably the halo is fluoro.

In one particular embodiment when $R^3$ is an 5- to 6- membered aromatic heterocyclyl group the substituents are halo, methoxy, and cyano.

When $R^6$ or $R^{10}$ are substituted alkyl groups, they can be substituted with 1, 2 or 3 substitutents selected from hydroxy, $C_{1-6}$alkyoxy, cyano, halo, $NR^{8a}$ $R^{8b}$, $CONR^{8a}R^{8b}$, $SO_2NR^{8a}R^{8b}$, $NR^{8a}COR^{8b}$ or $NR^{8a}$ $SO_2R^{8b}$, preferably hydroxy or fluorine.

In one particular embodiment $R^6$ is a substituted or unsubstituted ($C_{1-6}$)alkyl, chloro or CHxFn wherein n is 1, 2, or 3, x is 0, 1 or 2 and n and x add up to 3 and $R^{10}$ is hydrogen or $R^{10}$ is a substituted or unsubstituted ($C_{1-6}$)alkyl, chloro or CHxFn wherein n is 1, 2, or 3, x is 0, 1 or 2 and n and x add up to 3 and $R^6$ is hydrogen

In one particular embodiment $R^6$ is *t*-butyl, isopropyl or CHxFn, more preferably $R^6$ is isopropyl or CHxFn even more preferably isopropyl or $CF_3$ and $R^{10}$ is hydrogen or $R^{10}$ is *t*-butyl, isopropyl or CHxFn, more preferably $R^{10}$ is isopropyl or CHxFn, more preferably isopropyl or $CF_3$ and $R^6$ is hydrogen

Alternatively Rb can independently be selected from hydrogen, $C_{1-6}$ alkyl, $C_{1-6}$ alkoxy, halo substituted $C_{1-6}$ alkoxy, hydroxy, cyano, halo, sulfonyl, $CONH_2$ and COOH.

In one particular embodiment Rb is selected from halo, methoxy, and cyano.

In one particular embodiment $R^6$ is ($C_{1-6}$)alkyl, chloro or CHxFn wherein n is 1, 2, or 3, x is 0, 1 or 2 and n and x add up to 3 and $R^{10}$ is hydrogen

Alternatively compounds of formula (I) are compounds of formula (Ib)

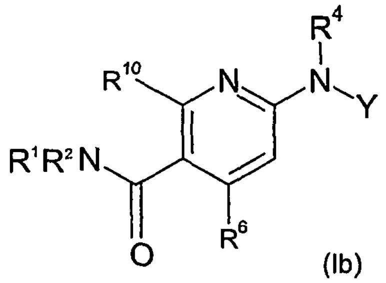

(Ib)

wherein:

Y is phenyl, substituted with one, two or three substituents;
$R^1$ is selected from hydrogen, $C_{1-6}$ alkyl, $C_{3-7}$ cycloalkyl, or halosubstituted$C_{1-6}$ alkyl;
$R^2$ is $CH_2R^3$;
$R^3$ is an optionally substituted 5- to 6- membered aromatic heterocyclyl group, or group A:

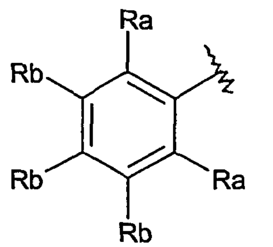

(A)

$R^4$ is selected from hydrogen, $C_{1-6}$ alkyl, $C_{3-7}$ cycloalkyl, or halosubstituted$C_{1-6}$ alkyl, $COCH_3$, or $SO_2Me$;
$R^6$ is $(C_{1-6})$alkyl, chloro or CHxFn wherein n is 1, 2, or 3, x is 0, 1 or 2 and n and x add up to 3 and $R^{10}$ is hydrogen or $R^{10}$ is $(C_{1-6})$alkyl, chloro or CHxFn wherein n is 1, 2, or 3, x is 0, 1 or 2 and n and x add up to 3 and $R^6$ is hydrogen;
Ra can be independently selected from hydrogen, fluoro, chloro or trifluoromethyl;
Rb can independently be selected from hydrogen, $C_{1-6}$ alkyl, $C_{1-6}$ alkoxy, halo$C_{1-6}$ alkoxy, a hydroxy group, a cyano group, halo, a sulfonyl group, $CONH_2$, or COOH;
and pharmaceutically acceptable derivatives thereof.

In one particular embodiment the compounds are selective for CB2 over CB1. Preferably the compounds are 100 fold selective i.e. compounds of formula (I) have an EC50 value at the cloned human cannabinoid CB2 receptor of at least 100 times the EC50 values at the cloned humna cannabinoid CB1 receptor or have less than 10% efficacy at the CB1 receptor.

The invention is described using the following definitions unless otherwise indicated.

The term "pharmaceutically acceptable derivative" means any pharmaceutically acceptable salt, ester, salt of such ester or solvate of the compounds of formula (I), or any other compound which upon administration to the recipient is capable of providing (directly or indirectly) a compound of formula (I) or an active metabolite or residue thereof.

It will be appreciated by those skilled in the art that compounds of formula (I) may be modified to provide pharmaceutically acceptable derivatives thereof at any of the functional groups in the compounds, and that the compounds of formula (I) may be derivatised at more than one position.

It will be appreciated that, for pharmaceutical use, the salts referred to above will be physiologically acceptable salts, but other salts may find use, for example in the preparation of compounds of formula (I) and the physiological acceptable salts thereof. Pharmaceutically acceptable salts include those described by Berge, Bighley and Monkhouse , J. Pharm. Sci., 1977, 66, 1-19. The term "pharmaceutically acceptable salts" includes salts prepared from pharmaceutically acceptable non-toxic bases including inorganic bases and organic bases. Salts derived from inorganic bases indude aluminum, ammonium, calcium, copper, ferric, ferrous, lithium, magnesium, manganic salts, manganous, potassium, sodium, zinc, and the like. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine,

diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropyl amine, tromethamine, and the like. When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like.

Preferred examples of pharmaceutically acceptable salts include the ammonium, calcium, magnesium, potassium, and sodium salts, and those formed from maleic, fumaric, benzoic, ascorbic, pamoic, succinic, hydrochloric, sulfuric, bismethylenesalicylic, methanesulfonic, ethanedisulfonic, propionic, tartaric, salicylic, citric, gluconic, aspartic, stearic, palmitic, itaconic, glycolic, p-aminobenzoic, glutamic, benzenesulfonic, cyclohexylsulfamic, phosphoric and nitric acids.

The terms 'halogen or halo' are used to represent fluorine, chlorine, bromine or iodine.

The term 'alkyl' as a group or part of a group means a straight or branched chain alkyl group or combinations thereof, for example a methyl, ethyl, n-propyl, i-propyl, n-butyl, s-butyl, t-butyl, pentyl, hexyl, 1,1-dimethylethyl, or combinations thereof.

The term 'alkoxy' as a group or as part of a group means a straight, branched or cyclic chain alkyl group having an oxygen atom attached to the chain, for example a methoxy, ethoxy, n-propoxy, i-propoxy, n-butoxy, s-butoxy, t-butoxy group, pentoxy, hexyloxy group, cyclopentoxy or cyclohexyloxy group.

The term 'cycloalkyl' means a closed 3- to 7- membered non-aromatic ring, for example cyclobutyl, cyclopentyl, cyclohexyl or cycloheptyl

The term 'cycloalkenyl' means a closed non-aromatic carbon ring containing 1 or more double bonds, for example cyclobutenyl, cyclopentenyl, cyclohexenyl or cycloheptenyl, or cyclooctenyl.

The term 'alkynyl' as a group or part of a group means a straight or branched chain carbon chain or combinations containing 1 or more triple carbon bonds for example a ethynyl, propynyl, butynyl, pentynyl, hexynyl or combinations thereof.

The term 'aryl' means a 5- or 6- membered aromatic ring, for example phenyl, or a 7-to 12- membered bicyclic ring system where at least one of the rings is aromatic, for example naphthyl.

When $R^3$ is an aromatic heterocyclyl group, the ring may contain 1, 2, 3, or 4 hetero atoms. In one particular embodiment the hetero atoms are selected from oxygen, nitrogen or sulphur. Examples of 5- membered heterocyclyl groups in this instance include furanyl, dioxalanyl, pyrrolyl, oxazolyl, thiazolyl, imidazolyl, oxadiazolyl, thiadiazolyl, triazolyl, triazinyl, isothiazolyl, isoxazolyl, thienyl, pyrazolyl or tetrazolyl. Examples of 6-membered heterocyclyl groups are pyridyl, pyrizinyl, pyrimidinyl, pyrazinyl, triazinyl, or tetrazinyl.

Preferred compounds of the present invention can be selected from:

6-(3-chloro-phenylamino)-N-(4-fluoro-benzyl)-4-isopropyl-nicotinamide;
N-benzyl-6-(3-chloro-phenylamino)-4-isopropyl-nicotinamide;
6-(3-chloro-phenylamino)-N-(4-cyano-benzyl)-4-isopropyl-nicotinamide;
6-(3-cyano-phenylamino)-N-(4-fluoro-benzyl)-4-isopropyl-nicotinamide;
6-(4-bromo-2-chloro-phenylamino)-N-(4-fluoro-benzyl)-4-isopropyl-nicotinamide;
6-(2,4-dichloro-phenylamino)-N-(4-fluoro-benzyl)-4-isopropyl-nicotinamide;
6-(2-bromo-4-chloro-phenylamino)-N-(4-fluoro-benzyl)-4-isopropyl-nicotinamide;
6-(2-chloro-4-fluoro-phenylamino)-N-(4-fluoro-benzyl)-4-isopropyl-nicotinamide;
6-(5-chloro-2-fluoro-phenylamino)-N-(4-fluoro-benzyl)-4-isopropyl-nicotinamide;
6-(4-cyano-2-methyl-phenylamino)-N-(4-fluoro-benzyl)-4-isopropyl-nicotinamide;
and pharmaceutically acceptable derivatives thereof.

Compounds of formula (I) can be prepared as set forth in the scheme 1:

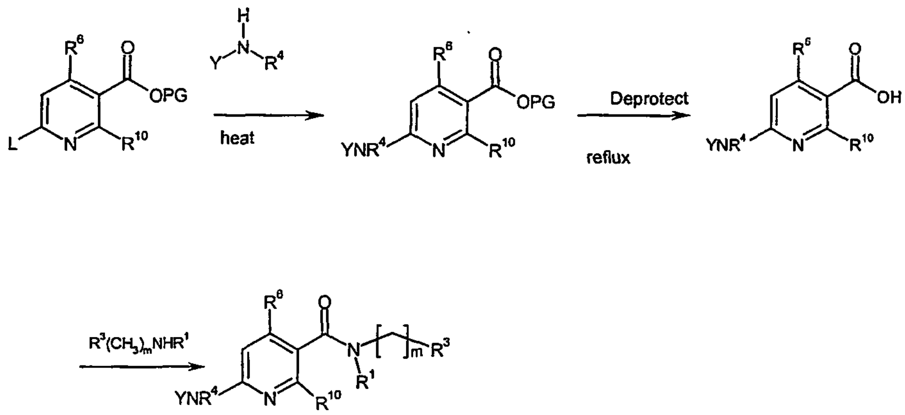


wherein $R^1$, $R^3$, $R^4$, $R^6$, Y , m and $R^{10}$ are as defined for compounds of formula (I), wherein L is a leaving group, for example halo, PG is a protecting group for example methyl, ethyl or benzyl.

Furthermore compounds of formula (I) when $R^{10}$ is unsubstituted or substituted $(C_{1-6})$alkyl or chloro and $R^6$ is hydrogen can be prepared as shown in scheme 2.

## Scheme 2

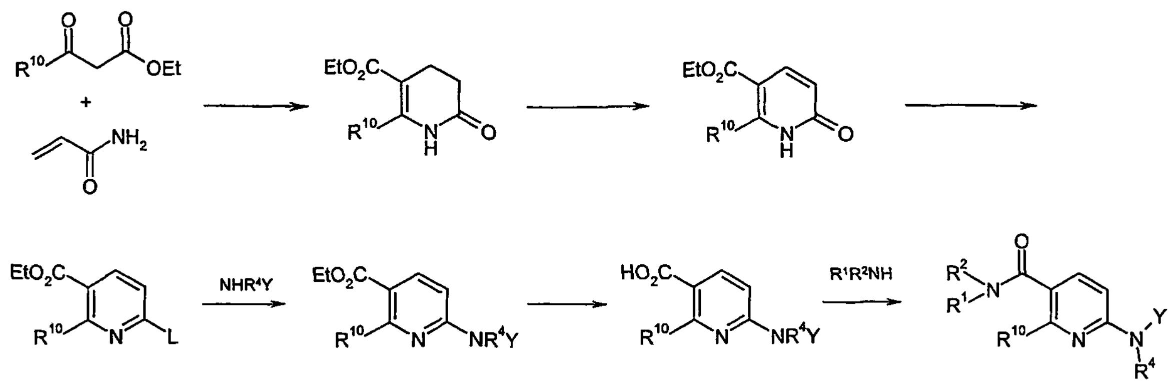


wherein L is a leaving group for example halogen, e.g. chloro, $R^1$, $R^2$, Y, $R^4$ are as defined for compounds of formula (I).

Furthermore compounds of formula (I) when $R^{10}$ is unsubstituted or substituted $(C_{1-6})$alkyl or chloro and $R^6$ is hydrogen can be prepared as shown in scheme 3.

**Scheme 3:**

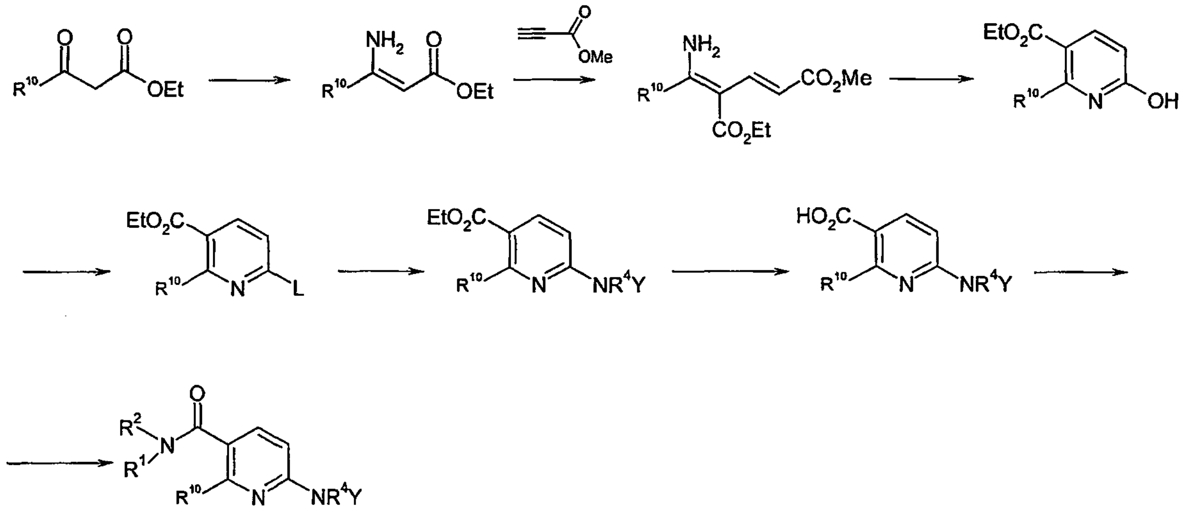

wherein L is a leaving group for example halogen, e.g. chloro, $R^1$, $R^2$, Y, $R^4$ are as defined for compounds of formula (I).

Furthermore compounds of formula (I) can be prepared as shown in scheme 4.

Scheme 4.

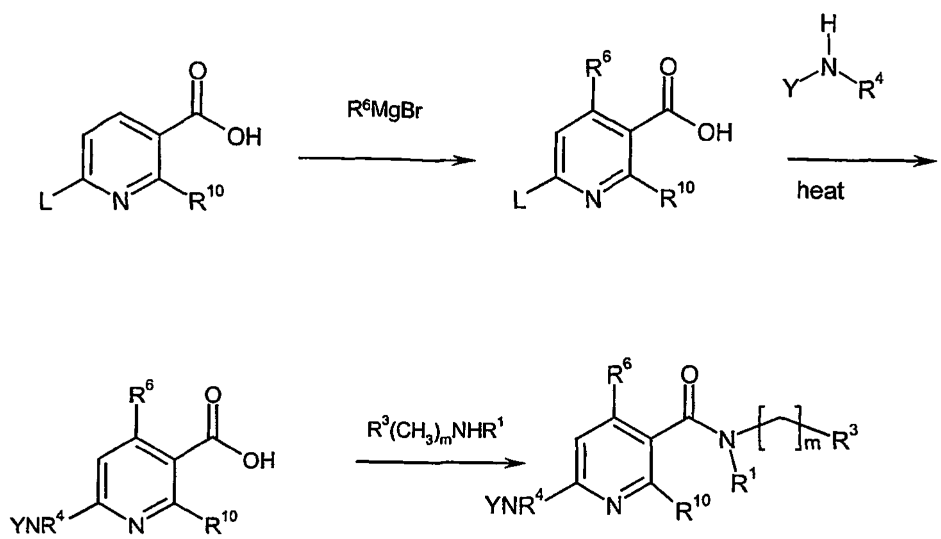


wherein L is a leaving group for example halogen, e.g. chloro, $R^1$, $R^3$, $R^4$, Y, $R^{10}$ and m are as defined for compounds of formula (I).

Furthermore compounds of formula (I) can be prepared as shown in scheme 4.

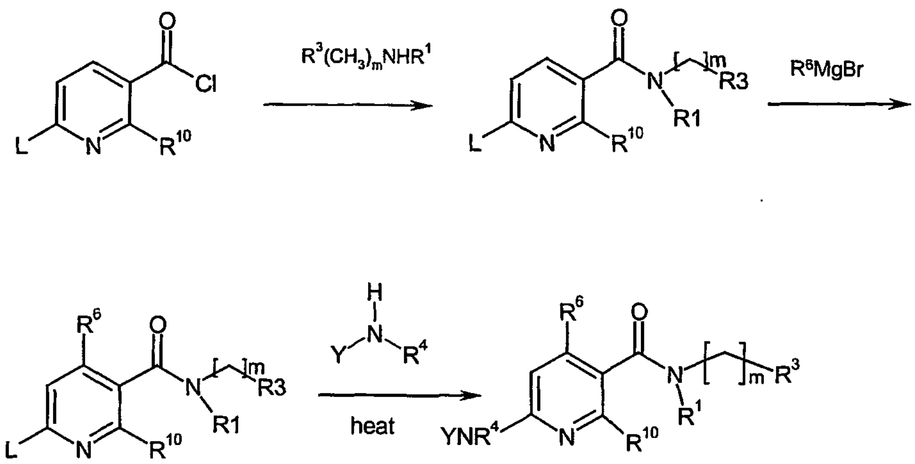


wherein L is a leaving group for example halogen, e.g. chloro, $R^1$, $R^3$, $R^4$, Y, $R^{10}$ and m are as defined for compounds of formula (I).

It is to be understood that the present invention encompasses all isomers of compounds of formula (I) and their pharmaceutically acceptable derivatives, including all geometric, tautomeric and optical forms, and mixtures thereof (e.g. racemic mixtures). Where additional chiral centres are present in compounds of formula (I), the present invention includes within its scope all possible diastereoisomers, including mixtures thereof. The different isomeric forms may be separated or resolved one from the other by conventional methods, or any given isomer may be obtained by conventional synthetic methods or by stereospecific or asymmetric syntheses.

The subject invention also includes isotopically-labeled compounds, which are identical to those recited in formulas I and following, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as $^{3}H$, $^{11}C$, $^{14}C$, $^{18}F$, $^{123}I$ and $^{125}I$.

Compounds of the present invention and pharmaceutically acceptable salts of said compounds that contain the aforementioned isotopes and/or other isotopes of other atoms are within the scope of the present invention. Isotopically-labeled compounds of the present invention, for example those into which radioactive isotopes such as $^{3}H$, $^{14}C$ are incorporated, are useful in drug and/or substrate tissue distribution assays. Tritiated, i.e., $^{3}H$, and carbon-14, i.e., $^{14}C$, isotopes are particularly preferred for their ease of preparation and detectability. $^{11}C$ and $^{8}F$ isotopes are particularly useful in PET (positron emission tomography), and $^{125}I$ isotopes are particularly useful in SPECT (single photon emission computerized tomography), all useful in brain imaging. Further, substitution with heavier isotopes such as deuterium, i.e., $^{2}H$, can afford certain therapeutic advantages resulting from greater metabolic stability, for example increased *in vivo* half-life or reduced dosage requirements and, hence, may be preferred in some circumstances. Isotopically labeled compounds of formula I and following of this invention can generally be prepared by carrying out the procedures disclosed in the Schemes and/or in the Examples below, by substituting a readily available isotopically labeled reagent for a non-isotopically labeled reagent.

The compounds of formula (I) may be prepared in crystalline or non-crystalline form, and, if crystalline, may optionally be hydrated or solvated. This invention includes within its scope stoichiometric hydrates or solvates as well as compounds containing variable amounts of water and/or solvent.

The compounds of the invention bind selectively to the CB2 receptor, and are therefore useful in treating CB2 receptor mediated diseases.

In view of their ability to bind to the CB2 receptor, the compounds of the invention may be useful in the treatment of the disorders that follow. Thus, the compounds of formula (I) may be useful as analgesics. For example they may be useful in the treatment of chronic inflammatory pain (e.g. pain associated with rheumatoid arthritis, osteo-arthritis, rheumatoid spondylitis, gouty arthritis and juvenile arthritis) including the property of disease modification and joint structure preservation; musculoskeletal pain; lower back and neck pain; sprains and strains; neuropathic pain; sympathetically maintained pain; myositis; pain associated with cancer and fibromyalgia; pain associated with migraine; pain associated with influenza or other viral infections, such as the common cold; rheumatic fever; pain associated with functional bowel disorders such as non-ulcer dyspepsia, non-cardiac chest pain and irritable bowel syndrome; pain associated with myocardial ischemia; post operative pain; headache; toothache; dysmenorrhea, chronic pain, dental pain algesia, pelvic pain, poststroke pain and menstrual pain.

The compounds of the invention may also be useful disease modification or joint structure preservation in multiple sclerosis, rheumatoid arthritis, osteo-arthritis, rheumatoid spondylitis, gouty arthritis and juvenile arthritis.

The compounds of the invention may be particularly useful in the treatment of neuropathic pain. Neuropathic pain syndromes can develop following neuronal injury and the resulting pain may persist for months or years, even after the original injury has healed. Neuronal injury may occur in the peripheral nerves, dorsal roots, spinal cord or certain regions in the brain. Neuropathic pain syndromes are traditionally classified according to the disease or event that precipitated them. Neuropathic pain syndromes include: diabetic neuropathy; sciatica; non-specific lower back pain; multiple sclerosis pain; fibromyalgia; HIV-related neuropathy; post-herpetic neuralgia; trigeminal neuralgia; and pain resulting from physical trauma, amputation, cancer, toxins or chronic inflammatory conditions. These conditions are difficult to treat and although several drugs are known to have limited efficacy, complete pain control is rarely achieved. The symptoms of neuropathic pain are incredibly heterogeneous and are often described as spontaneous shooting and lancinating pain, or ongoing, burning pain. In addition, there is pain associated with normally non-painful sensations such as "pins and needles" (paraesthesias and dysesthesias), increased sensitivity to touch (hyperesthesia), painful sensation following innocuous stimulation (dynamic, static or thermal allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

The compounds of formula (I) may also be useful in the treatment of fever.

The compounds of formula (I) may also be useful in the treatment of inflammation, for example in the treatment of skin conditions (e.g. sunburn, burns, eczema, dermatitis, psoriasis); ophthalmic diseases such as glaucoma, retinitis, retinopathies, uveitis and of acute injury to the eye tissue (e.g. conjunctivitis); lung disorders (e.g. asthma, bronchitis, emphysema, allergic rhinitis, respiratory distress syndrome, pigeon fancier's disease, farmer's lung, chronic obstructive pulmonary disease, (COPD); cough, gastrointestinal tract disorders (e.g. aphthous ulcer, Crohn's disease, atopic gastritis, gastritis varialoforme, ulcerative colitis, coeliac disease, regional ileitis, irritable bowel syndrome, inflammatory bowel disease, gastroesophageal reflux disease emesis, oesophagitis, organ transplantation; other conditions with an inflammatory component such as vascular disease, migraine, periarteritis nodosa, thyroiditis, aplastic anaemia, Hodgkin's disease, sclerodoma, myaesthenia gravis, multiple sclerosis, sorcoidosis, nephrotic syndrome, Bechet's syndrome, polymyositis, gingivitis, myocardial ischemia, pyrexia, systemic lupus erythematosus, tendinitis, bursitis, and Sjogren's syndrome.

The compounds of formula (I) may also be useful in the treatment of bladder hyperrelexia following bladder inflammation.

The compounds of formula (I) are also useful in the treatment of immunological diseases such as autoimmune diseases, immunological deficiency diseases or organ transplantation. The compounds of formula (I) are also effective in increasing the latency of HIV infection.

The compounds of formula (I) are also useful in the treatment of diseases of abnormal platelet function (e.g. occlusive vascular diseases).

The compounds of formula (I) are also useful in the treatment of neuritis, heart burn, dysphagia, pelvic hypersensitivity, urinary incontinence, cystitis or pruritis.

The compounds of formula (I) are also useful for the preparation of a drug with diuretic action.

The compounds of formula (I) are also useful in the treatment of impotence or erectile dysfunction.

The compounds of formula (I) are also useful for attenuating the hemodynamic side effects of non-steroidal anti-inflammatory drugs (NSAID's) and cyclooxygenase-2 (COX-2) inhibitors.

The compounds of formula (I) are also useful in the treatment of neurodegenerative diseases and neurodegeneration such as dementia, particularly degenerative dementia (including senile dementia, Alzheimer's disease, Pick's disease, Huntingdon's chorea, Parkinson's disease and Creutzfeldt-Jakob disease, motor neuron disease); vascular dementia (including multi-infarct dementia); as well as dementia associated with intracranial space occupying lesions; trauma; infections and related conditions (including HIV infection); dementia in Parkinson's disease; metabolism; toxins; anoxia and vitamin deficiency; and mild cognitive impairment associated with ageing, particularly Age Associated Memory Impairment. The compounds may also be useful for the treatment of amyotrophic lateral sclerosis (ALS) and neuroinflamation.

The compounds of formula (I) are also useful in neuroprotection and in the treatment of neurodegeneration following stroke, cardiac arrest, pulmonary bypass, traumatic brain injury, spinal cord injury or the like.

The compounds of formula (I) are also useful in the treatment of tinnitus.

The compounds of formula (I) are also useful in the treatment of psychiatric disease for example schizophrenia, depression (which term is used herein to include bipolar depression, unipolar depression, single or recurrent major depressive episodes with or without psychotic features, catatonic features, melancholic features, atypical features or postpartum onset, seasonal affective disorder, dysthymic disorders with early or late onset and with or without atypical features, neurotic depression and social phobia, depression accompanying dementia for example of the Alzheimer's type, schizoaffective disorder or the depressed type, and depressive disorders resulting from general medical conditions including, but not limited to, myocardial infarction, diabetes, miscarriage or abortion, *etc*), anxiety disorders (including generalised anxiety disorder and social anxiety disorder), panic disorder, agoraphobia, social phobia, obsessive compulsive disorder and post-traumatic stress disorder, memory disorders, including dementia, amnesic disorders and age-associated memory impairment, disorders of eating behaviours, including anorexia nervosa and bulimia nervosa, sexual dysfunction, sleep disorders (including disturbances of circadian rhythm, dyssomnia, insomnia, sleep apnea and narcolepsy), withdrawal from abuse of drugs such as of cocaine, ethanol, nicotine, benzodiazepines, alcohol, caffeine, phencyclidine (phencyclidine-like compounds), opiates (e.g. cannabis, heroin, morphine), amphetamine or amphetamine-related drugs (e.g. dextroamphetamine, methylamphetamine) or a combination thereof.

The compounds of formula (I) are also useful in preventing or reducing dependence on, or preventing or reducing tolerance or reverse tolerance to, a dependence - inducing agent. Examples of dependence inducing agents include opioids (e.g. morphine), CNS depressants (e.g. ethanol), psychostimulants (e.g. cocaine) and nicotine.

The compounds of formula (I) are also useful in the treatment of kidney dysfunction (nephritis, particularly mesangial proliferative glomerulonephritis, nephritic syndrome), liver dysfunction (hepatitis, cirrhosis), gastrointestinal dysfunction (diarrhoea) and colon cancer.

The compounds of formula (I) may useful for the treatment of bladder hyper-reflexia following bladder inflammation.

It is to be understood that references to treatment includes both treatment of established symptoms and prophylactic treatment unless explicitly stated otherwise.

According to a further aspect of the invention, we provide a compound of formula (I) or a pharmaceutically acceptable derivative thereof for use in human or veterinary medicine.

According to another aspect of the invention, we provide a compound of formula (I) or a pharmaceutically acceptable derivative thereof for use in the treatment of a condition which is mediated by the activity of cannabinoid 2 receptors.

The compounds of the present invention can be used in a method of treating a human or animal subject suffering from a condition which is mediated by the activity of cannabinoid 2 receptors which comprises administering to said subject a therapeutically effective amount of a compound of formula (I) or a pharmaceutically acceptable derivative thereof.

According to another aspect of the invention is provided the use of a compound of formula (I) or a pharmaceutically acceptable derivative thereof for the manufacture of a therapeutic agent for the treatment or prevention of a condition such as an immune disorder, an inflammatory disorder, pain, rheumatoid arthritis, multiple sclerosis, osteoarthritis or osteoporosis

Preferably the pain is selected from inflammatory pain, viseral pain, cancer pain, neuropathic pain, lower back pain, muscular sceletal, post operative pain, acute pain and migraine. More preferably the inflammatory pain is pain associated with rheumatoid arthritis or osteoarthritis.

In order to use a compound of formula (I) or a pharmaceutically acceptable derivative thereof for the treatment of humans and other mammals it is normally formulated in accordance with standard pharmaceutical practice as a pharmaceutical composition. Therefore in another aspect of the invention is provided a pharmaceutical composition comprising a compound of formula (I) or a pharmaceutically acceptable derivative thereof adapted for use in human or veterinary medicine.

As used herein, "modulator" means both antagonist, full or partial agonist and inverse agonist. In one embodiment of the present modulators are agonists.

The term "treatment" or "treating" as used herein includes the treatment of established disorders and also includes the prophylaxis thereof. The term "prophylaxis" is used herein to mean preventing symptoms in an already afflicted subject or preventing recurrence of symptoms in an afflicted subject and is not limited to complete prevention of an affliction.

Compounds of formula (1) and their pharmaceutically acceptable derivatives may be administered in a standard manner for the treatment of the indicated diseases, for example orally, parentarally, sub-lingually, dermally, intranasally, transdermally, rectally, via inhalation or via buccal administration.

Compositions of formula (I) and their pharmaceutically acceptable derivatives which are active when given orally can be formulated as syrups, tablets, capsules and lozenges. A syrup formulation will generally consist of a suspension or solution of the compound or salt in a liquid carrier for example, ethanol, peanut oil, olive oil, glycerine or water with a flavouring or colouring agent. Where the composition is in the form of a tablet, any pharmaceutical carrier routinely used for preparing solid formulations may be used. Examples of such carriers include magnesium stearate, terra alba, talc, gelatin, acacia, stearic acid, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils, and are incorporated in a soft gelatin capsule shell.

Typical parenteral compositions consist of a solution or suspension of a compound or derivative in a sterile aqueous or non-aqueous carrier optionally containing a parenterally acceptable oil, for example polyethylene glycol,

polyvinylpyrrolidone, lecithin, arachis oil or sesame oil.

Typical compositions for inhalation are in the form of a solution, suspension or emulsion that may be administered as a dry powder or in the form of an aerosol using a conventional propellant such as dichlorodifluoromethane or trichlorofluoromethane.

A typical suppository formulation comprises a compound of formula (I) or a pharmaceutically acceptable derivative thereof which is active when administered in this way, with a binding and/or lubricating agent, for example polymeric glycols, gelatins, cocoa-butter or other low melting vegetable waxes or fats or their synthetic analogs.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

Each dosage unit for oral administration contains suitably from 0.01 mg/Kg to 500 mg/Kg for example 0.1 mg to 500 mg/Kg, and preferably from 0.01 mg to 100 mg/Kg for example 1 mg/Kg to 100mg/Kg, and each dosage unit for parenteral administration contains suitably from 0.1 mg to 100 mg/Kg, of a compound of formula (I) or a pharmaceutically acceptable derivative thereof calculated as the free acid. Each dosage unit for intranasal administration contains suitably 1-400 mg and preferably 10 to 200 mg per person. A topical formulation contains suitably 0.01 to 5.0% of a compound of formula (I).

The daily dosage regimen for oral administration is suitably about 0.01 mg/Kg to 40 mg/Kg, of a compound of formula (I) or a pharmaceutically acceptable derivative thereof calculated as the free acid. The daily dosage regimen for parenteral administration is suitably about 0.001 mg/Kg to 40 mg/Kg, of a compound of formula (I) or a pharmaceutically acceptable derivative thereof calculated as the free acid. The daily dosage regimen for intranasal administration and oral inhalation is suitably about 10 to about 500 mg/person. The active ingredient may be administered from 1 to 6 times a day, sufficient to exhibit the desired activity.

It may be advantageous to prepare the compounds of the present invention as nanoparticles. This may improve the oral bioavailability of the compounds. For the purposes of the present invention "nanoparticulate" is defined as solid particles with 50% of the particles having a particle size of less than 1$\mu$m, more preferably less than 0.75$\mu$m

The particle size of the solid particles of compound (I) may be determined by laser diffraction. A suitable machine for determining particle size by laser diffraction is a Lecotrac laser particle size analyser, using an HELOS optical bench fitted with a QUIXEL dispersion unit.

Numerous processes for the synthesis of solid particles in nanoparticulate form are known. Typically these processes involve a milling process, preferably a wet milling process in the presence of a surface modifying agent that inhibits aggregation and/or crystal growth of the nanoparticles once created. Alternatively these processes may involve a precipitation process, preferably a process of precipitation in an aqueous medium from a solution of the drug in a non-aqueous solvent.

Representative processes for the preparation of solid particles in nanoparticulate form are described in the patents and publications listed below.

U.S. Patent No. 4,826,689 to Violanto & Fischer, U. S. Patent No. 5,145,684 to Liversidge et al

U.S Patent No. 5,298,262 to Na & Rajagopalan, U.S. Patent No. 5,302,401 Liversidge et al

U.S. Patent No. 5,336,507 to Na & Rajagopalan, U.S. Patent No. 5,340,564 to Illig & Sarpotdar

U.S. Patent No. 5,346,702 to Na Rajagopalan, U.S. Patent No. 5,352,459 to Hollister et al

U.S. Patent No. 5,354,560 to Lovrecich, U.S. Patent No. 5,384,124 to Courteille et al, U.S. Patent No. 5,429,824 to June, U.S. Patent No. 5,503,723 to Ruddy et al, U.S. Patent No. 5,510 118 to Bosch et al, U.S. Patent No. 5,518 to Bruno et al, U.S. Patent No. 5,518,738 to Eickhoff et al, U.S. Patent No. 5,534,270 to De Castro, U.S. Patent No. 5,536,508 to Canal et al, U.S. Patent No. 5,552,160 to Liversidge et al, U.S. Patent No. 5,560,931 to Eickhoff et al, U.S. Patent No. 5,560,932 to Bagchi et al, U.S. Patent No. 5,565,188 to Wong et al, U.S. Patent No. 5,571,536 to Eickhoff et al, U.S. Patent No. 5,573,783 t o Desieno & Stetsko, U.S Patent No. 5,580,579 to Ruddy et al, U.S. Patent No 5,585,108 to Ruddy et al, U.S. Patent No. 5,587,143 to Wong, U.S. Patent No. 5,591456 to Franson et al, U.S. Patent No. 5,622,938 to Wong, U.S. Patent No 5,662,883 to Bagchi et al, U.S. Patent No. 5,665,331 to Bagchi et al, U.S Patent No. 5,718,919 to Ruddy et al, U.S. Patent No. 5,747,001 to Wiedmann et al, WO93/25190, WO96/24336, WO 97/14407, WO 98/35666, WO 99/65469, WO 00/18374, WO 00/27369, WO 00/30615 and WO 01/41760.

Such processes may be readily adapted for the preparation of compound (I) in nanoparticulate form.

The process preferably uses a wet milling step carried out in a mill such as a dispersion mill in order to produce a nanoparticulate form of the compound. The processes may be put into practice using a conventional wet milling technique, such as that described in Lachman *et al.,* The Theory and Practice of Industrial Pharmacy, Chapter 2, "Milling" p.45 (1986).

In a further refinement, WO02/0019 (SmithKline Beecham plc) describes a wet milling procedure using a mill in which at least some of the surfaces are made of nylon (polyamide) comprising one or more internal lubricants, for use in the preparation of solid particles of a drug substance in nanoparticulate form.

The compounds of the invention may be prepared in nanoparticulate form by wet milling a suspension of compound in a mill having at least one chamber and agitation means, said chamber(s) and/or said agitation means comprising a lubricated nylon, as described in WO02/00196

The suspension of a compound of the invention for use in the wet milling is typically a liquid suspension of the coarse compound in a liquid medium. By "suspension" is meant that the compound is essentially insoluble in the liquid medium. Representative liquid media include an aqueous medium. Using the above described process the average particle size of coarse compound of the invention may be up to 1 mm in diameter. This advantageously avoids the need to pre-process the compound.

The aqueous medium to be subjected to the milling comprises compound (I) present in from about 1% to about 40% w/w, preferably from about 10% to about 30% w/w, more preferably about 20% w/w.

The aqueous medium may further comprise one or more pharmaceutically acceptable water-soluble carriers which are suitable for steric stabilisation and the subsequent processing of compound (I) after milling to a pharmaceutical composition, e.g. by spray drying. Pharmaceutically acceptable excipients most suitable for steric stabilisation and spray-drying are surfactants such as poloxamers, sodium lauryl sulphate and polysorbates etc; stabilisers such as celluloses e.g. hydroxypropylmethyl cellulose; and carriers such as carbohydrates e.g. mannitol.

The aqueous medium to be subjected to the milling may further comprise hydroxypropylmethyl cellulose (HPMC) present from about 0.1 to about 10% w/w.

The process may comprise the subsequent step of drying compound of the invention to yield a powder.

Accordingly, a suitable process for preparing a pharmaceutical composition containing a compound of the present invention comprises producing compound of formula (I) in nanoparticulate form optionally followed by drying to yield a powder. A further aspect of the invention is a pharmaceutical composition comprising a compound of formula (I) or a pharmaceutically acceptable derivative thereof in which the compound of formula (I) or a pharmaceutically acceptable derivative thereof is present in solid particles in nanoparticulate form, in admixture with one or more pharmaceutically acceptable carriers or excipients.

By "drying" is meant the removal of any water or other liquid vehicle used during the process to keep compound of formula (I) in liquid suspension or solution. This drying step may be any process for drying known in the art, including freeze drying, spray granulation or spray drying. Of these methods spray drying is particularly preferred. All of these techniques are well known in the art. Spray drying/fluid bed granulation of milled compositions is carried out most suitably using a spray dryer such as a Mobile Minor Spray Dryer [Niro, Denmark], or a fluid bed drier, such as those manufactured by Glatt, Germany.

In a further aspect the invention provides a pharmaceutical composition as hereinbefore defined, in the form of a dried powder, obtainable by wet milling solid particles of compound of formula (I) followed by spray-drying the resultant suspension.

Preferably, the pharmaceutical composition as hereinbefore defined, further comprises HPMC present in less than 15% w/w, preferably in the range 0.1 to 10% w/w.

The $CB_2$ receptor compounds for use in the instant invention may be used in combination with other therapeutic agents, for example COX-2 inhibitors, such as celecoxib, deracoxib, rofecoxib, valdecoxib, parecoxib or COX-189; 5-lipoxygenase inhibitors; NSAID's, such as aspirin, diclofenac, indomethacin, nabumetone or ibuprofen; leukotriene receptor antagonists; DMARD's such as methotrexate; adenosine A1 receptor agonists; sodium channel blockers, such as lamotrigine; NMDA receptor modulators, such as glycine receptor antagonists; gabapentin and related compounds; tricyclic antidepressants such as amitriptyline; neurone stabilising antiepileptic drugs; mono-aminergic uptake inhibitors such as venlafaxine; opioid analgesics; local anaesthetics; $5HT_1$ agonists, such as triptans, for example sumatriptan, naratriptan, zolmitriptan, eletriptan, frovatriptan, almotriptan or rizatriptan; $EP_1$ receptor ligands, $EP_4$ receptor ligands; $EP_2$ receptor ligands; $EP_3$ receptor ligands; $EP_4$ antagonists; $EP_2$ antagonists and $EP_3$ antagonists; bradykinin receptor ligands and vanilloid receptor ligand, antirheumatoid arthritis drugs, for example anti TNF drugs e.g. enbrel, remicade, anti-IL-1 drugs, or DMARDS e.g. leflunamide. When the compounds are used in combination with other therapeutic agents, the compounds may be administered either sequentially or simultaneously by any convenient route.

Additional COX-2 inhibitors are disclosed in US Patent Nos. 5,474,995 US5,633,272; US5,466,823, US6,310,099 and US6,291,523; and in WO 96/25405, WO 97/38986, WO 98/03484, WO 97/14691, WO99/12930, WO00/26216, WO00/52008, WO00/38311, WO01/58881 and WO02/18

The compound of the present invention may be administered in combination with other active substances such as 5HT3 antagonists, NK-1 antagonists, serotonin agonists, selective serotonin reuptake inhibitors (SSRI), noradrenaline re-uptake inhibitors (SNRI), tricyclic antidepressants and/or dopaminergic antidepressants.

Suitable 5HT3 antagonists which may be used in combination of the compound of the inventions include for example ondansetron, granisetron, metoclopramide.

Suitable serotonin agonists which may be used in combination with the compound of the invention include sumatriptan, rauwolscine, yohimbine, metoclopramide.

Suitable SSRIs which may be used in combination with the compound of the invention include fluoxetine,

citalopram, femoxetine, fluvoxamine, paroxetine, indalpine, sertraline, zimeldine.

Suitable SNRIs which may be used in combination with the compound of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

It will be appreciated that the compounds of any of the above combinations or compositions may be administered simultaneously (either in the same or different pharmaceutical formulations), separately or sequentially.

The invention thus provides, in a further aspect, a combination comprising a compound of formula (I) or a pharmaceutically acceptable derivative thereof together with a further therapeutic agent or agents.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

**Determination of cannabinoid CB1 Receptor Agonist Activity**

The cannabinoid CB1 receptor agonist activity of the compounds of formula (I) was determined in accordance with the following experimental method.

**Experimental Method**

Yeast *(Saccharomyces cerevisiae)* cells expressing the human cannabinoid CB1 receptor were generated by integration of an expression cassette into the ura3 chromosomal locus of yeast strain MMY23. This cassette consisted of DNA sequence encoding the human CB1 receptor flanked by the yeast GPD promoter to the 5' end of CB1 and a yeast transcriptional terminator sequence to the 3' end of CB1. MMY23 expresses a yeast/mammalian chimeric G-protein alpha subunit in which the C-terminal 5 amino acids of Gpa1 are replaced with the C-terminal 5 amino acids of human G$\alpha$i3 (as described in Brown *et al.* (2000), Yeast **16**:11-22). Cells were grown at 30°C in liquid Synthetic Complete (SC) yeast media (Guthrie and Fink (1991), Methods in Enzymology, Vol. 194) lacking uracil, tryptophan, adenine and leucine to late logarithmic phase (approximately 6 $OD_{600}$/ml).

Agonists were prepared as 10 mM stocks in DMSO. $EC_{50}$ values (the concentration required to produce 50% maximal response) were estimated using dilutions 5 of between 3- and 5-fold (BiomekFX, Beckman) into DMSO. Agonist solutions in DMSO (1% final assay volume) were transferred into black, clear bottom, microtitre plates from NUNC (96- or 384-well). Cells were suspended at a density of 0.2 $OD_{600}$/ml in SC media lacking histidine, uracil, tryptophan, adenine and leucine and supplemented with 10mM 3-aminotriazole, 0.1M sodium phosphate pH 7.0, and 20$\mu$M fluorescein di-$\beta$-D-glucopyranoside (FDGlu). This mixture (50ul per well for 384-well plates, 200ul per well for 96-well plates) was added to agonist in the assay plates (Multidrop 384, Labsystems). After incubation at 30°C for 24 hours, fluorescence resulting from degradation of FDGlu to fluorescein due to exoglucanase, an endogenous yeast enzyme produced during agonist-stimulated cell growth, was determined using a Spectrofluor microtitre plate reader (Tecan; excitation wavelength: 485nm; emission wavelength: 535nm). Fluorescence was plotted against compound concentration and iteratively curve fitted using a four parameter fit to generate a concentration effect value. Efficacy ($E_{max}$) was calculated from the equation

$$E_{max} = Max_{[compound\ X]} - Min_{[compound\ X]} / Max_{[HU210]} - Min_{[HU210]} \times 100\%$$

where $Max_{[compound\ X]}$ and $Min_{[compound\ X]}$ are the fitted maximum and minimum respectively from the concentration effect curve for compound X, and $Max_{[HU210]}$ and $Min_{[HU210]}$ are the fitted maximum and minimum respectively from the concentration effect curve for (6aR,10aR)-3-(1,1'-Dimethylheptyl)-6a,7,10,10a-tetrahydro-1-hydroxy-6,6-dimethyl-6H-dibenzo[b,d]pyran-9-methanol (HU210; available from Tocris). Equieffective molar ratio (EMR) values were calculated from the equation

$$EMR = EC_{50\ [compound\ X]} / EC_{50\ [HU210]}$$

Where $EC_{50\ [compound\ X]}$ is the $EC_{50}$ of compound X and $EC_{50\ [HU210]}$ is the $EC_{50}$ of HU210.

Compounds of the Examples tested according to this method had $EC_{50}$ values >30,000nM at the cloned human cannabinoid CB1 receptor.

**Determination of cannabinoid CB2 Receptor Agonist Activity**

The cannabinoid CB2 receptor agonist activity of the compounds of formula (I) was determined in accordance with the following experimental method.

**Experimental Method**

Yeast *(Saccharomyces cerevisiae)* cells expressing the human cannabinoid CB2 receptor were generated by integration of an expression cassette into the *ura*3 chromosomal locus of yeast strain MMY23. This cassette consisted of DNA sequence encoding the human CB2 receptor flanked by the yeast GPD promoter to the 5' end of CB2 and a yeast transcriptional terminator sequence to the 3' end of CB2. MMY23 expresses a yeast/mammalian chimeric G-protein alpha subunit in which the C-terminal 5 amino acids of Gpa1 are replaced with the C-terminal 5 amino acids of human Gαi3 (as described in Brown *et al.* (2000), *Yeast* **16**:11-22). Cells were grown at 30°C in liquid Synthetic Complete (SC) yeast media (Guthrie and Fink (1991), Methods in Enzymology, Vol. 194) lacking uracil, tryptophan, adenine and leucine to late logarithmic phase (approximately 6 $OD_{600}$/ml).

Agonists were prepared as 10 mM stocks in DMSO. $EC_{50}$ values (the concentration required to produce 50% maximal response) were estimated using dilutions of between 3- and 5-fold (BiomekFX, Beckman) into DMSO. Agonist solutions in DMSO (1% final assay volume) were transferred into black, clear bottom, microtitre plates from NUNC (96- or 384-well). Cells were suspended at a density of 0.2 $OD_{600}$/ml in SC media lacking histidine, uracil, tryptophan, adenine and leucine and supplemented with 10mM 3-aminotriazole, 0.1 M sodium phosphate pH 7.0, and 20M fluorescein di-β-D-glucopyranoside (FDGlu). This mixture (50ul per well for 384-well plates, 200ul per well for 96-well plates) was added to agonist in the assay plates (Multidrop 384, Labsystems). After incubation at 30°C for 24 hours, fluorescence resulting from degradation of FDGlu to fluorescein due to exoglucanase, an endogenous yeast enzyme produced during agonist-stimulated cell growth, was determined using a Spectrofluor microtitre plate reader (Tecan; excitation wavelength: 485nm; emission wavelength: 535nm). Fluorescence was plotted against compound concentration and iteratively curve fitted using a four parameter fit to generate a concentration effect value. Efficacy ($E_{max}$) was calculated from the equation

$$E_{max} = Max_{[compound\ X]} - Min_{[compound\ X]} / Max_{[HU210]} - Min_{[HU210]} \times 100\%$$

where $Max_{[compound\ X]}$ and $Min_{[compound\ X]}$ are the fitted maximum and minimum respectively from the concentration effect curve for compound X, and $Max_{[HU210]}$ and $Min_{[HU210]}$ are the fitted maximum and minimum respectively from the concentration effect curve for (6aR,10aR)-3-(1,1'-Dimethylheptyl)-6a,7,10,10a-tetrahydro-1-hydroxy-6,6-dimethyl-6H-dibenzo[b,d]pyran-9-methanol (HU210; available from Tocris). Equieffective molar ratio (EMR) values were calculated from the equation

$$EMR = EC_{50\ [compound\ X]} / EC_{50\ [HU210]}$$

Where $EC_{50\ [compound\ X]}$ is the $EC_{50}$ of compound X and $EC_{50\ [HU210]}$ is the $EC_{50}$ of HU210.

Compounds of Examples 1 to 4, 15, 17 to 24, 44 to 58 70 to 73 and 78 tested according to this method had $EC_{50}$ values of less than 300nM and efficacy values of >50% at the cloned human cannabinoid CB2 receptor.

Compounds of Examples 5 to 8, 14, 16, and 25 to 32, 74 to 76 had $EC_{50}$ >300nM but <1000nM and efficacy >50% at the cloned human cannabinoid CB2 receptor.

Compounds of Examples 9 to 13, 33 to 43 and 59 to 69, 77 and 79 had $EC_5O$ >1000nM and/or efficacy <50% at the cloned human cannabinoid CB2 receptor.

The following examples are illustrative, but not limiting of the embodiments of the present invention.

The following abbreviations are used herein and are represented by.

THF is tetrahydrofuran;

DDQ is 2,3,-dichloro-5,6-dicyano-1,4-benzoquinone;
PTFE is polytetrafluoroethylene;
HPLC is high performance liquid chromatography;
DMF is N,N-dimethylforamide
EtOH is ethanol

All NMR experimental data was recorded at 4.00MHz unless otherwise indicated

**Conditions, Hardware, and Software used for Mass-directed Autopurification Hardware**

Waters 600 gradient pump, Waters 2700 sample manager, Waters Reagent Manager, Micromass ZMD mass spectrometer, Gilson 202 - fraction collector, Gilson Aspec - waste collector.

**Software**

Micromass Masslynx version 3.5

**Column**

The column used is typically a Supelco ABZ+ column whose dimensions are 10mm internal diameter by 100mm in length. The stationary phase particle size is 5μm.

**Solvents**

A. Aqueous solvent = Water + 0.1 % Formic Acid
B. Organic solvent = MeCN: Water 95:5 +0.05% Formic Acid

Make up solvent = MeOH: Water 80:20 +50mMol Ammonium Acetate
Needle rinse solvent = MeOH: Water: DMSO 80:10:10

**Methods**

Five methods are used depending on the analytical retention time of the compound of interest. They all have a flow rate of 20ml/min and a 15-minute runtime, which comprises of a 10-minute gradient followed by a 5-minute column flush and re-equilibration step.

Method 1 MDP 1.5-2.2 = 0-30%B
Method 2 MDP 2.0-2.8 = 5-30% B
Method 3 MDP 2.5-3.0 = 15-55%B
Method 4 MDP 2.8-4.0 = 30-80% B
Method 5 MDP 3.8-5.5 = 50-90% B

**Conditions, Hardware, and Software used for Biotage Horizon HPFC System.**

Column: Biotage C18HS 25+S
Fraction volume: 9ml; UV Threshold : 0.03AU
Solvent A= Water , B= Acetonitrile ; Gradient :

| Volume(ml) | A | B |
|---|---|---|
| 0 | 70% | 30% |
| 240 | 0% | 100% |

**Description 1: Methyl 6-(3-chlorophenylamino)-4-(trifluoromethyl)-nicotinate**

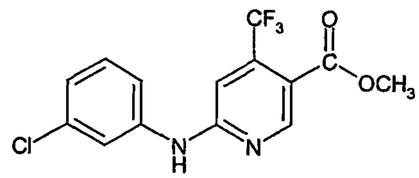

A mixture of methyl 6-chloro-4-(trifluoromethyl)-nicotinate (0.7 g, ex Fluorochem) and 3-chloroaniline (0.62 mL) was heated at 120˚C for 6 h. The reaction mixture solidified and the crude crystals were used for the next step without further purification.
LC-MS (ESI+): t = 10.20 min,(MH+) 331 and 333.

**Description 2: 6-(3-Chlorophenylamino)-4-(trifluoromethyl)-nicotinic acid hydrochloride**

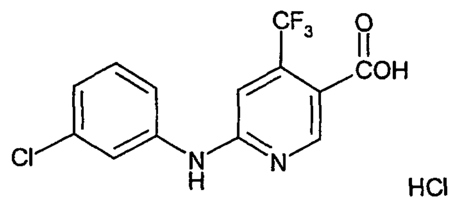

To a suspension of methyl 6-(3-chlorophenylamino)-4-(trifluoromethyl)-nicotinate (Description 1) (1.0 g) in ethanol (5 mL) was added a solution of potassium hydroxide (510 mg) in water (5 mL) and the solution was stirred at reflux for 30 min. After removal of the ethanol under reduced pressure, the mixture was diluted with water (10 mL) and washed twice with dichloromethane. Concentrated hydrochloric acid was added to adjust pH to 1 and the precipitated solid was filtered and dried *in vacuo* at 60 ˚C to afford 6-(3-chlorophenylamino)-4-(trifluoromethyl)-nicotinic acid as its hydrochloride salt (0.62 g).
LC-MS (ESI+): t = 8.51 min, (MH+) 317 and 319.

**Description 3: 6-Chloro-4-isopropyl-nicotinic acid.**

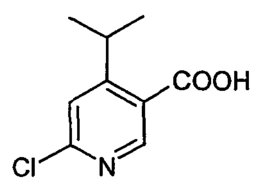

2M isopropylmagnesium bromide in tetrahydrofuran (48 ml) was added dropwise over 1 hour to a solution of 6-chloronicotinic acid (Aldrich) (6.0 g) in dry tetrahydrofuran (100 ml) at 0˚ under nitrogen and the solution stirred at 0˚ for 3 hours then at room temperature for 15 hours. It was cooled to -60˚ and acetic acid (48 ml), tetrahydrofuran (40 ml) and manganese (III) acetate dihydrate (20.4 g) added successively. The mixture was stirred at -70˚ for 30 minutes, then at room temperature for 1 hour. The suspension was filtered through Celite and the filtrate evaporated under reduced pressure. The residue was partitioned between dichloromethane (150 ml) and water (120 ml) and the aqueous layer separated and washed with dichloromethane (2 x 50 ml). The combined organic layers were dried ($MgSO_4$) and evap-

orated under reduced pressure to afford, after silica gel chromatography using 3:1 isohexane:ethyl acetate, 6-chloro-4-isopropyl-nicotinic acid (2.31g,).
NMR (DMSO-d6)δ 1.21 (6H, d), 3.76 (1 H, m), 7.60 (1 H, s), 8.67 (1 H, s), 13.55 (1 H, br s).
LC/MS t = 2.6 min, [MH+] 200 consistent with molecular formula $C_9H_{10}{}^{35}ClNO_2$

**Description 4: 6-(3-Chlorophenylamino)-4-isopropyl-nicotinic acid.**

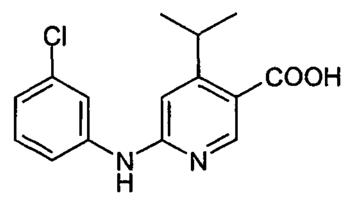

A mixture of 6-chloro-4-isopropyl-nicotinic acid (Description 3) (0.50 g) and 3-chloroaniline (265 mg) was stirred at 120° for 1.5 hours. Isopropanol was added and the mixture chilled. Insoluble solid was filtered off, washed successively with isopropanol and ether and dried *in vacuo* at 50° to afford 6-(3-chlorophenylamino)-4-isopropyl-nicotinic acid (0.51 g).
NMR (DMSO-d6) δ 1.19 (6H, d), 3.93 (1 H, m), 6.85 (1 H, s), 6.99 (1 H, d), 7.31 (1 H, t), 7.53 (1H, d), 8.00 (1 H, s), 8.64 (1 H, s), 9.73 (1 H, s), 12.6 (1 H, br s).
LC/MS t = 3.63 min, [MH+] 291, consistent with molecular formula $C_{15}H_{15}{}^{35}ClNO_2$

**Description 5: 6-Chloro-*N*-(4-fluoro-benzyl)-nicotinamide**

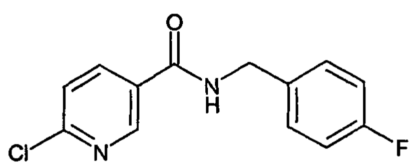

A solution of 4-fluorobenzylamine (4.6g) and triethylamine (5.57g) in dichloromethane (60ml) was added over 1 hour to a stirred solution of 6-chloronicotinoyl chloride (Lancaster Synthesis) (6.46g) in dichloromethane (60ml) at 0° under nitrogen. Stirring was continued for 1 hour, and the reaction allowed to warm to ambient temperature. The solution was diluted with dichloromethane, washed with aqueous 1 M hydrochloric acid, aqueous saturated sodium bicarbonate, and water. The dried ($Na_2SO_4$) organic layer was evaporated to dryness, and triturated with dichloromethane to give 6-chloro-N-(4-fluoro-benzyl)-nicotinamide (6.83g).
NMR (d6-DMSO) δ 4.47 (2H,d), 7.18 (2H, t), 7.37 (2H, m), 7.66 (1 H, d), 8.28 (1 H, d), 8.85 (1H, s), 9.31 (1 H, t).
LC/MS t = 2.50 min, [MH+] 265

**Description 6: 6-Chloro-*N*-(4-fluoro-benzyl)-4-isopropyl-nicotinamide**

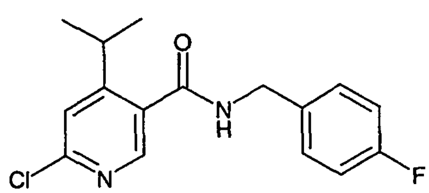

Isopropylmagnesium chloride (2M in tetrahydrofuran, 38ml) was added dropwise over 30 min to a stirred solution of 6-chloro-*N*-(4-fluoro-benzyl)-nicotinamide (Description 5) (6.83g) in THF (35ml) at 0˚ under nitrogen. After stirring at ambient temperature for 16h, the solution was cooled to 0˚, and treated with dry methanol (6 ml) over 3 min. After 15 min, DDQ (6.45g) was added and stirring continued for 30 min. The mixture was concentrated under reduced pressure to to 6 to 7 ml. The oil was warmed to 50˚, treated with t-butyl methyl ether (120ml), and stirred at 55˚ for 1 h. The mixture was filtered, and the solid washed with t-butyl methyl ether. The combined filtrates were evaporated, and the residue purified by Biotage chromatography over silica gel (40g), eluting with isohexane/ethyl acetate (7:3) to give 6-chloro-N-(4-fluoro-benzyl)-4-isopropyl-nicotinamide (4.45g).
NMR ($d^6$-DMSO) δ 1.20 (6H, d), 3.22 (1 H, multiplet), 4.46 (2H, d), 7.18 (2H, t), 7.39 (2H, m), 7.55 (1H, s), 8.37(1H, s), 9.15 (1 H, t).
LC/MS t = 3.0 min, [$MH^+$] 307

**Description 7: 6-(3-Chloro-phenylamino)-4-trifluoromethyl-nicotinic acid**

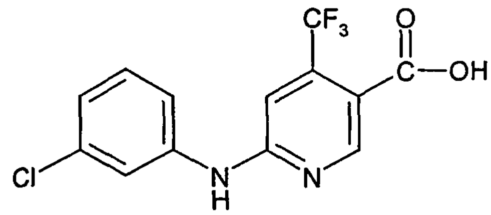

A solution of KOH (1.68 g, 31 mmol) in 30 mL of EtOH / $H_2O$ (1:1) was added to the crude mixture from Description 1 and the resulting mixture was stirred under reflux for 3h. The solution was concentrated in vacuo, diluted with water and washed three times (3 x 15 mL) with diethyl ether. Upon acidification of the aqueous layer to pH1 with 37% HCl the title compound precipitated out as the hydrochloride salt, which was filtered and dried under vacuum. The solid (2.05 g, 5.82 mmol) was then suspended in dichloromethane (25 mL), in the presence of PS-diisopropylethylamine (1.5 g, 5.8 mmol, loading 3.88 mmol/g, ex Argonaut Technologies) and stirred at room temperature for 30 min. After filtration of the resin and evaporation in vacuo of the solvent, the title compound was isolated as a white solid (1.5 g).
$^1$H NMR (300 MHz, DMSO-$d_6$) δ: 13.16 (s br, 1H); 10.28 (s, 1H); 8.80 (s, 1H); 8.01 (dd, 1H); 7.58 (ddd, 1 H); 7.35 (dd, 1 H); 7.28 (s, 1 H); 7.06 (ddd, 1H).
MS m/z (ESI+): 317 ($MH^+$).

**Description 8: C-(2-Fluoro-pyridin-4-yl)-methylamine dihydrochloride.**

(a). 4-Bromomethyl-2-fluoro-pyridine.

To a solution of 2-fluoro-4-methylpyridine (1.0 g, ex Lancaster) in carbon tetrachloride (10 ml) was added N-bromosuccinimide (1.6 g, ex Lancaster) and 1,1'- azobis (cyclohexanecarbonitrile) (100 mg, ex Aldrich). The mixture was then refluxed for 24h. Carbon tetrachloride was removed under reduced pressure and the crude oily solid was used in the next stage without purification.
LC/MS, t = 2.38 min, [$MH^+$] 190 and 192.

(b). (2-Fluoro-pyridin-4-ylmethyl)-carbamic acid tert-butyl ester.

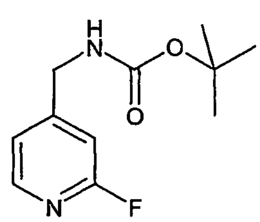

To crude 4-bromomethyl-2-fluoro-pyridine in an ice bath was added 25% ammonia solution (10 ml, ex BDH) and the mixture stirred at 0° for 5h. Ammonia solution was removed under reduced pressure and the yellow oily solid residue dissolved in dichloromethane (10 ml) and dimethylformamide (1 ml). The solution was cooled in an ice bath and triethylamine (1.5 ml, ex BDH) was added followed by di-tert-butyl dicarbonate (1.0 g, ex Avocado). The solution was stirred at 0°for 1h and then the dichloromethane removed under reduced pressure. The residue was dissolved in ethyl acetate and washed twice with water, dried ($MgSO_4$) and evaporated to give a yellow oil. This was purified by Biotage chromatography (100 g, silica column) eluting with 30% ethyl acetate in hexane to afford the title compound as a white solid (358 mg).

NMR (DMSO-d6) δ 1.40 (9H, s), 4.20 (2H, d), 6.97 (1 H, s), 7.20 (1 H, d), 7.60 (1 H, t), 8.17 (1 H, d)

LC/MS, t = 2.60 min, $[M - Me2C{=}CH2 + H]^+$ 171

c) (2-Fluoro-pyridin-4-ylmethyl)-carbamic acid tert-butyl ester (350mg) was treated at room temperature with 4N hydrochloric acid in 1,4-dioxan (5 ml) and stirred for 2h. The white precipitate was filtered, washed with fresh ether and dried to afford the title compound (200 mg).

NMR (400MHz, DMSO-d6) δ 4.14 (2H, d), 7.38 (1H, s), 7.51 (1H, d), 8.28 (1H, d), 8.82 (3H, s).

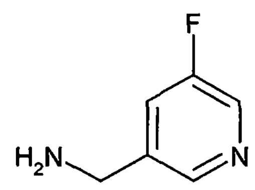

**Description 9: 6-(2,3-Dichloro-phenylamino)-4-trifluoromethyl-nicotinic acid methyl ester**

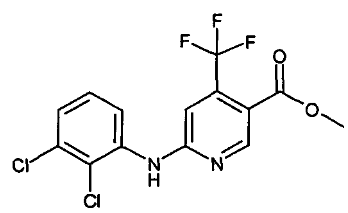

A mixture of methyl-6-chloro-4-(trifluoromethyl)-nicotinate (2.0 g, 8.37 mmol, ex Fluorochem) and 2,3-dichloroaniline (4.06 g, 25 mmol) was heated at 130°C for 18 h, to afford the title compound that was used for the next step without further purification.

MS m/z (ESI+): 365 (MH+).

**Description 10: 6-(2,3-Dichloro-phenylamino)-4-trifluoromethyl-nicotinic acid**

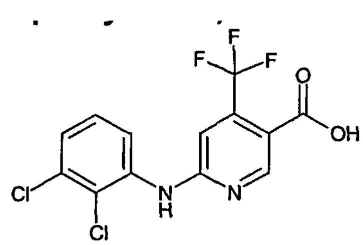

A solution of KOH (1.4 g, 25 mmol) in 20 mL of EtOH / $H_2O$ (1:1) was added to the crude mixture from Description 9 and the resulting mixture was stirred under reflux for 3h. The solution was concentrated in vacuo, diluted with water and washed three times (3 x 15 mL) with diethyl ether. Upon acidification of the aqueous layer to pH1 with 37 %HCl, the title compound precipitated out as hydrochloride salt which was filtered and dried under vacuum. The solid (2.7 g,

7 mmol) was then suspended in dichloromethane (20 mL), in the presence of PS-diisopropylethylamine (1.80 g, 7 mmol, loading 3.88 mmol/g, ex Argonaut Technologies) and stirred at room temperature for 30 min. After filtration of the resin and evaporation in vacuo of the solvent, the title compound was isolated as a white solid (2.45 g).
$^1$H NMR (300 MHz, DMSO-$d_6$) δ: 13.17 (s br, 1 H); 9.61 (s, 1 H); 8.68 (s, 1 H); 7.88 (dd, 1H); 7.44 (dd, 1 H); 7.42 (s, 1H); 7.37 (dd, 1H).
MS m/z (ESI+): 351 (MH+).

**Description 11: 6-(2,4-Dichloro-phenylamino)-4-trifluoromethyl-nicotinic acid methyl ester**

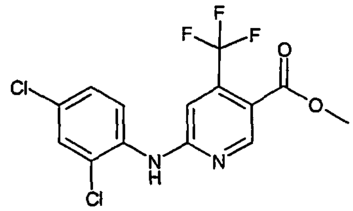

A mixture of methyl-6-chloro-4-(trifluoromethyl)-nicotinate (2.0 g, 8.37 mmol ex Fluorochem) and 2,4-dichloroaniline (4.05 g, 25 mmol) was heated at 130°C for 15 h, to afford the title compound that was used for the next step without further purification.
MS m/z (ESI+): 365 (MH+).

**Description 12: 6-(2,4-Dichloro-phenylamino)-4-trifluoromethyl-nicotinic acid**

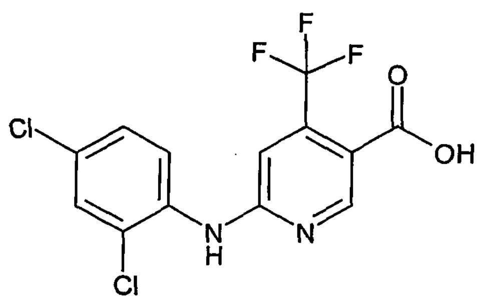

A solution of KOH (1.4 g, 25 mmol) in 20 mL of EtOH / $H_2O$ (1:1) was added to the crude mixture from Description 11 and the resulting mixture was stirred under reflux for 3h. The solution was concentrated in vacuo, diluted with water and washed three times (3 x 15 mL) with diethyl ether. Upon acidification of the aqueous layer to pH1 with 37% HCl, the title compound precipitated out as hydrochloride salt that was filtered and dried under vacuum. The solid (2.89 g, 7.5 mmol) was then suspended in dichloromethane (20 mL), in the presence of PS-diisopropylethylamine (1.93 g, 7.5 mmol, loading 3.88 mmol/g, ex Argonaut Technologies) and stirred at room temperature for 30 min. After filtration of the resin and evaporation in vacuo of the solvent, the title compound was isolated as a white solid (2.62 g).
$^1$H NMR (300 MHz, DMSO-$d_6$) δ: 13.16 (s br, 1 H); 9.49 (s, 1 H); 8.67 (s, 1 H); 7.94 (d, 1 H); 7.67 (d, 1 H); 7.43 (dd, 1 H); 7.40 (s, 1 H).
MS m/z (ESI+): 351 (MH+).

**Description 13: 6-(2,3-Dichloro-phenylamino)-N-(cyclobutyl)-4-trifluoromethyl-nicotinamide**

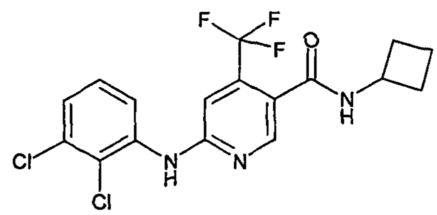

N-methylmorpholine (48 uL, 0.43 mmol), cyclobutylamine (13 mg, 0.18 mmol), 1-hydroxybenzotriazole (30 mg, 0.22 mmol), 1-(3-dimethylamino-propyl)-3-ethylcarbodiimide hydrochloride (32 mg, 0.17 mmol) were added to a solution of 6-(2,3-dichloro-phenylamino)-4-trifluoromethyl nicotinic acid (Description 10) (50 mg, 0.14 mmol) in dimethylformamide (3 mL). After stirring at room temperature for 6 h, dimethylformamide was evaporated under reduced pressure and dichloromethane was added. The solution was washed with an aqueous solution of 5% $NaHCO_3$ (5 mL), with water (10 mL), then with brine (2 x 3 mL) and was evaporated under reduced pressure. The crude residue was triturated with diethyl ether, filtered and dried under vacuum to afford the title compound (46 mg, yield=81 %).
$^1H$ NMR (300 MHz, DMSO-$d_6$) δ: 9.27 (s br, 1 H); 8.66 (d br, 1 H); 8.27 (s, 1H); 7.90 (dd, 1H); 7.42-7.31 (m, 3H); 4.30 (m, 1 H); 2,21 (m, 2H); 1.97 (m, 2H); 1.66 (m, 2H).
MS m/z (EI+); TSQ 700; source 180°C; 70 V; 200 uA: 403 ($M^+$.), 375, 332.

**Description 14: 6-(2,4-Dichloro-phenylamino)-N-(tetrahydropyran-4-ylmethyl)-4-trifluoromethyl-nicotinamide**

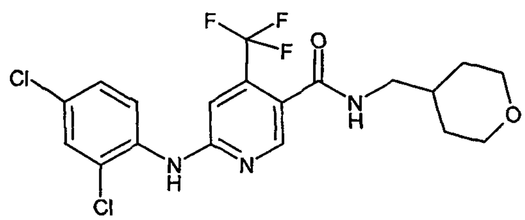

1-Hydroxy-7-azabenzotriazole (33 mg, 0.24 mmol), tetrahydropyran-4-ylmethylamine (17 mg, 0.14 mmol) and PS-carbodiimide (218 mg, 0.28 mmol, loading 1.31 mmol/g, ex Argonaut Technologies) were added to a solution of 6-(2,4-dichloro-phenylamino)-4-trifluoromethyl nicotinic acid (Description 12) (75 mg, 0.21 mmol) in 3 mL of dichloromethane. After orbital shaking at room temperature overnight, the resin was filtered and washed repeatedly with dichloromethane; the filtrate was treated with an aqueous solution of 5% $NaHCO_3$. The organic layer was separated through Phase Separator cartridge, dried over sodium sulphate and evaporated in vacuo. The solid residue was triturated with acetonitrile, filtered and dried under vacuum to afford the title compound (44 mg, yield=46 %).
$^1H$ NMR (300 MHz, DMSO-$d_6$) δ: 9.18 (s, 1H); 8.48 (t br, 1 H); 8.27 (s, 1 H); 7.98 (d, 1 H); 7.66 (d, 1 H); 7.42 (dd, 1 H); 7.37 (s, 1 H); 3.84 (dd, 2H); 3.26 (dd, 2H); 3.10 (dd, 1 H); 1.74 (m, 1 H); 1.60 (d br, 2H); 1.18 (m, 2H).
MS m/z (EI+); TSQ 700; source 180°C; 70 V; 200 uA: 447($M^+$.), 412, 333, 314.

**Description 15: (6-methyl-pyridin-3-yl)-methylamine-dihydrochloride**

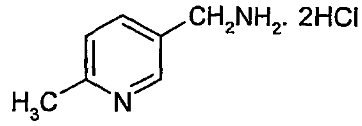

A mixture of 5-cyano-2-methylpyridine (ex Lancaster)(0.5 g), Raney nickel (0.5 g) and acetic acid (15 ml) was

hydrogenated at 50 psi for 24 hours. The catalyst was filtered off and the filtrate evaporated under reduced pressure. Water (20 ml) was added and the solution basified to pH 9 with sodium carbonate. The mixture was extracted with dichloromethane (25 ml then 2 x 10 ml) and the combined extracts washed with water, dried ($MgSO_4$), and evaporated under reduced pressure. The residue was dissolved in ether and the solution treated with 4N hydrogen chloride in dioxan (1.5 ml). The solvent was removed under reduced pressure to give, after trituration with hot isopropanol, (6-methyl-pyridin-3-yl)-methylamine dihydrochloride (35 mg).
NMR (DMSO-d6) δ 2.71 (3H, s), 4.19 (2H, d), 7.84 (1 H, d), 8.43 (1 H, d), 8.66 (3H, br s), 8.86 (1H, s).

**Description 16: 6-Hydroxy-2-trifluoromethyl-4,5-dihydro-pyridine-3-carboxylic acid ethyl ester**

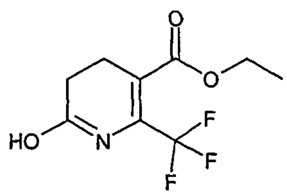

A mixture of ethyl 4,4,4-trifluoroacetoacetate (14.7 mL, 0.1 mol, 1.6 eq), acrylamide (4.5 g, 0.063 mol, 1.0 eq) and p-toluenesulphonic acid (0.156 g, 0.82 mmol, 0.013 eq) in toluene (60 mL) was refluxed for 38 h with azeotropic removal of water (Dean-Stark conditions). The reaction mixture was then concentrated to a small volume, by slow distillation of toluene at atmospheric pressure. Toluene (60 mL) was added and again the reaction mixture was concentrated, through slow distillation of toluene. After repeating this operation three times, the reaction mixture was concentrated in vacuo and the solid residue was purified by flash chromatography (silica gel, eluent gradient: from hexane / ethyl acetate 9:1 to hexane / ethyl acetate 8:2). The title compound was obtained as a brownish solid (3.8 g, yield = 25%).
LC-MS (ESI+), MH+: 238, 210, 190.

**Description 17. 6-Hydroxy-2-trifluoromethyl-nicotinic acid ethyl ester**

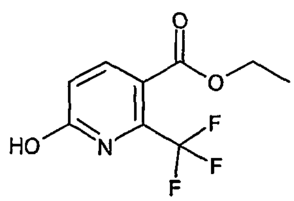

A solution of 6-hydroxy-2-trifluoromethyl-4,5-dihydro-pyridine-3-carboxylic acid ethyl ester (Description 16) (4.7 g, 19.8 mmol, 1 eq) and N-bromo succinimide (3.51 g, 19.8 mmol, 1 eq) in 15 mL of carbon tetrachloride was heated under reflux for 20 h. The resulting precipitate was filtered off and the filtrate was concentrated under reduced pressure to afford a brownish solid that was purified by flash chromatography (silica gel, eluent gradient: from hexane / ethyl acetate 9:1 to hexane / ethyl acetate 8:2). The title compound was obtained as a white solid (4.3 g, yield = 92%).
LC-MS (ESI+), MH+: 236.

**Description 18. 6-Chloro-2-trifluoromethyl-nicotinic acid ethyl ester**

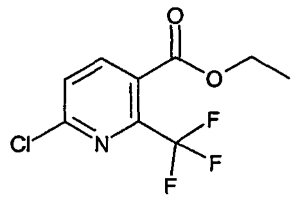

A mixture of 6-hydroxy-2-trifluoromethyl-nicotinic acid ethyl ester (Description 17) (2.6 g, 11.0 mmol, 1.0 eq) and phenyl dichlorophosphate (2.47 mL, 16.5 mmol, 1.5 eq) was heated under microwaves irradiation for 30 min (170˚C, power = 70 W). The reaction mixture was poured into ice, stirred for 20 min and diluted with ethyl acetate (50 mL). The pH was adjusted to 10, by addition of a saturated aqueous solution of sodium bicarbonate (50 mL) and then the organic layer was separated, washed with water, dried over $Na_2SO_4$ and concentrated in vacuo. The resulting solid residue was purified by flash chromatography (silica gel, eluent gradient: from hexane to hexane / ethyl acetate 98:2) to give 1.7 g of the title compound (yield = 61%).
LC-MS (ESI+), MH+: 254 and 256.

**Description 19. 6-(3-Chloro-phenylamino)-2-trifluoromethyl-nicotinic acid ethyl ester**

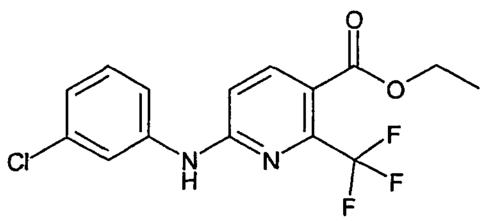

A mixture of 6-chloro-2-trifluoromethyl-nicotinic acid ethyl ester (Description 18) (1.4 g, 5.53 mmol, 1.0 eq) and 3-chloroaniline (2.91 mL, 27.6 mmol, 5.0 eq) was heated at 160˚C for 52 h to afford a black solid which was used for the next step without further purification. LC-MS (ESI+), MH+: 345 and 347.

**Description 20. 6-(3-Chloro-phenylamino)-2-trifluoromethyl-nicotinic acid hydrochloride**

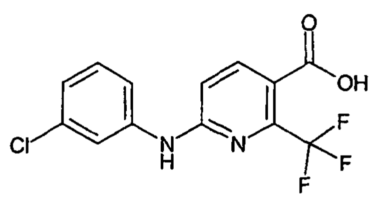

A solution of KOH (1.18 g) in water (25 mL) was added to a mixture of crude 6-(3-chloro-phenylamino)-2-trifluoromethyl-nicotinic acid ethyl ester from Description 19 in ethanol (25 mL) and refluxed for 8h. After evaporation of ethanol under reduced pressure, the reaction mixture was diluted with water (35 mL) and repeatedly washed with diethyl ether (200 mL x 5 times). The aqueous layer was treated with conc. HCl to adjust the pH to 3 and the title compound precipitated out as its hydrochloride salt, was filtered and dried at 40˚C in oven (1.71 g).
LC-MS (ESI+), MH+: 317 and 319.

The amines which are coupled with acids to make the Examples are all commercially available, except, C-(2-fluoro-pyridin-4-yl)-methylamine dihydrochloride, (Description 8), and C-(1*H*-imidazol-2-yl)-methylamine which has the CAS- Registry number 53332-80-2, and for which a synthetic procedure is disclosed in the literature, 4-aminomethyl-benzamide (Example 8) - UpJohn Patent application WO97/4540 (1997), N-(4-aminomethyl)-phenyl)-methansulfonamide (Example 12) Schering patent application WO90/00548, 4-aminomethyl-N-methyl-benzamide (Example 13) where the freebase is prepared as in WO94/17035 which can be converted to the hydrochloride salt by known means.

**Example 1: 2-(3-Chlorophenylamino)-4-trifluoromethylpyridine-5-carboxylic acid (pyridin-4-ylmethyl) amide**

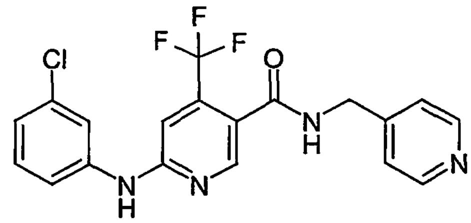

To a solution of 6-(3-chlorophenylamino)-4-(trifluoromethyl)-nicotinic acid hydrochloride (Description 2) (0.2 g) in dimethylformamide (5 mL) were added N-methylmorpholine (283 μL), C-pyridin-4-yl-methylamine (62 μL), 1-hydroxybenzotriazole hydrate (104 mg), 1-(3-dimethylamino-propyl)-3-ethylcarbodiimide hydrochloride (118 mg). After stirring at room temperature for 6 h, dimethylformamide was evaporated under reduced pressure and dichloromethane added. The solution was washed with a 5% aqueous solution of potassium carbonate (5 mL), then with brine (2 x 3 mL) and was evaporated under reduced pressure. Chromatographic purification (silica gel; hexane, ethyl acetate 8:2) afforded the title compound (62 mg).
$^1$H NMR (300 MHz, DMSO-d6) δ 9.95 (1 H, br s), 9.1 (1 H, t), 8.55 (3H, m), 8.05 (1 H, s), 7.5 (1 H, d), 7.35 (3H, t), 7.22 (1 H, s), 7.05 (1 H, d), 4.5 (2H, d).
MS m/z (EI+): 406 and 408 ($M^+$.), 299, 236.
IR (KBr): 3467 cm-1, 3248,1646.

**Example 2: 2-(3-Chlorophenylamino)-4-trifluoromethylpyridine-5-carboxylic acid benzylamide**

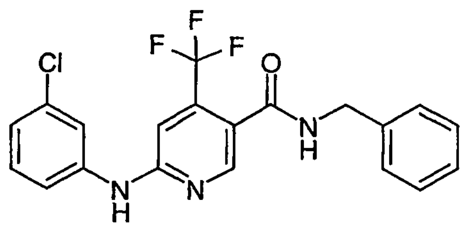

In a manner similar to the method described above, 6-(3-chlorophenylamino)-4-(trifluoromethyl)-nicotinic acid hydrochloride (Description 2) (0.2 g) was reacted benzylamine (67μL) to afforded 2-(3-chlorophenylamino)-4-trifluoromethylpyridine-5-carboxylic acid benzylamide (48 mg).
$^1$H NMR (300 MHz, DMSO-d6) δ 9.9 (1 H,s) 9.0 (1 H, t), 8.5 (1 H, s), 8.02 (1 H, s), 7.5 (1 H, d), 7.15-7.4 (7H, m), 7.02 (1 H, d), 4.45 (2H, d).
MS m/z (EI+): 405 and 407 ($M^+$.), 336, 299, 236.
IR (KBr): 3401 cm-1, 3308, 1648.

**Example 3: 6-(3-Chlorophenylamino)-N-(4-fluorobenzyl)-4-isopropyl-nicotinamide**

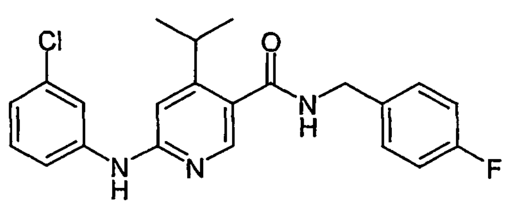

To a solution of 6-(3-chlorophenylamino)-4-isopropyl-nicotinic acid (Description 4) (48 mg) in dimethylformamide (2.5 ml) was added successively N-ethylmorpholine (69 μl), 4-fluorobenzylamine (23 μl), 1-hydroxybenzotriazole hydrate (40 mg) and 1-(3-dimethylamino-propyl)-3-ethylcarbodiimide hydrochloride (40 mg). The solution was stirred for 3 h and allowed to stand overnight. Dimethylformamide was removed under reduced pressure and ethyl acetate (8 ml) added. The solution was washed sequentially with aqueous 5% sodium bicarbonate solution (5 ml), water (5 ml) and brine (2 x 5 ml). The dried ($MgSO_4$) solution was evaporated to afford the title compound (56 mg).
NMR (DMSO-d6) δ 1.15 (6H, d), 3.43 (1 H, m), 4.41 (2H, d), 6.79 (1 H, s), 6.93 (1 H, d), 7.17 (2H, t), 7.28 (1 H, t), 7.38 (2H, m), 7.46 (1 H, d), 8.06 (1 H, t), 8.21 (1 H, s), 8.91 (1 H, t), 9.44 (1 H, s).
LC/MS t = 3.5 min, [$MH^+$] 398.

**Table 1:**

| Compounds of Example 4 to 13 were prepared in a manner similar to that described in Example 3. | | | |
|---|---|---|---|
| Ex No. | Name | Structure | RT (min), (MH+) Consistent with molecular formula |
| 4 | N-Benzyl-6-(3-chlorophenylamino)-4-isopropyl-nicotinamide | 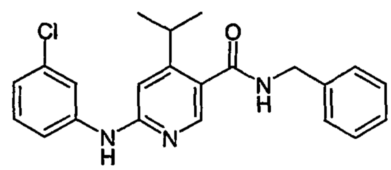 | 3.6 380 $C_{22}H_{22}{}^{35}ClN_3O$ |
| 5 | 6-(3-Chloro-phenylamino)-N-(4-cyano-benzyl)-4-isopropyl-nicotinamide | 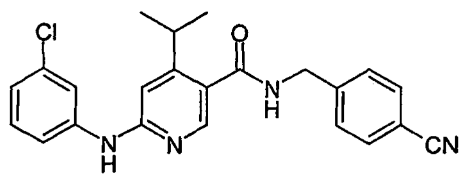 | 3.3 405 $C_{23}H_{21}{}^{35}ClN_4O$ |
| 6 | 6-(3-Chloro-phenylamino)-4-isopropyl-N-(4-methoxy-benzyl)-nicotinamide | 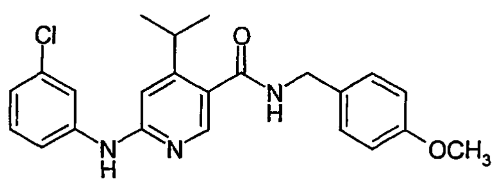 | 3.5 410 $C_{23}H_{24}{}^{35}ClN_3O_2$ |
| 7 | 6-(3-Chloro-phenylamino)-N-(3,4-difluoro-benzyl)-4-isopropyl-nicotinamide | 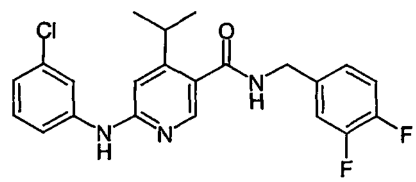 | 3.6 416 $C_{22}H_{20}{}^{35}ClF_2N_3O$ |
| 8 | N-(4-Carbamoyl-benzyl)-6-(3-chloro-phenylamino)-4-isopropyl-nicotinamide | 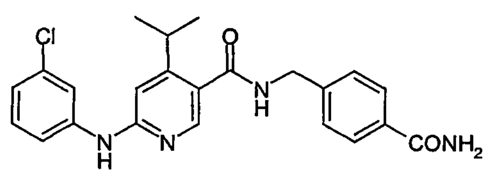 | 3.0 423 $C_{23}H_{23}{}^{35}ClN_4O_2$ |
| 9 | 6-(3-Chloro-phenylamino)-N-(2,4-difluoro-benzyl)-4-isopropyl-nicotinamide | 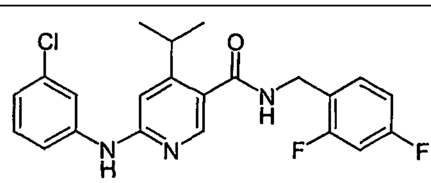 | 3.6 416 $C_{22}H_{20}{}^{35}ClF_2N_3O$ |

(continued)

| Compounds of Example 4 to 13 were prepared in a manner similar to that described in Example 3. | | | |
|---|---|---|---|
| Ex No. | Name | Structure | RT (min), (MH+) Consistent with molecular formula |
| 10 | 6-(3-Chloro-phenylamino)-4-isopropyl-N-(4-methanesulfonyl-benzyl)-nicotinamide | 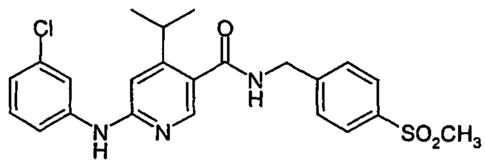 | 3.2<br>458<br>$C_{23}H_{24}{}^{35}ClN_{3}0_{3}S$ |
| 11 | N-(4-Acetylamino-benzyl)-6-(3-chloro-phenylamino)-4-isopropyl-nicotinamide | 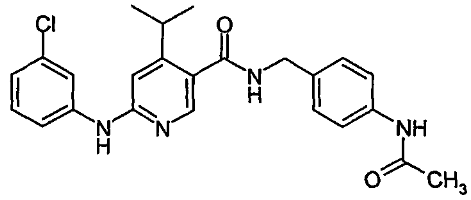 | 3.1<br>437<br>$C_{24}H_{25}{}^{35}ClN_{4}O_{2}$ |
| 12 | 6-(3-Chloro-phenylamino)-4-isopropyl-N-(4-methane-sulfonylamino-benzyl)-nicotinamide | 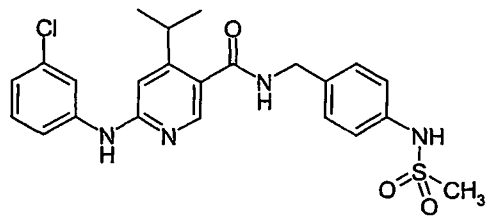 | 3.2<br>473<br>$C_{23}H_{25}{}^{35}ClN_{4}O_{3}S$ |
| 13 | 6-(3-Chloro-phenylamino)-4-isopropyl-N-(4-methylcarbamoyl-benzyl)-nicotinamide | 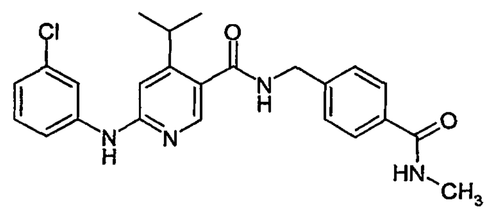 | 3.1<br>437<br>$C_{24}H_{25}{}^{35}ClN_{4}O_{2}$ |

**Example 14: N-(4-Fluoro-benzyl)-4-isopropyl-6-(3-trifluoromethyl-phenylamino)-nicotinamide**

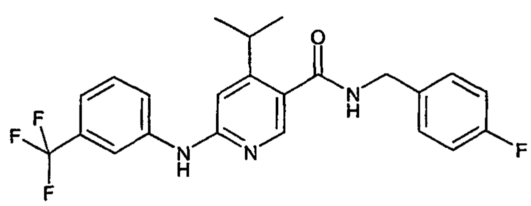

A mixture of 6-chloro-N-(4-fluoro-benzyl)-4-isopropyl-nicotinamide (Description 6) (80 mg), 3-trifluoromethyl-aniline (63 mg), methanesulphonic acid (50mg), and 1,4-dioxan (0.8ml) were heated at 180° in the microwave apparatus for 30 min. The mixture was diluted with methanol (3 ml) and purified on the Biotage Horizon HPFC System to give N-(4-Fluoro-benzyl)-4-isopropyl-6-(3-trifluoromethyl-phenylamino)-nicotinamide (43 mg).

NMR ($d^6$-DMSO) $\delta$ 1.20 (6H, d), 3.47 (1 H, m), 4.42 (2H, d), 6.85 (1 H,s), 7.1-7.3 (3H, m), 7.4 (2H, br s), 7.5 (1H, m), 7.85 (1 H, d), 8.25 (1 H, s), 8.35 (1 H, s), 8.95 (1H, br s), 9.65 (1H, s).

LC/MS t = 3.69min, [$MH^+$] 432 consistent with molecular formula $C_{20}H_{15}F_{4}N_{3}O$

**Example 15: 6-(3-Chloro-4-fluoro-phenylamino)-*N*-(4-fluoro-benzyl)-4-isopropyl-nicotinamide**

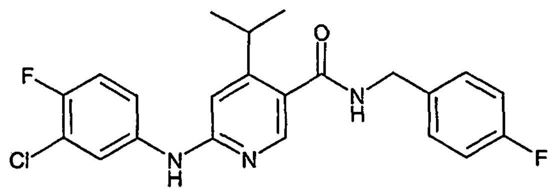

A mixture of 6-chloro-*N*-(4-fluoro-benzyl)-4-isopropyl-nicotinamide (Description 6) (100 mg), 3-chloro-4-fluoroaniline (47 mg), methanesulphonic acid (31 mg), and 1,4-dioxan (1 ml) was irradiated at 180° in the microwave apparatus for 30 min. The solution was evaporated, and the residue partitioned between ethyl acetate and brine. The organic layer was washed with brine and evaporated. The residue was purified on the Biotage Horizon HPFC System to give 6-(3-chloro-4-fluoro-phenylamino)-N-(4-fluoro-benzyl)-4-isopropyl-nicotinamide (41 mg) as a white solid.
NMR ($d^6$-DMSO) $\delta$ 1.12 (6H, d), 3.42 (1 H, multiplet), 4.40 (2H, d), 6.77 (1 H, s), 7.19 (2H, t), 7.3-7.4 (3H, m), 7.45-7.5 (1H. m), 8.17 (1 H, dd), 8.21 (1 H, s), 8.9 (1 H, t), 9.45 (1H, s).
LC/MS t = 3.50 min [$MH^+$] 416 consistent with the molecular formula $C_{22}H_{20}{}^{35}ClF_2N_3O$

**Example 16: 6-(2-Cyano-3-methyl-phenylamino)-*N*-(4-fluoro-benzyl)-4-isopropyl-nicotinamide**

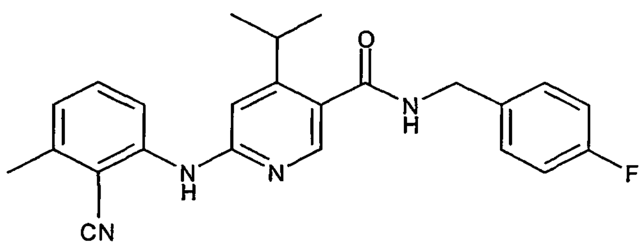

A mixture of 6-chloro-*N*-(4-fluoro-benzyl)-4-isopropyl-nicotinamide (Description 6) (100 mg), 2-amino-6-methylbenzonitrile (43 mg), cesium carbonate (168mg), tris(dibenzylideneacetone)dipalladium (0) (ex-Aldrich, 3.36mg) and 4,5-bis(diphenylphosphino)-9,9-dimethylxanthene (ex-Aldrich, 2.3 mg) and 1,4-dioxan (1 ml) was heated to relux under nitrogen for 24h. When cool, the mixture was diluted with ethyl acetate and filtered through a PTFE disc (1.0 M) disc and the filtrate evaporated. The residue was purified using the Biotage Horizon HPFC System and the resultant product was triturated with ether, washed with ether, and dried in vacuo at 40° to give 6-(2-cyano-3-methyl-phenylamino)-*N*-(4-fluoro-benzyl)-4-isopropyl-nicotinamide (15mg).
NMR (d6-DMSO) 1.16 (6H, d), 2.46 (3H, s), 3.35-3.45 (1 H, m), 4.36-4.47 (2H, m), 6.95 (1H, s), 7.08 (1 H, d), 7.13 (1 H, t), 7.35 (2H,m), 7.39 (2H, t), 7.68 (1 H, d), 8.07 (1 H, s), 8.9 (1 H, m), 9.14 (1 H, s).
LC/MS t = 3.27min, [MH+] 403 consistent with molecular formula $C_{24}H_{23}FN_4O$

**Table 2**

Compounds of Examples 17 to 24 were prepared in a manner similar to Example 14 (Method A) or Example 15 (Method B)

| Ex. No. | Structure | Name | Method | Ret. Time [MH+] Molecular Formula |
|---|---|---|---|---|
| 17 | 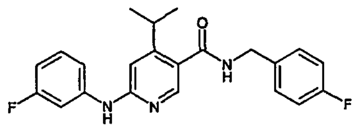 | N-(4-Fluoro-benzyl)-6-(3-fluoro-phenylamino)-4-isopropyl-nicotinamide | A | 3.40min<br>$MH^+$ 382<br>$C_{22}H_{21}F_2N_3$ O |
| 18 | 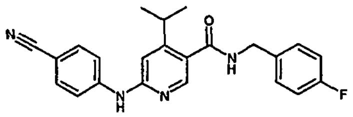 | 6-(4-Cyano-phenylamino)-N-(4-fluoro-benzyl)-4-isopropyl-nicotinamide | A | 3.30min<br>$MH^+$ 389<br>$C_{23}H_{21}FN_4O$ |
| 19 | 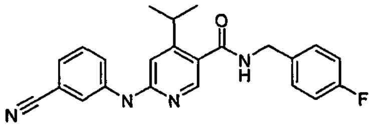 | 6-(3-Cyano-phenylamino)-N-(4-fluoro-benzyl)-4-isopropyl-nicotinamide | A | 3.30min<br>$MH^+$ 389<br>$C_{23}H_{21}FN_4O$ |
| 20 | 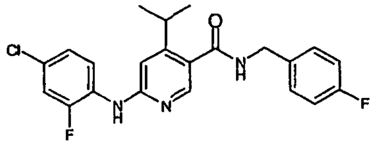 | 6-(4-Chloro-2-fluoro-phenylamino)-N-(4-fluoro-benzyl)-4-isopropyl-nicotinamide | A | 3.60min<br>$MH^+$ 416<br>$C_{22}H_{20}{}^{35}ClF_2N_3$ O |
| 21 | 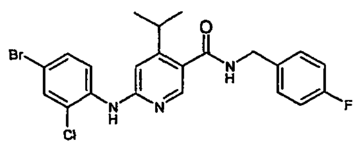 | 6-(4-Bromo-2-chloro-phenylamino)-N-(4-fluoro-benzyl)-4-isopropyl-nicotinamide | A | 3.79min<br>$MH^+$ 478<br>$C_{22}H_{20}{}^{81}BrCl\ FN_3O$ |
| 22 | 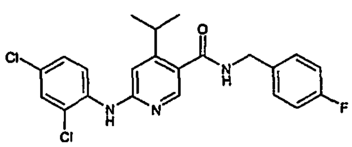 | 6-(2,4-Dichloro-phenylamino)-N-(4-fluoro-benzyl)-4-isopropyl-nicotinamide | A | 3.73min<br>$MH^+$ 433<br>$C_{22}H_{20}Cl_2FN\ _3O$ |
| 23 | 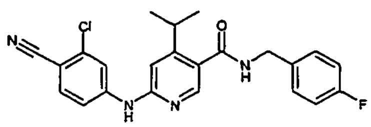 | 6-(3-Chloro-4-cyano-phenylamino)-N-(4-fluoro-benzyl)-4-isopropyl-nicotinamide | B | 3.30min<br>$MH^+$ 423<br>$C_{23}H_{20}\ {}^{35}ClF\ N_4O$ |
| 24 | 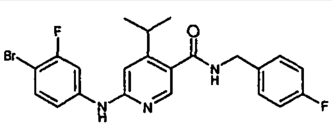 | 6-(4-Bromo-3-fluoro-phenylamino)-N-(4-fluoro-benzyl)-4-isopropyl-nicotinamide | B | 3.80min<br>$MH^+$ 462<br>$C_{22}H_{20}{}^{81}\ BrF\ _2N_3O$ |

**Table 3**

| Compounds of Examples 25 to 32 were prepared in a manner similar to Example 14 (Method A) or Example 15 (Method B) | | | | |
|---|---|---|---|---|
| Ex. No. | Structure | Name | Method | Ret. Time [MH+] Molecular Formula |
| 25 | 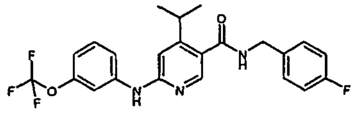 | N-(4-Fluoro-benzyl)-4-isopropyl-6-(3-trifluoromethoxy-phenylamino)-nicotinamide | A | 3.7min<br>$MH^+$ 448<br>$C23H_{21}F_4N_3O$ 2 |
| 26 | 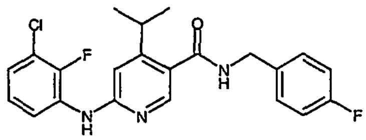 | 6-(3-Chloro-2-fluoro-phenylamino)-N-(4-fluoro-benzyl)-4-isopropyl-nicotinamide | B | 3.40min<br>$MH^+$ 416<br>$C_{22}H_{20}{}^{35}ClF_2\ N_3O$ |
| 27 | 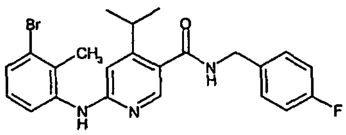 | 6-(3-Bromo-2-methyl-phenylamino)-N-(4-fluoro-benzyl)-4-isopropyl-nicotinamide | B | 3.50min<br>$MH^+$ 458<br>$C_{23}H_{23}{}^{81}\ BrFN\ _3O$ |
| 28 | 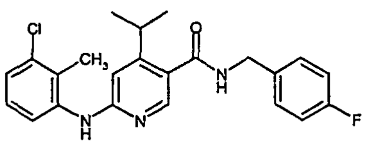 | 6-(3-Chloro-2-methyl-phenylamino)-N-(4-fluoro-benzyl)-4-isopropyl-nicotinamide | B | 3.50min<br>$MH^+$ 412<br>$C_{23}H_{23}{}^{35}ClFN\ _3O$ |
| 29 | 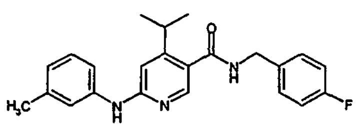 | N-(4-Fluoro-benzyl)-4-isopropyl-6-m-tolylamino-nicotinamide | A | 3.90min<br>$MH^+$ 378<br>$C_{23}H_{24}FN_3O$ |
| 30 | 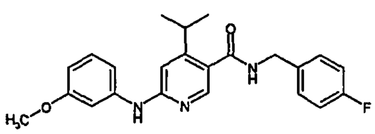 | N-(4-Fluoro-benzyl)-4-isopropyl-6-(3-methoxy-phenylamino)- | A | 3.20min<br>$MH^+$ 394<br>$C_{23}H_{24}FN_3O_2$ |
| | | nicotinamide | | |
| 31 | 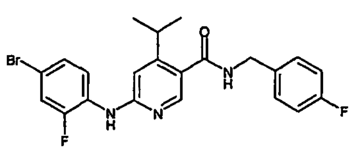 | 6-(4-Bromo-2-fluoro-phenylamino)-N-(4-fluoro-benzyl)-4-isopropyl-nicotinamide | A | 3.70min<br>$MH^+$ 462<br>$C_{22}H_{20}{}^{81}\ BrF_2\ N_3O$ |
| 32 | 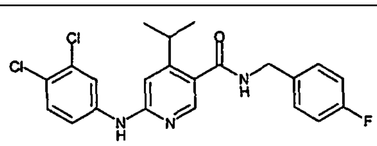 | 6-(3,4-Dichloro-phenylamino)-N-(4-fluoro-benzyl)-4-isopropyl-nicotinamide | B | 3.90min<br>$MH^+$ 433<br>$C_{22}H_{20}\ {}^{35}Cl_2F\ N_3O$ |

**Table 4**

| Compunds of Examples 33 to 42 were prepared in a manner similar to Example 14 (Method A), Example 15 (Method B) or Example 16 (Method C). | | | | |
|---|---|---|---|---|
| Ex. No. | Structure | Name | Metho d | Ret. Time [MH⁺] Molecular Formula |
| 33 | 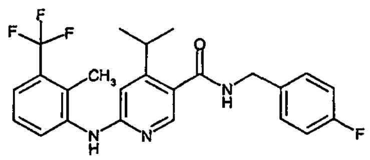 | N-(4-Fluoro-benzyl)-4-isopropyl-6-(2-methyl-3-trifluoromethyl-phenylamino)-nicotinamide | B | 3.60min<br>$MH^+$ 446<br>$C_{24}H_{23}F_4N_3$ O |
| 34 | 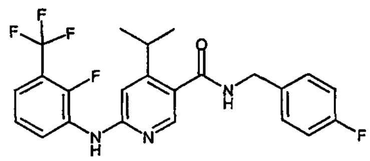 | N-(4-Fluoro-benzyl)-6-(2-fluoro-3-trifluoromethyl-phenylamino)-4-isopropyl-nicotinamide | B | 3.70min<br>$MH^+$ 450<br>$C_{23}H_{20}F_5N_3$ O |
| 35 | 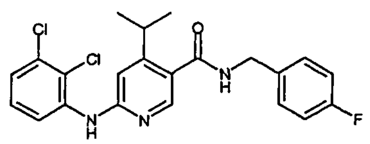 | 6-(2,3-Dichloro-phenylamino)-N-(4-fluoro-benzyl)-4-isopropyl-nicotinamide | B | 3.70min<br>$MH^+$ 433<br>$C_{22}H_{20}{}^{35}Cl_2$ $FN_3O$ |
| 36 | 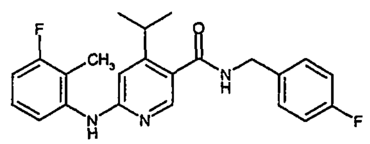 | N-(4-Fluoro-benzyl)-6-(3-fluoro-2-methyl-phenylamino)-4-isopropyl-nicotinamide | B | 3.31 min<br>$M^+$ 396<br>$C_{23}H_{23}F_2N_3O$ |
| 37 | 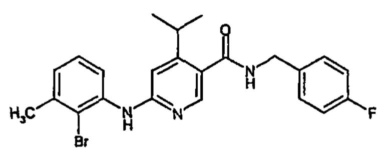 | 6-(2-Bromo-3-methyl-phenylamino)-N-(4-fluoro-benzyl)-4-isopropyl-nicotinamide | B | 3.61 min<br>$MH^+$ 458<br>$C_{23}H_{23}{}^{81}BrF$ $N_3O$ |
| 38 | 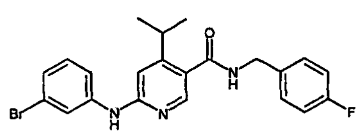 | 6-(3-Bromo-phenylamino)-N-(4-fluoro-benzyl)-4-isopropyl-nicotinamide | A | 3.64min<br>$MH^+$444<br>$C_{22}H_{21}{}^{81}BrF$ $N_3O$ |
| 39 | 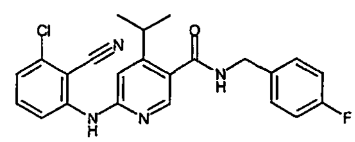 | 6-(3-Chloro-2-cyano-phenylamino)-N-(4-fluoro-benzyl)-4-isopropyl-nicotinamide | C | 3.39min<br>$MH^+$ 423<br>$C_{23}H_{20}{}^{35}ClF$ $N_4O$ |
| 40 | 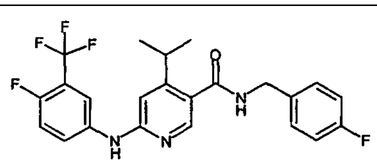 | N-(4-Fluoro-benzyl)-6-(4-fluoro-3-trifluoromethyl-phenylamino)-4-isopropyl-nicotinamide | B | 3.71 min<br>$MH^+$ 450<br>$C_{23}H_{20}F_5N_3$ O |

(continued)

| Compunds of Examples 33 to 42 were prepared in a manner similar to Example 14 (Method A), Example 15 (Method B) or Example 16 (Method C). | | | | |
|---|---|---|---|---|
| Ex. No. | Structure | Name | Metho d | Ret. Time [$MH^+$] Molecular Formula |
| 41 | 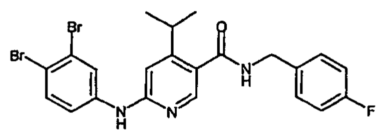 | 6-(3,4-Dibromo-phenylamino)-N-(4-fluoro-benzyl)-4-isopropyl-nicotinamide | B | 3.90min $MH^+$ 524 $C_{22}H_{20}{}^{81}Br_2FN_3O$ |
| 42 | 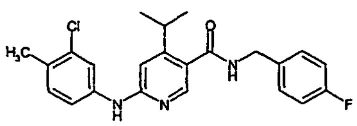 | 6-(3-Chloro-4-methyl-phenylamino)-N-(4-fluoro-benzyl)-4.-isopropyl-nicotinamide | B | 3.74min $MH^+$ 412 $C_{23}H_{23}{}^{35}ClFN_3O$ |

**Example 43: 6-(3-Chloro-phenylamino)-N-(1H-imidazol-2-ylmethyl)-4-trifluoromethyl-nicotinamide**

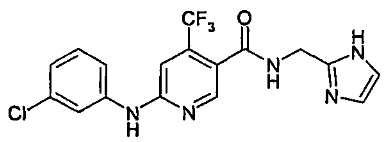

PS-carbodiimide (0.31 g, 0.4 mmol, loading 1.31 mmol/g, ex Argonaut Technologies) and 1-hydroxy-7-azabenzotriazole (0.046 g, 0.34 mmol) were added to a solution of 6-(3-chlorophenylamino)-4-(trifluoromethyl)-nicotinic acid (Description 7) (0.07 g, 0.22 mmol) in dry dichloromethane (3 mL) and the mixture was stirred at room temperature overnight. The resin was filtered and washed repeatedly with dichloromethane, the solvent was then removed in vacuo. The solid residue was dissolved in anhydrous N-methylpyrrolidone (1 mL) and 2-aminomethyl imidazole (19 mg, 0.22 mmol) was added. The solution was heated in a sealed tube under microwave irradiation for 30 min at 140˚C (power=20-30 W). The reaction mixture was diluted with dichloromethane, washed with an aqueous solution of 10% $K_2CO_3$, dried over magnesium sulphate and evaporated under reduced pressure. Chromatographic purification through preparative HPLC on a Symmetry $C_{18}$ column, by gradient elution with a solvent system water / TFA 99.9:0.1 respectively (A) and $CH_3CN$ / TFA 99.9:0.1 respectively (B) with the following gradient: 5% B (3 min); 5% B $\rightarrow$ 95 % B (11 min); 95 % B (1 min); 95 % B $\rightarrow$ 5 % B (2 min) afforded the title compound as its trifluoroacetate salt, which was suspended in dichloromethane and treated with 0.5 N NaOH. The organic layer was dried over $Na_2SO_4$ and evaporated under reduced pressure to give the title compound (50 mg, yield=57%).
$^1H$ NMR (300 MHz, DMSO-$d_6$) $\delta$: 9.90 (s, 1 H); 9.01 (t br, 1 H); 8.58 (s, 1 H); 8.04 (t, 1 H); 7.49 (ddd, 1 H); 7.34 (dd, 1H); 7.17 (s, 1H); 7.06 (m, 2H); 7.04 (ddd, 1 H); 4.48 (d, 2H).
MS m/z (ESI+): AQA; Spray 3.5kV; Skimmer 30V; Probe 250 ˚C: 396 (MH+).

**Table 5.**

Compounds of Examples 44 to 58 were prepared in a manner simmilar to Description 13 (Method A) or Description 14 (Method B).

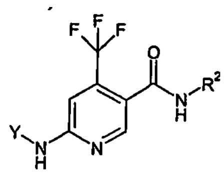

| Ex No | Chemical name | Method | Y | $R^2$ | $^1H$ NMR (Solvent) ppm and/or MS |
|---|---|---|---|---|---|
| 44 | N-(4-Fluoro-benzyl)-6-(3-chloro-phenylamino)-4-trifluoromethyl-nicotinamide | B | 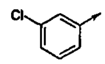 | 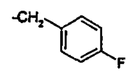 | $^1H$ NMR (300 MHz, DMSO-$d_6$) δ: 9.89 (s, 1 H); 9.02 (t br, 1 H); 8.47 (s, 1H); 8.02 (dd, 1 H); 7.50 (dd, 1 H); 7.36 (m, 3H); 7.16 (m, 3H); 7.03 (dd, 1H); 4.42 (d, 2H). ESI Pos: AQA; Spray 3 kV; Source 20 V; Probe 250 ˚C: 424 (MH+). |
| 45 | N-(2-Fluoro-pyridin-4-ylmethyl)-6-(3-chloro-phenylamino)-4-trifluoromethyl-nicotinamide | B | 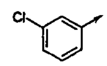 | 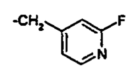 | ESI Pos: AQA; Spray 3.5kV; Skimmer 30V; Probe 250˚C: 426 (MH+). |
| 46 | N-(4-Fluoro-benzyl)-6-(3-bromo-phenylamino)-4-trifluoromethyl-nicotinamide | B | 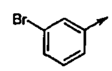 | 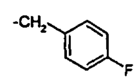 | ESI Pos: AQA; Spray 3.5kV; Skimmer 30V; Probe 250˚C: 468 (MH+). |
| 47 | N-(2-Fluoro-pyridin-4-ylmethyl)-6-(3-bromo-phenylamino)-4-trifluoromethyl-nicotinamide | B | 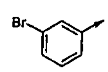 | 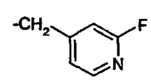 | ESI Pos: AQA; Spray 3.5kV; Skimmer 30V; Probe 250˚C: 469 (MH+). |
| 48 | N-(2-Fluoro-pyridin-4-ylmethyl)-6-(3-fluoro-phenylamino)-4-trifluoromethyl-nicotinamide | B | 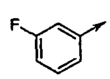 | 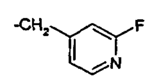 | ESI Pos: AQA; Spray 3.5kV; Skimmer 30V; Probe 250˚C: 408 (MH+). |

(continued)

Compounds of Examples 44 to 58 were prepared in a manner simmilar to Description 13 (Method A) or Description 14 (Method B).

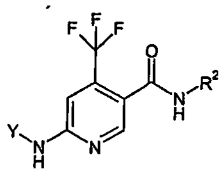

| Ex No | Chemical name | Method | Y | $R^2$ | $^{1}$H NMR (Solvent) ppm and/or MS |
|---|---|---|---|---|---|
| 49 | N-(4-Fluoro-benzyl)-6-(3-fluoro-phenylamino)-4-trifluoromethyl-nicotinamide | B | 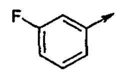 | 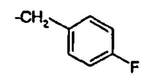 | ESI Pos: AQA; Spray 3.5kV; Skimmer 30V; Probe 250°C: 408 (MH+). |
| 50 | N-(2-Fluoro-pyridin-4-ylmethyl)-6-(2,3-dichloro-phenylamino)-4-trifluoromethyl-nicotinamide | A | 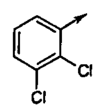 | 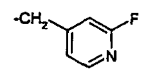 | $^{1}$H NMR (300 MHz, DMSO-$d_6$) δ: 9.36 (s br, 1 H); 9.14 (t br, 1H); 8.45 (s, 1 H); 8.20 (d, 1 H); 7.91 (dd, 1 H); 7.43-7.28 (m, 4H); 7.07 (s br, 1 H); 4.51 (d, 2H). EI+; TSQ 700; source 180°C; 70 V; 200 uA: 458 ($M^{+}$.), 423, 332, 269, 236. |
| 51 | N-(2-Fluoro-pyridin-4-ylmethyl)-6-(2,4-dichloro-phenylamino)-4-trifluoromethyl-nicotinamide | B | 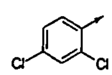 | 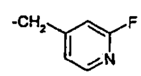 | ESI Pos: AQA; Spray 3.5kV; Skimmer 30V; Probe 250°C: 460 (MH+). |
| 52 | N-(4-Fluoro-benzyl)-6-(2,4-dichloro-phenylamino)-4-trifluoromethyl-nicotinamide | B | 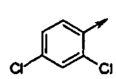 | 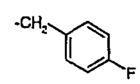 | ESI Pos: AQA; Spray 3.5kV; Skimmer 30V; Probe 250°C: 459 (MH+). |
| 53 | N-(4-Fluoro-benzyl)-6-(3,5-dichloro-phenylamino)-4-trifluoromethyl-nicotinamide | B | 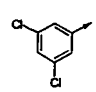 | 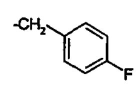 | ESI Pos: AQA; Spray 3.5kV; Skimmer 30V; Probe 250°C: 459 (MH+). |

(continued)

Compounds of Examples 44 to 58 were prepared in a manner simmilar to Description 13 (Method A) or Description 14 (Method B).

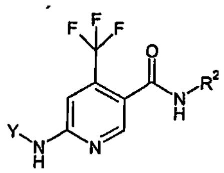

| Ex No | Chemical name | Method | Y | $R^2$ | $^1$H NMR (Solvent) ppm and/or MS |
|---|---|---|---|---|---|
| 54 | N-(2-Fluoro-pyridin-4-ylmethyl)-6-(3,5-dichloro-phenylamino)-4-trifluoromethyl-nicotinamide | B | 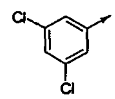 | 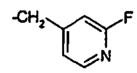 | ESI Pos: AQA; Spray 3.5kV; Skimmer 30V; Probe 250°C: 459 (MH+). |
| 55 | N-(4-Fluoro-benzyl)-6-(3,4-dichloro-phenylamino)-4-trifluoromethyl-nicotinamide | B | 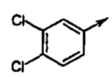 | 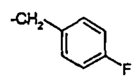 | $^1$H NMR (300 MHz, DMSO-$d_6$) δ: 10.01 (s br, 1H); 9.03 (t br, 1 H); 8.49 (s, 1 H); 8.22 (dd, 1 H); 7.55 (ABq, 2H); 7.37 (m, 2H); 7.21-7.12 (m, 3H); 4.43 (d, 2H). ESI Pos: AQA; Spray 3 kV; Source 20 V; Probe 250°C: 458 (MH+). |
| 56 | N-(2-Fluoro-pyridin-4-ylmethyl)-6-(3,4-dichloro-phenylamino)-4-trifluoromethyl-nicotinamide | B | 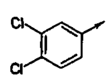 | 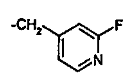 | ESI Pos: AQA; Spray 3.5kV; Skimmer 30V; Probe 250°C: 460 (MH+). |
| 57 | N-(Pyridin-4-ylmethyl)-6-(2-methyl-5-chloro-phenylamino)-4-trifluoromethyl-nicotinamide | A | 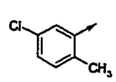 | 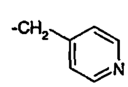 | $^1$H NMR (300 MHz, DMSO-$d_6$) δ: 9.07 (t br, 1 H); 8.96 (s, 1 H); 8.51 (m. 2H); 8.45 (m, 1 H); 7.87 (d, 1 H); 7.33 (m, 2H); 7.30 (s, 1 H); 7.26 (d, 1 H); 7.08 (dd, 1 H); 4.46 (d, 2H); 2.24 (s, 3H). EI+; TSQ 700; source 180°C; 70 V; 200 uA: 420 ($M^+$.), 328. |

(continued)

Compounds of Examples 44 to 58 were prepared in a manner simmilar to Description 13 (Method A) or Description 14 (Method B).

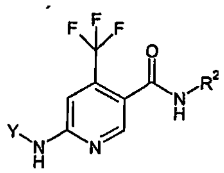

| Ex No | Chemical name | Method | Y | $R^2$ | $^1$H NMR (Solvent) ppm and/or MS |
|---|---|---|---|---|---|
| 58 | N-(Pyridin-4-ylmethyl)-6-(2-methyl-4-chloro-phenylamino)-4-trifluoromethyl-nicotinamide | A | 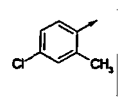 | 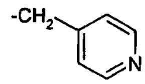 | $^1$H NMR (300 MHz, DMSO-$d_6$) δ: 9.04 (t br, 1 H); 8.98 (s, 1 H); 8.51 (m, 2H); 8.37 (s, 1 H); 7.63 (d, 1H); 7.34 (d, 1H); 7.31 (m, 2H); 7.25 (dd, 1 H); 7.16 (s, 1 H); 4.45 (d, 2H); 2.23 (s, 3H). EI+; TSQ 700; source 180˚C; 70 V; 200 uA: 420 ($M^+$.); 405; 313. |

**Table 6.**

Compounds of Examples 59 to 67 were prepared in a manner similar to that of Description 14 (Method B) and Example 43 (Method D) above.

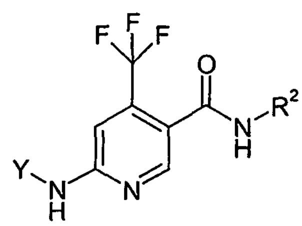

| Ex. No. | Chemical name | Method | Y | $R^2$ | $^1$H NMR (Solvent) ppm and/or MS |
|---|---|---|---|---|---|
| 59 | N-Phenethyl-6-(3-chloro-phenylamino)-4-trifluoromethyl-nicotinamide | B | 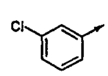 | 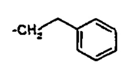 | ESI Pos: AQA; Spray 3.5kV; Skimmer 30V; Probe 250˚C: 420 |

(continued)

Compounds of Examples 59 to 67 were prepared in a manner similar to that of Description 14 (Method B) and Example 43 (Method D) above.

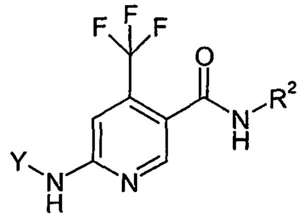

| Ex. No. | Chemical name | Method | Y | $R^2$ | $^1$H NMR (Solvent) ppm and/or MS |
|---|---|---|---|---|---|
| 60 | N-(5-Methyl-[1,3,4]oxadiazol-2-ylmethyl)-6-(3-chloro-phenylamino)-4-trifluoromethyl-nicotinamide | D | 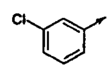 | 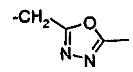 | ESI Pos: AQA; Spray 3.5kV; Skimmer 30V; Probe 250˚C: 412 (MH+). |
| 61 | N-Phenethyl-6-(3-bromo-phenylamino)-4-trifluoromethyl-nicotinamide | B | 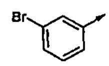 | 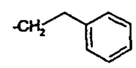 | ESI Pos: AQA; Spray 3.5kV; Skimmer 30V; Probe 250˚C: 464 (MH+). |
| 62 | N-Phenethyl-6-(3-fluoro-phenylamino)-4-trifluoromethyl-nicotinamide | B | 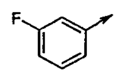 | 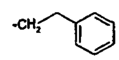 | ESI Pos: AQA; Spray 3.5kV; Skimmer 30V; Probe 250˚C: 404 (MH+). |
| 63 | N-(5-Methyl-[1,3,4]oxadiazol-2-ylmethyl)-6-(2,4-dichloro-phenylamino)-4-trifluoromethyl-nicotinamide | D | 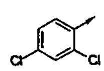 | 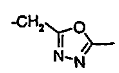 | ESI Pos: AQA; Spray 3.5kV; Skimmer 30V; Probe 250˚C: 446 (MH+). |
| 64 | N-(5-Methyl-4H-[1,2,4]triazol-3-ylmethyl)-6-(2,4-dichloro-phenylamino)-4-trifluoromethyl-nicotinamide | D | 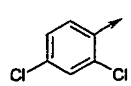 | 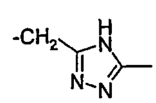 | ESI Pos: AQA; Spray 3.5kV; Skimmer 30V; Probe 250˚C: 445 (MH+). |

(continued)

Compounds of Examples 59 to 67 were prepared in a manner similar to that of Description 14 (Method B) and Example 43 (Method D) above.

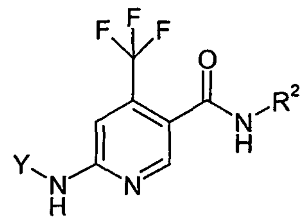

| Ex. No. | Chemical name | Method | Y | $R^2$ | $^1$H NMR (Solvent) ppm and/or MS |
|---|---|---|---|---|---|
| 65 | N-Phenethyl-6-(3,4-dichloro-phenylamino)-4-trifluoromethyl-nicotinamide | B | 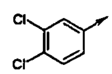 | 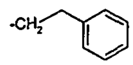 | ESI Pos: AQA; Spray 3.5kV; Skimmer 30V; Probe 250˚C: 454 (MH+). |
| 66 | N-Phenethyl-6-(3,5-dichloro-phenylamino)-4-trifluoromethyl-nicotinamide | B | 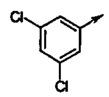 | 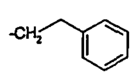 | ESI Pos: AQA; Spray 3.5kV; Skimmer 30V; Probe 250˚C: 454 (MH+). |

**Example 67: 6-(3-Chloro-phenylamino)-4-isopropyl-N-pyrimidin-4-ylmethyl-nicotinamide**

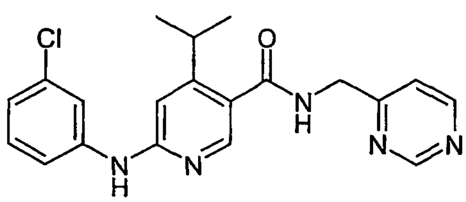

To a solution of 6-(3-chloro-phenylamino)-4-isopropyl-nicotinic acid (Description 4) (30 mg) in dimethylformamide (1.5 ml) was added successively N-ethylmorpholine (42 μl), pyrimidin-4-yl-methylamine (Ref.: Maury et al., Bull. Soc. Chim. Belg., 91 (2), 153, (1982))(14 mg), 1-hydroxybenzotriazole hydrate (25 mg) and 1-(3-dimethylamino-propyl)-3-ethylcarbodiimide hydrochloride (25 mg). The solution was stirred for 3 h and allowed to stand overnight. Dimethylformamide was removed under reduced pressure and ethyl acetate (5 ml) added. The solution was washed sequentially with 5% sodium bicarbonate solution (3 ml), water (3 ml), brine (2 x 3 ml), dried ($MgSO_4$), and evaporated to afford the title compound (25 mg).

NMR (DMSO-d6) δ 1.18 (6H, d), 3.45 (1 H, m), 4.51 (2H, d), 6.82 (1 H, s), 6.94 (1 H, d), 7.29 (1 H, t), 7.47 (1 H, d), 7.52 (1 H, d), 8.09 (1H. t), 8.36 (1 H, s), 8.77 (1 H, d), 9.04 (1 H, t), 9.13 (1 H, s), 9.48 (1 H, s).

LC/MS t = 2.9 min, [$MH^+$] 382 consistent with molecular formula $C_{20}H_{20}{}^{35}ClN_5O$

**Example 68: 6-(3-Chloro-phenylamino)-4-isopropyl-N-pyrazin-2-ylmethyl-nicotinamide**

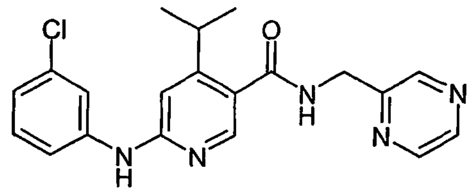

In a manner similar to Example 67, 6-(3-chloro-phenylamino)-4-isopropyl-nicotinic acid (Description 4) (30 mg) and pyrazin-2-yl-methylamine (Ref.: Hirschberg and Mattner, J. Med. Chem., 11 (4), 911, (1968)) (14 mg) afforded the title compound (28 mg).
LC/MS t = 3.0 min, [$MH^+$] 382 consistent with molecular formula $C_{20}H_{20}{}^{35}ClN_5O$

**Example 69: 6-(3-Chloro-phenylamino)-4-isopropyl-N-(6-methyl-pyridin-3-ylmethyl)-nicotinamide**

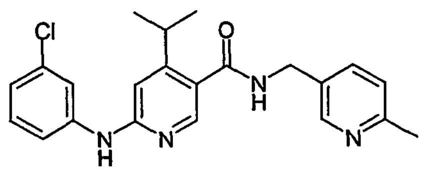

In a manner similar to Example 67, 6-(3-chloro-phenylamino)-4-isopropyl-nicotinic acid (Description 4) (30 mg) and (6-methyl-pyridin-3-yl)-methylamine dihydrochloride (Description 15) (24.5 mg) afforded the title compound (17 mg).
LC/MS t = 2.6 min, [$MH^+$] 395 consistent with molecular formula $C_{22}H_{23}{}^{35}ClN_4O$.

**Table 7**

| Compounds of Examples 70 to 77 in the following tables were prepared in the same manner as for Example 14 - Method A, or as for Example 15- Method B. | | | | |
|---|---|---|---|---|
| Example No. | Chemical Structure | Name | Method | Ret. Time [MH+] Molecular Formula |
| 70 | 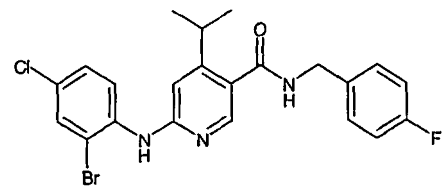 | 6-(2-Bromo-4-chloro-phenylamino)-N-(4-fluoro-benzyl)-4-isopropyl-nicotinamide | A | 3.8<br>476<br>$C_{22}H_{20}{}^{79}Br{}^{35}ClFN_3O$ |
| 71 | 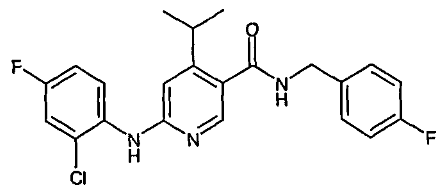 | 6-(2-Chloro-4-fluoro-phenylamino)-N-(4-fluoro-benzyl)-4-isopropyl-nicotinamide | A | 3.4<br>416<br>$C_{22}H_{20}{}^{35}ClF_2N_3O$ |
| 72 | 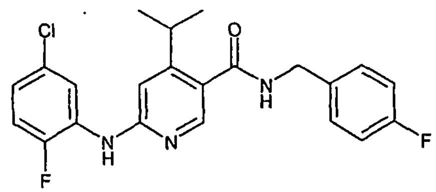 | 6-(5-Chloro-2-fluoro-phenylamino)-N-(4-fluoro-benzyl)-4-isopropyl-nicotinamide | A | 3.6<br>416<br>$C_{22}H_{20}{}^{35}ClF_2N_3O$ |
| 73 | 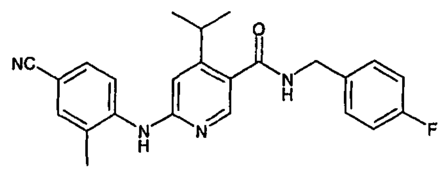 | 6-(4-Cyano-2-methyl-phenylamino)-N-(4-fluoro-benzyl)-4-isopropyl-nicotinamide | A | 3.3<br>403<br>$C_{24}H_{23}FN_4O$ |
| 74 | 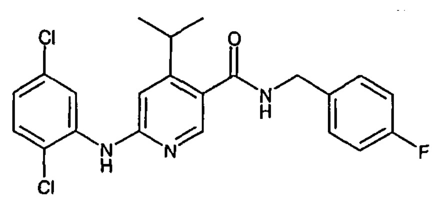 | 6-(2,5-Dichloro-phenylamino)-N-(4-fluoro-benzyl)-4-isopropyl-nicotinamide | A | 3.2<br>432<br>$C_{22}H_{20}{}^{35}Cl_2FN_3O$ |

(continued)

| Compounds of Examples 70 to 77 in the following tables were prepared in the same manner as for Example 14 - Method A, or as for Example 15- Method B. | | | | |
|---|---|---|---|---|
| Example No. | Chemical Structure | Name | Method | Ret. Time [MH+] Molecular Formula |
| 75 | 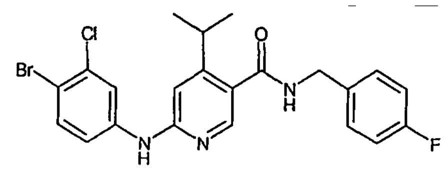 | 6-(4-Bromo-3-chloro-phenylamino)-N-(4-fluoro-benzyl)-4-isopropyl-nicotinamide | A | 3.9<br>476<br>$C_{22}H_{20}{}^{79}Br^{35}ClFN_3O$ |
| 76 | 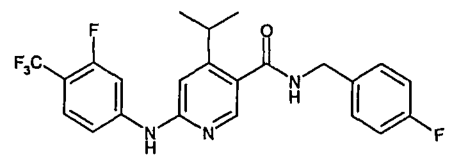 | N-(4-fluoro-benzyl)-6-(3-Fluoro-4-trifluormethyl-phenylamino)- 4-isopropyl-nicotinamide | B | 3.8<br>450<br>$C_{23}H_{20}F_5N_3O$ |
| 77 | 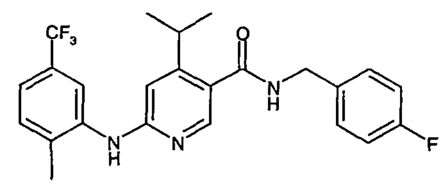 | N-(4-fluoro-benzyl)- 4-isopropyl-6-(2-methyl-5-trifluoromethyl-phenylamino)-nicotinamide | A, but crude product purified using MDAP | 3.7<br>446<br>$C_{24}H_{23}F_4N_3O$ |

**Example 78. 6-(3-Chloro-phenylamino)-N-(pyridin-4-ylmethyl)-2-trifluoromethyl-nicotinamide**

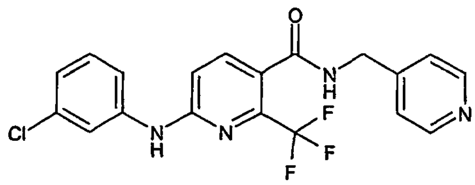

N-methyl morpholine (0.25 mL, 2.27 mmol, 4.0 eq), 1-hydroxy-benzotriazole (120 mg, 0.88 mmol, 1.5 eq), N-(3-dimethylaminopropyl)-N-ethylcarbodiimide hydrochloride (130 mg, 0.68 mmol, 1.2 eq) and 4-(aminomethyl)-pyridine (0.076 mL, 0.73 mmol, 1.3 eq) were subsequently added to a solution of 6-(3-chloro-phenylamino)-2-trifluoromethyl-nicotinic acid hydrochloride (Description 20) (200 mg, 0.56 mmol, 1.0 eq) in anhydrous DMF (10 mL) and stirred at ambient temperature for 16h. After evaporation of the solvent in vacuo, the mixture was diluted with ethyl acetate (10 mL) and washed subsequently with a saturated aqueous solution of $NaHCO_3$ (20 mL x 2 times) and brine (20 mL). The organic phase was dried over sodium sulphate and concentrated in vacuo to afford a black residue that was purified by flash chromatography (silica gel, eluent gradient: from hexane / ethyl acetate 7:3 to hexane / ethyl acetate 6:4). The title compound was obtained as a grey solid (130 mg, yield = 60%).
EI; TSQ 700; source 180 C; 70 V; 200 uA: 406($M^+$.), 337, 299.
$^1$H NMR (300 MHz, DMSO-$d_6$) δ: 9.86(s, 1H); 9.06(t br, 1 H); 8.53(m, 2H); 8.02(dd, 1 H); 7.85(d, 1H); 7.52(ddd, 1 H); 7.24(dd, 1 H); 7.33(m, 2H); 7.13(d, 1 H); 7.02(ddd, 1 H); 4.46(d, 2H).

Example 79 in was prepared as described for the Example 78, from the appropriate starting materials via similar intermediates, prepared in a similar manner to the intermediates described in Descriptions 16 to 20.

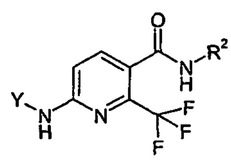

| Ex. No | Chemical Name | Y | $R^2$ | $^1$H NMR (Solvent) ppm and/or MS |
|---|---|---|---|---|
| 79 | 6-(3-Chloro-phenylamino)-N-benzyl-2-trifluoromethyl-nicotinamide | 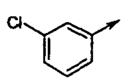 | 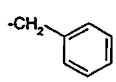 | EI; TSQ 700; source 180 C; 70 V; 200 uA: 405 ($M^+$.), 336, 299. $^1$H NMR (300 MHz, DMSO-$d_6$) δ: 9.83(s, 1 H); 8.94(t br, 1 H); 8.02(dd, 1 H); 7.79(d, 1H); 7.52(dd, 1H); 7.39-7.22(m, 6H); 7.10(d, 1H);7.00 (dd, 1 H); 4.43(d, 2H) |

Formulations for pharmaceutical use incorporating compounds of the present invention can be prepared in various forms and with numerous excipients. Examples of such formulations are given below.

**Example 80: Inhalant Formulation**

A compound of formula (I) or a pharmaceutically acceptable derivative thereof, (1 mg to 100 mg) is aerosolized from a metered dose inhaler to deliver the desired amount of drug per use.

**Example 81: Tablet Formulation**

| Tablets/Ingredients | | Per Tablet |
|---|---|---|
| 1. | Active ingredient (Compound of formula (I) or pharmaceutically acceptable derivative) | 40 mg |
| 2. | Corn Starch | 20 mg |
| 3. | Alginic acid | 20 mg |
| 4. | Sodium Alginate | 20 mg |
| 5. | Mg stearate | 1.3 mg |

Procedure for tablet formulation:

Ingredients 1, 2, 3 and 4 are blended in a suitable mixer/blender. Sufficient water is added portion-wise to the blend with careful mixing after each addition until the mass is of a consistency to permit its conversion to wet granules. The wet mass is converted to granules by passing it through an oscillating granulator using a No. 8 mesh (2.38 mm) screen. The wet granules are then dried in an oven at 140˚F (60˚C) until dry. The dry granules are lubricated with ingredient No. 5, and the lubricated granules are compressed on a suitable tablet press.

**Example 83: Parenteral Formulation**

A pharmaceutical composition for parenteral administration is prepared by dissolving an appropriate amount of a compound of formula (I) in polyethylene glycol with heating. This solution is then diluted with water for injections Ph Eur. (to 100 ml). The solution is then rendered sterile by filtration through a 0.22 micron membrane filter and sealed in sterile containers.

**Claims**

**1.** A compound of formula (I):

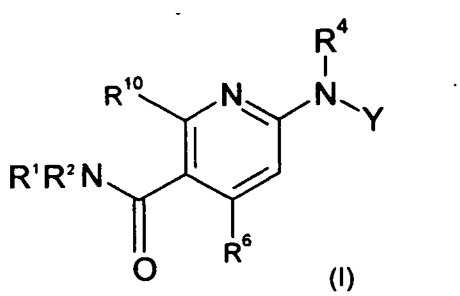

(I)

wherein:

Y is phenyl, substituted with one, two or three substituents;
$R^1$ is selected from hydrogen, $C_{1-6}$ alkyl, $C_{3-7}$ cycloalkyl, or halosubstituted$C_{1-6}$ alkyl;
$R^2$ is $(CH_2)mR^3$;
$R^3$ is an unsubstituted or substituted 5- to 6- membered aromatic heterocyclyl group, or group A:

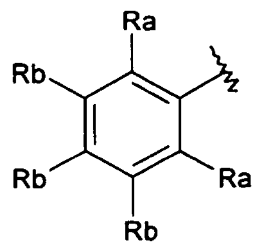

(A)

$R^4$ is selected from hydrogen, $C_{1-6}$ alkyl, $C_{3-7}$ cycloalkyl, or halosubstituted$C_{1-6}$ alkyl, $COCH_3$, and $SO_2Me$;
$R^6$ is unsubstituted or substituted $(C_{1-6})$alkyl or chloro and $R^{10}$ is hydrogen or $R^{10}$ is unsubstituted or substituted $(C_{1-6})$alkyl or chloro and $R^6$ is hydrogen;
Ra can be independently selected from hydrogen, fluoro, chloro or trifluoromethyl;
Rb can independently be selected from hydrogen, $C_{1-6}$ alkyl, $C_{1-6}$ alkoxy, halo substituted C $_{1-6}$ alkoxy, hydroxy, cyano, halo, sulfonyl, $CONH_2$, COOH, $SO_2CH_3$, $NHCOCH_3$, $NHSO_2CH_3$ and $CONHCH_3$;
m is 1 or 2;
or a pharmaceutically acceptable derivative thereof.

**2.** A compound as claimed in claim 1 wherein the compound is of formula (Ia):

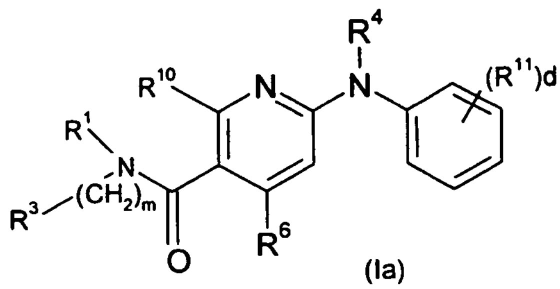

(Ia)

wherein

$R^1$ is selected from hydrogen, $C_{1-6}$ alkyl, $C_{3-7}$ cycloalkyl, or halosubstituted$C_{1-6}$ alkyl;
$R^3$ is furanyl, dioxalanyl, pyrrolyl, oxazolyl, thiazolyl, imidazolyl, oxadiazolyl, thiadiazolyl, triazolyl, triazinyl, isothiazolyl, isoxazolyl, thienyl, pyrazolyl, tetrazolyl, pyridyl, pyrizinyl, pyrimidinyl, pyrazinyl, triazinyl, or tetrazinyl which can be unsubstituted or substituted with 1, 2 or 3 substitutents selected from $C_{1-6}$ alkyl, $C_{1-6}$ alkoxy, halosubstituted$C_{1-6}$ alkoxy, halosubstituted$C_{1-6}$ alkyl, hydroxy, cyano, halo, sulfonyl, $CONH_2$ and COOH , or $R^3$ is group A:

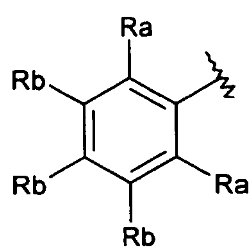

(A)

$R^4$ is selected from hydrogen, $C_{1-6}$ alkyl, $C_{3-7}$ cycloalkyl, or halosubstituted$C_{1-6}$ alkyl, $COCH_3$, and $SO_2Me$;
$R^6$ is unsubstituted or substituted $(C_{1-6})$alkyl, chloro and $R^{10}$ is hydrogen or $R^{10}$ is unsubstituted or substituted

($C_{1-6}$)alkyl or chloro and $R^6$ is hydrogen;
Ra can be independently selected from hydrogen, fluoro, chloro or trifluoromethyl;
Rb can independently be selected from hydrogen, $C_{1-6}$ alkyl, $C_{1-6}$ alkoxy, halosubstitutedC $_{1-6}$ alkoxy, hydroxy, cyano, halo, sulfonyl, $CONH_2$, COOH, $SO_2CH_3$, $NHCOCH_3$, $NHSO_2CH_3$ and $CONHCH_3$;
$R^{11}$ is $C_{1-6}$ alkyl, halosubstituted$C_{1-6}$ alkyl, $C_{1-6}$ alkoxy, hydroxy, cyano, halo, $C_{1-6}$ alkylsulfonyl, $CONH_2$, $NHCOCH_3$, COOH, halosubstituted$C_{1-6}$ alkoxy, $C_{1-6}$alkynyl, $C_{1-6}$alkynyl, $SO_2NR^{8a}R^{8b}$;
d is 1,2, or 3:
m is 1 or 2;
$R^{8a}$ and $R^{8b}$ are independently selected from hydrogen or $C_{1-6}$alkyl;
or a pharmaceutically acceptable derivative thereof.

**3.** A compound as claimed in claim 1 or 2 wherein $R^1$ is hydrogen or $C_{1-6}$alkyl

**4.** A compound as claimed in any one of claims 1 to 3 wherein $R^4$ is hydrogen or methyl.

**5.** A compound as claimed in any precceding claim whereing $R^3$ is selected from group A, pyridinyl, pyrimidinyl, imidazoyl, oxadiazoyl, triazolyl or pyrazinyl.

**6.** A compound as claimed in any preceeding claim selected from 2-(3-Chlorophenylamino)-4-trifluoromethylpyridine-5-carboxylic acid (pyridin-4-ylmethyl) amide, 2-(3-Chlorophenylamino)-4-trifluoromethylpyridine-5-carboxylic acid benzylamide, 6-(3-Chlorophenylamino)-N-(4-fluorobenzyl)-4-isopropyl-nicotinamide, N-Benzyl-6-(3-chloro-phenylamino)-4-isopropyl-nicotinamide, 6-(3-Chloro-phenylamino)-N-(4-cyano-benzyl)-4-isopropyl-nicotinamide, 6-(3-Chloro-phenylamino)-4-isopropyl-N-(4-methoxy-benzyl)-nicotinamide, 6-(3-Chloro-phenylamino)-N-(3,4-difluoro-benzyl)-4-isopropyl-nicotinamide, N-(4-Carbamoyl-benzyl)-6-(3-chloro-phenylamino)-4-isopropyl-nicotinamide, 6-(3-Chloro-phenylamino)-N-(2,4-difluoro-benzyl)-4-isopropyl-nicotinamide, 6-(3-Chloro-phenylamino)-4-isopropyl-N-(4-methanesulfonyl-benzyl)-nicotinamide, N-(4-Acetylamino-benzyl)-6-(3-chloro-phenylamino)-4-isopropyl-nicotinamide, 6-(3-Chloro-phenylamino)-4-isopropyl-N-(4-methane-sulfonylamino-benzyl)-nicotinamide, 6-(3-Chloro-phenylamino)-4-isopropyl-N-(4-methylcarbamoyl-benzyl)-nicotinamide, N-(4-Fluoro-benzyl)-4-isopropyl-6-(3-trifluoromethyl-phenylamino)-nicotinamide, 6-(3-Chloro-4-fluoro-phenylamino)- N-(4-fluoro-benzyl)-4-isopropyl-nicotinamide, 6-(2-Cyano-3-methyl-phenylamino)-N-(4-fluoro-benzyl)-4-isopropyl-nicotinamide, N-(4-Fluoro-benzyl)-6-(3-fluoro-phenylamino)-4-isopropyl-nicotinamide, 6-(4-Cyano-phenylamino)-N-(4-fluoro-benzyl)-4-isopropyl-nicotinamide, 6-(3-Cyano-phenylamino)-N-(4-fluoro-benzyl)-4-isopropyl-nicotinamide, 6-(4-Chloro-2-fluoro-phenylamino)-N-(4-fluoro-benzyl)-4-isopropyl-nicotinamide, 6-(4-Bromo-2-chloro-phenylamino)-N-(4-fluoro-benzyl)-4-isopropyl-nicotinamide, 6-(2,4-Dichloro-phenylamino)-N-(4-fluoro-benzyl)-4-isopropyl-nicotinamide, 6-(3-Chloro-4-cyano-phenylamino)-N-(4-fluoro-benzyl)-4-isopropyl-nicotinamide, 6-(4-Bromo-3-fluoro-phenylamino)-N-(4-fluoro-benzyl)-4-isopropyl-nicotinamide, N-(4-Fluoro-benzyl)-4-isopropyl-6-(3-trifluoromethoxy-phenylamino)-nicotinamide, 6-(3-Chloro-2-fluoro-phenylamino)-N-(4-fluoro-benzyl)-4-isopropyl-nicotinamide, 6-(3-Bromo-2-methyl-phenylamino)-N-(4-fluoro-benzyl)-4-isopropyl-nicotinamide, 6-(3-Chloro-2-methyl-phenylamino)-N-(4-fluoro-benzyl)-4-isopropyl-nicotinamide, N-(4-Fluoro-benzyl)-4-isopropyl-6-m-tolylamino-nicotinamide, N-(4-Fluoro-benzyl)-4-isopropyl-6-(3-methoxy-phenylamino)-nicotinamide, 6-(4-Bromo-2-fluoro-phenylamino)-N-(4-fluoro-benzyl)-4-isopropyl-nicotinamide, 6-(3,4-Dichloro-phenylamino)-N-(4-fluoro-benzyl)-4-isopropyl-nicotinamide, N-(4-Fluoro-benzyl)-4-isopropyl-6-(2-methyl-3-trifluoromethyl-phenylamino)-nicotinamide, N-(4-Fluoro-benzyl)-6-(2-fluoro-3-trifluoromethyl-phenylamino)-4-isopropyl-nicotinamide, 6-(2,3-Dichloro-phenylamino)-N-(4-fluoro-benzyl)-4-isopropyl-nicotinamide, N-(4-Fluoro-benzyl)-6-(3-fluoro-2-methyl-phenylamino)-4-isopropyl-nicotinamide, 6-(2-Bromo-3-methyl-phenylamino)-N-(4-fluoro-benzyl)-4-isopropyl-nicotinamide, 6-(3-Bromo-phenylamino)- N-(4-fluoro-benzyl)-4-isopropyl-nicotinamide, 6-(3-Chloro-2-cyano-phenylamino)-N-(4-fluoro-benzyl)-4-isopropyl-nicotinamide, N-(4-Fluoro-benzyl)-6-(4-fluoro-3-trifluoromethyl-phenylamino)-4-isopropyl-nicotinamide, 6-(3,4-Dibromo-phenylamino)-N-(4-fluoro-benzyl)-4-isopropyl-nicotinamide, 6-(3-Chloro-4-methyl-phenylamino)-N-(4-fluoro-benzyl)-4-isopropyl-nicotinamide, 6-(3-Chloro-phenylamino)-N-(1H-imidazol-2-ylmethyl)-4-trifluoromethyl-nicotinamide, N-(4-Fluoro-benzyl)-6-(3-chloro-phenylamino)-4-trifluoromethyl-nicotinamide, N-(2-Fluoro-pyridin-4-ylmethyl)-6-(3-chloro-phenylamino)-4-trifluoromethyl-nicotinamide, N-(4-Fluoro-benzyl)-6-(3-bromo-phenylamino)-4-trifluoromethyl-nicotinamide, N-(2-Fluoro-pyridin-4-ylmethyl)-6-(3-bromo-phenylamino)-4-trifluoromethyl-nicotinamide, N-(2-Fluoro-pyridin-4-ylmethyl)-6-(3-fluoro-phenylamino)-4-trifluoromethyl-nicotinamide, N-(4-Fluoro-benzyl)-6-(3-fluoro-phenylamino)-4-trifluoromethyl-nicotinamide, N-(2-Fluoro-pyridin-4-ylmethyl)-6-(2,3-dichloro-phenylamino)-4-trifluoromethyl-nicotinamide, N-(2-Fluoro-pyridin-4-ylmethyl)-6-(2,4-dichloro-phenylamino)-4-trifluoromethyl-nicotinamide, N-(4-Fluoro-benzyl)-6-(2,4-dichloro-phenylamino)-4-trifluoromethyl-nicotinamide, N-(4-Fluoro-benzyl)-6-(3,5-dichloro-phenylamino)-4-trifluoromethyl-nicotinamide, N-(2-Fluoro-pyridin-4-ylmethyl)-6-(3,5-dichloro-phenylamino)-4-trifluoromethyl-nicotinamide, N-(4-Fluoro-benzyl)-6-(3,4-dichloro-phenylamino)-4-trifluoromethyl-nicotinamide, N-(2-Fluoro-pyridin-4-ylmethyl)-6-(3,4-dichloro-phenylamino)-4-trifluoromethyl-nicotinamide, N-(Pyridin-4-ylmethyl)-6-(2-methyl-5-chloro-phenylamino)-4-trifluoromethyl-nicotinamide, N-(Pyridin-4-ylmethyl)-6-(2-methyl-4-chloro-phenylamino)-4-trifluoromethyl-nicotinamide, N-Phenethyl-6-(3-chloro-phenylamino)-4-trifluoromethyl-nicotinamide, N-(5-Methyl-[1,3,4]oxadiazol-2-ylmethyl)-6-(3-chloro-phenylamino)-4-trifluoromethyl-nicotinamide, N-Phenethyl-6-(3-bromo-phenylamino)-4-trifluoromethyl-nicotinamide, N-Phenethyl-6-(3-fluoro-phenylamino)-4-trifluoromethyl-nicotinamide, N-(5-Methyl-[1,3,4]oxadiazol-2-ylmethyl)-6-(2,4-dichloro-phenylamino)-4-trifluoromethyl-nicotinamide, N-(5-Methyl-4H-[1,2,4]triazol-3-ylmethyl)-6-(2,4-dichloro-phenylamino)-4-trifluoromethyl-nicotinamide, N-Phenethyl-6-(3,4-dichloro-phenylamino)-4-trifluoromethyl-nicotinamide, N-Phenethyl-6-(3,5-dichloro-phenylamino)-4-trifluoromethyl-nicotinamide, 6-(3-Chloro-phenylamino)-4-isopropyl-N-pyrimidin-4-ylmethyl-nicotinamide, 6-(3-Chloro-phenylamino)-4-isopropyl-N-pyrazin-2-ylmethyl-nicotinamide, 6-(3-Chloro-phenylamino)-4-isopropyl-N-(6-methyl-pyridin-3-ylmethyl)-nicotinamide, 6-(2-Bromo-4-chloro-phenylamino)-N-(4-fluoro-benzyl)-4-isopropyl-nicotinamide, 6-(2-Chloro-4-fluoro-phenylamino)-N-(4-fluoro-benzyl)-4-isopropyl-nicotinamide, 6-(5-Chloro-2-fluoro-phenylamino)-N-(4-fluoro-benzyl)-4-isopropyl-nicotinamide, 6-(4-Cyano-2-methyl-phenylamino)-N-(4-fluoro-benzyl)-4-isopropyl-nicotinamide, 6-(2,5-Dichloro-phenylamino)-N-(4-fluoro-benzyl)-4-isopropyl-nicotinamide, 6-(4-Bromo-3-chloro-phenylamino)-N-(4-fluoro-benzyl)-4-isopropyl-nicotinamide, N-(4-fluoro-benzyl)-6-(3-Fluoro-4-trifluormethyl-phenylamino)- 4-isopropyl-nicotinamide, N-(4-fluoro-benzyl)- 4-isopropyl-6-(2-methyl-5-trifluoromethyl-phenylamino)-nicotinamide, 6-(3-Chloro-phenylamino)-N-(pyridin-4-ylmethyl)-2-trifluoromethyl-nicotinamide, and 6-(3-Chloro-phenylamino)-N-benzyl-2-trifluoromethyl-nicotinamide; or a pharmaceutically acceptable derivative thereof.

**7.** A pharmaceutical composition comprising a compound as claimed in any one of claims 1 to 6 or a pharmaceutically acceptable derivative thereof.

**8.** A pharmaceutical composition as claimed in claim 7 further comprising a pharmaceutical carrier or diluent thereof.

**9.** The use of a therapeutically effective amount of a compound of formula (I) as claimed in any one of claims 1 to 6 or a pharmaceutically acceptable derivative thereof for the manufacture of a medicament for the treatment of an immune disorder, an inflammatory disorder, pain, rheumatoid arthritis, multiple sclerosis, osteoarthritis or osteoporosis.

**10.** The use according to claim 9 wherein the pain is selected from inflammatory pain, viseral pain, cancer pain, neuropathic pain, lower back pain, muscular sceletal, post operative pain, acute pain or migraine.

**11.** A pharmaceutical composition comprising a compound of formula (I) or a pharmaceutically acceptable derivative thereof in which the compound of formula (I) or a pharmaceutically acceptable derivative thereof is present in solid particles in nanoparticulate form, in admixture with one or more pharmaceutically acceptable carriers or excipients.

**12.** The pharmaceutical composition according to claim 11 which is a dired powder obtainable by wet milling solid particles of compound of formula (I) followed by spray-drying the resultant suspension.

**13.** A pharmaceutical composition comprising a compound of formula (I) or a pharmaceutically acceptable derivative thereof together with a further therapeutic agent or agents.

## Patentansprüche

**1.** Verbindung der Formel (I):

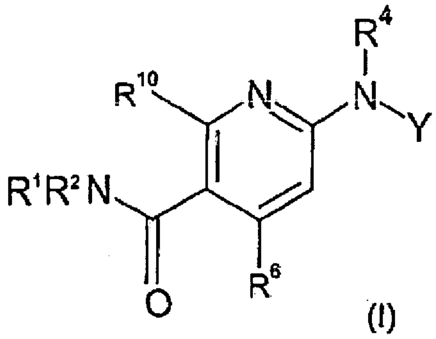

(I)

wobei:

Y Phenyl ist, substituiert mit einem, zwei oder drei Substituenten,
$R^1$ ausgewählt ist aus Wasserstoff, $C_{1-6}$-Alkyl, $C_{3-7}$-Cycloalkyl oder mit Halogen substituiertem $C_{1-6}$-Alkyl;
$R^2$ $(CH_2)mR^3$ ist;
$R^3$ ein unsubstituierter oder substituierter 5- bis 6-gliedriger aromatischer Heterocyclylrest oder Rest A ist:

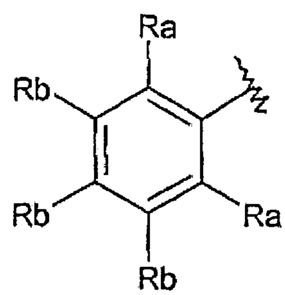

(A)

$R^4$ ausgewählt ist aus Wasserstoff, $C_{1-6}$-Alkyl, $C_{3-7}$-Cycloalkyl oder mit Halogen substituiertem $C_{1-6}$-Alkyl, $COCH_3$ und $SO_2Me$;
$R^6$ unsubstituiertes oder substituiertes $(C_{1-6})$-Alkyl oder Chlor ist und $R^{10}$ Wasserstoff ist oder $R^{10}$ unsubstituiertes oder substituiertes $(C_{1-6})$-Alkyl oder Chlor ist und $R^6$ Wasserstoff ist;
Ra unabhängig ausgewählt sein kann aus Wasserstoff, Fluor, Chlor oder Trifluormethyl;
Rb unabhängig ausgewählt sein kann aus Wasserstoff, $C_{1-6}$-Alkyl, $C_{1-6}$-Alkoxy, mit Halogen substituiertem $C_{1-6}$-Alkoxy, Hydroxy, Cyano, Halogen, Sulfonyl, $CONH_2$, COOH, $SO_2CH_3$, $NHCOCH_3$, $NHSO_2CH_3$ und $CONHCH_3$;
m 1 oder 2 ist;
oder ein pharmazeutisch verträgliches Derivat davon.

**2.** Verbindung gemäß Anspruch 1, wobei die Verbindung eine der Formel (Ia) ist:

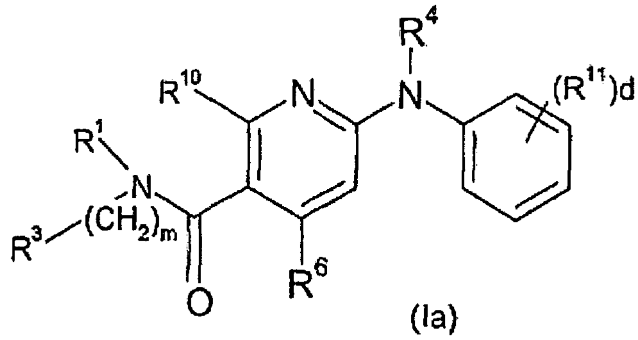

(Ia)

wobei

$R^1$ ausgewählt ist aus Wasserstoff, $C_{1-6}$-Alkyl, $C_{3-7}$-Cycloalkyl oder mit Halogen substituiertem $C_{1-6}$-Alkyl;
$R^3$ Furanyl, Dioxalanyl, Pyrrolyl, Oxazolyl, Thiazolyl, Imidazolyl, Oxadiazolyl, Thiadiazolyl, Triazolyl, Triazinyl, Isothiazolyl, Isoxazolyl, Thienyl, Pyrazolyl, Tetrazolyl, Pyridyl, Pyrizinyl, Pyrimidinyl, Pyrazinyl, Triazinyl oder Tetrazinyl ist, welche unsubstituiert oder mit 1, 2 oder 3 Substituenten substituiert sein können, ausgewählt aus $C_{1-6}$-Alkyl, $C_{1-6}$-Alkoxy, mit Halogen substituiertem $C_{1-6}$-Alkoxy, mit Halogen substituiertem $C_{1-6}$-Alkyl, Hydroxy, Cyano, Halogen, Sulfonyl, $CONH_2$ und COOH, oder $R^3$ ein Rest A ist:

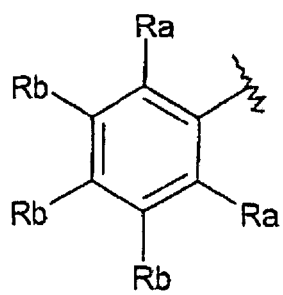

(A)

$R^4$ ausgewählt ist aus Wasserstoff, $C_{1-6}$-Alkyl, $C_{3-7}$-Cycloalkyl oder mit Halogen substituiertem $C_{1-6}$-Alkyl, $COCH_3$ und $SO_2Me$;
$R^6$ unsubstituiertes oder substituiertes ($C_{1-6}$)-Alkyl, Chlor ist und $R^{10}$ Wasserstoff ist oder $R^{10}$ unsubstituiertes oder substituiertes ($C_{1-6}$)-Alkyl oder Chlor ist und $R^6$ Wasserstoff ist; Ra unabhängig ausgewählt sein kann aus Wasserstoff, Fluor, Chlor oder Trifluormethyl;
Rb unabhängig ausgewählt sein kann aus Wasserstoff, $C_{1-6}$-Alkyl, $C_{1-6}$-Alkoxy, mit Halogen substituiertem $C_{1-6}$-Alkoxy, Hydroxy, Cyano, Halogen, Sulfonyl, $CONH_2$, COOH, $SO_2CH_3$, $NHCOCH_3$, $NHSO_2CH_3$ und $CONHCH_3$;
$R^{11}$ $C_{1-6}$-Alkyl, mit Halogen substituiertes $C_{1-6}$-Alkyl, $C_{1-6}$-Alkoxy, Hydroxy, Cyano, Halogen, $C_{1-6}$-Alkylsulfonyl, $CONH_2$, $NHCOCH_3$, COOH, mit Halogen substituiertes $C_{1-6}$-Alkoxy, $C_{1-6}$-Alkinyl, $SO_2N$-$R^{8a}R^{8b}$ ist;
d 1, 2 oder 3 ist;
m 1 oder 2 ist;
$R^{8a}$ und $R^{8b}$ unabhängig ausgewählt sind aus Wasserstoff oder $C_{1-6}$-Alkyl;
oder ein pharmazeutisch verträgliches Derivat davon.

3. Verbindung gemäß Anspruch 1 oder 2, wobei $R^1$ Wasserstoff oder $C_{1-6}$-Alkyl ist.

4. Verbindung gemäß einem der Ansprüche 1 bis 3, wobei $R^4$ Wasserstoff oder Methyl ist.

5. Verbindung gemäß einem der vorstehenden Ansprüche, wobei $R^3$ ausgewählt ist aus dem Rest A, Pyridinyl, Pyrimidinyl, Imidazolyl, Oxadiazolyl, Triazolyl oder Pyrazinyl.

**6.** Verbindung gemäß einem der vorstehenden Ansprüche, ausgewählt aus
2-(3-Chlorphenylamino)-4-trifluormethylpyridin-5-carbonsäure-(pyridin-4-ylmethyl)amid,
2-(3-Chlorphenylamino)-4-trifluormethylpyridin-5-carbonsäurebenzylamid,
6-(3-Chlorphenylamino)-N-(4-fluorbenzyl)-4-isopropylnicotinamid,
N-Benzyl-6-(3-chlorphenylamino)-4-isopropylnicotinamid,
6-(3-Chlorphenylamino)-N-(4-cyanobenzyl)-4-isopropylnicotinamid,
6-(3-Chlorphenylamino)-4-isopropyl-N-(4-methoxybenzyl)nicotinamid,
6-(3-Chlorphenylamino)-N-(3,4-difluorbenzyl)-4-isopropylnicotinamid,
N-(4-Carbamoylbenzyl)-6-(3-chlorphenylamino)-4-isopropylnicotinamid,
6-(3-Chlorphenylamino)-N-(2,4-difluorbenzyl)-4-isopropylnicotinamid,
6-(3-Chlorphenylamino)-4-isopropyl-N-(4-methansulfonylbenzyl)nicotinamid,
N-(4-Acetylaminobenzyl)-6-(3-chlorphenylamino)-4-isopropylnicotinamid,
6-(3-Chlorphenylamino)-4-isopropyl-N-(4-methansulfonylaminobenzyl)nicotinamid,
6-(3-Chlorphenylamino)-4-isopropyl-N-(4-methylcarbamoylbenzyl)nicotinamid,
N-(4-Fluorbenzyl)-4-isopropyl-6-(3-trifluormethylphenylamino)nicotinamid,
6-(3-Chlor-4-fluorphenylamino)-N-(4-fluorbenzyl)-4-isopropylnicotinamid,
6-(2-Cyano-3-methylphenylamino)-N-(4-fluorbenzyl)-4-isopropylnicotinamid,
N-(4-Fluorbenzyl)-6-(3-fluorphenylamino)-4-isopropylnicotinamid,
6-(4-Cyanophenylamino)-N-(4-fluorbenzyl)-4-isopropylnicotinamid,
6-(3-Cyanophenylamino)-N-(4-fluorbenzyl)-4-isopropylnicotinamid,
6-(4-Chlor-2-fluorphenylamino)-N-(4-fluorbenzyl)-4-isopropylnicotinamid,
6-(4-Brom-2-chlorphenylamino)-N-(4-fluorbenzyl)-4-isopropylnicotinamid,
6-(2,4-Dichlorphenylamino)-N-(4-fluorbenzyl)-4-isopropylnicotinamid,
6-(3-Chlor-4-cyanophenylamino)-N-(4-fluorbenzyl)-4-isopropylnicotinamid,
6-(4-Brom-3-fluorphenylamino)-N-(4-fluorbenzyl)-4-isopropylnicotinamid,
N-(4-Fluorbenzyl)-4-isopropyl-6-(3-trifluormethoxyphenylamino)nicotinamid,
6-(3-Chlor-2-fluorphenylamino)-N-(4-fluorbenzyl)-4-isopropylnicotinamid,
6-(3-Brom-2-methylphenylamino)-N-(4-fluorbenzyl)-4-isopropylnicotinamid,
6-(3-Chlor-2-methylphenylamino)-N-(4-fluorbenzyl)-4-isopropylnicotinamid,
N-(4-Fluorbenzyl)-4-isopropyl-6-m-tolylaminonicotinamid,
N-(4-Fluorbenzyl)-4-isopropyl-6-(3-methoxyphenylamino)nicotinamid,
6-(4-Brom-2-fluorphenylamino)-N-(4-fluorbenzyl)-4-isopropylnicotinamid,
6-(3,4-Dichlorphenylamino)-N-(4-fluorbenzyl)-4-isopropylnicotinamid,
N-(4-Fluorbenzyl)-4-isopropyl-6-(2-methyl-3-trifluormethylphenylamino)nicotinamid,
N-(4-Fluorbenzyl)-6-(2-fluor-3-trifluormethylphenylamio)-4-isopropylnicotinamid,
6-(2,3-Dichlorphenylamino)-N-(4-fluorbenzyl)-4-isopropylnicotinamid,
N-(4-Fluorbenzyl)-6-(3-fluor-2-methylphenylamino)-4-isopropylnicotinamid,
6-(2-Brom-3-methylphenylamino)-N-(4-fluorbenzyl)-4-isopropylnicotinamid,
6-(3-Bromphenylamino)-N-(4-fluorbenzyl)-4-iospropylnicotinamid,
6-(3-Chlor-2-cyanophenylamino)-N-(4-fluorbenzyl)-4-isopropylnicotinamid,
N-(4-Fluorbenzyl)-6-(4-fluor-3-trifluormethylphenylamino)-4-isopropylnicotinamid,
6-(3,4-Dibromphenylamino)-N-(4-fluorbenzyl)-4-isopropylnicotinamid,
6-(3-Chlor-4-methylphenylamino)-N-(4-fluorbenzyl)-4-isopropylnicotinamid,
6-(3-Chlorphenylamino)-N-(1H-imidazol-2-ylmethyl)-4-trifluormethylnicotinamid,
N-(4-Fluorbenzyl)-6-(3-chlorphenylamino)-4-trifluormethylnicotinamid,
N-(2-Fluorpyridin-4-ylmethyl)-6-(3-chlorphenylamino)-4-trifluormethylnicotinamid,
N-(4-Fluorbenzyl)-6-(3-bromphenylamino)-4-trifluormethylnicotinamid,
N-(2-Fluorpyridin-4-ylmethyl)-6-(3-bromphenylamino)-4-trifluormethylnicotinamid,
N-(2-Fluorpyridin-4-ylmethyl)-6-(3-fluorphenylamino)-4-trifluormethylnicotinamid,
N-(4-Fluorbenzyl)-6-(3-fluorphenylamino)-4-trifluormethylnicotinamid,
N-(2-Fluorpyridin-4-ylmethyl)-6-(2,3-dichlorphenylamino)-4-trifluormethylnicotinamid,
N-(2-Fluorpyridin-4-ylmethyl)-6-(2,4-dichlorphenylamino)-4-trifluormethylnicotinamid,
N-(4-Fluorbenzyl)-6-(2,4-dichlorphenylamino)-4-trifluormethylnicotinamid,
N-(4-Fluorbenzyl)-6-(3,5-dichlorphenylamino)-4-trifluormethylnicotinamid,
N-(2-Fluorpyridin-4-ylmethyl)-6-(3,5-dichlorphenylamino)-4-trifluormethylnicotinamid,
N-(4-Fluorbenzyl)-6-(3,4-dichlorphenylamino)-4-trifluormethylnicotinamid,
N-(2-Fluorpyridin-4-ylmethyl)-6-(3,4-dichlorphenylamino)-4-trifluormethylnicotinamid,
N-(Pyridin-4-ylmethyl)-6-(2-methyl-5-chlorphenylamino)-4-trifluormethylnicotinamid,

N-(Pyridin-4-ylmethyl)-6-(2-methyl-4-chlorphenylamino)-4-trifluormethylnicotinamid,
N-Phenethyl-6-(3-chlorphenylamino)-4-trifluormethylnicotinamid,
N-(5-Methyl-[1,3,4]oxadiazol-2-ylmethyl)-6-(3-chlorphenylamino)-4-trifluormethyl-nicotinamid,
N-Phenethyl-6-(3-bromphenylamino)-4-trifluormethylnicotinamid,
N-Phenethyl-6-(3-fluorphenylamino)-4-trifluormethylnicotinamid,
N-(5-Methyl-[1,3,4]oxadiazol-2-ylmethyl)-6-(2,4-dichlorphenylamino)-4-trifluormethylnicotinamid,
N-(5-Methyl-4H-[1,2,4]triazol-3-ylmethyl)-6-(2,4-dichlorphenylamino)-4-trifluormethyl-nicotinamid,
N-Phenethyl-6-(3,4-dichlorphenylamino)-4-trifluormethylnicotinamid,
N-Phenethyl-6-(3,5-dichlorphenylamino)-4-trifluormethylnicotinamid,
6-(3-Chlorphenylamino)-4-isopropyl-N-pyrimidin-4-ylmethylnicotinamid,
6-(3-Chlorphenylamino)-4-isopropyl-N-pyrazin-2-ylmethylnicotinamid,
6-(3-Chlorphenylamino)-4-isopropyl-N-(6-methylpyridin-3-ylmethyl)nicotinamid,
6-(2-Brom-4-chlorphenylamino)-N-(4-fluorbenzyl)-4-isopropylnicotinamid,
6-(2-Chlor-4-fluorphenylamino)-N-(4-fluorbenzyl)-4-isopropylnicotinamid,
6-(5-Chlor-2-fluorphenylamino)-N-(4-fluorbenzyl)-4-isopropylnicotinamid,
6-(4-Cyano-2-methylphenylamino)-N-(4-fluorbenzyl)-4-isopropylnicotinamid,
6-(2,5-Dichlurphenylamino)-N-j4-fluorbenzylj-4-isopropyInicotinamid,
6-(4-Brom-3-chlorphenylamino)-N-(4-fluorbenzyl)-4-isopropylnicotinamid,
N-(4-Fluorbenzyl)-6-(3-fluor-4-trifluormethylphenylaminoj-4-isopropylnicotinamid,
N-(4-Fluorbenzyl)-4-isopropyl-6-(2-methyl-5-trifluormethylphenylamino)nicotinamid,
6-(3-Chlorphenylamino)-N-(pyridin-4-ylmethyl)-2-trifluormethylnicotinamid, und
6-(3-Chlorphenylamino)-N-benzyl-2-trifluormethylnicotinamid
oder ein pharmazeutisch verträgliches Derivat davon.

**7.** Arzneimittel, umfassend eine Verbindung gemäß einem der Ansprüche 1 bis 6 oder ein pharmazeutisch verträgliches Derivat davon.

**8.** Arzneimittel gemäß Anspruch 7, ferner umfassend einen pharmazeutischen Träger oder ein Verdünnungsmittel davon.

**9.** Verwendung einer therapeutisch wirksamen Menge einer Verbindung der Formel (I) gemäß einem der Ansprüche 1 bis 6 oder ein pharmazeutisch verträgliches Derivat davon zur Herstellung eines Medikaments zur Behandlung einer Immunstörung, einer Entzündungsstörung, Schmerz, rheumatoider Arthritis, Multipler Sklerose, Osteoarthritis, oder Osteoporose.

**10.** Verwendung gemäß Anspruch 9, wobei der Schmerz ausgewählt ist aus Entzündungsschmerz, viszeralem Schmerz, Krebsschmerz, neurophatischem Schmerz, Schmerz am unteren Rücken, Schmerzen der Skelettmuskulatur, Schmerz nach Operation, akutem Schmerz oder Migräne.

**11.** Arzneimittel, umfassend eine Verbindung der Formel (I) oder ein pharmazeutisch verträgliches Derivat davon, in welchem die Verbindung der Formel (I) oder ein pharmazeutisch verträgliches Derivat davon in festen Teilchen in Nanoteilchen-Form vorliegt, in Beimischung mit einem oder mehreren pharmazeutisch verträglichen Trägem oder Exzipienten.

**12.** Arzneimittel gemäß Anspruch 11, welches ein getrocknetes Pulver ist, erhältlich durch Nassmahlen von festen Teilchen der Verbindung der Formel (I), gefolgt von Spraytrocknen der erhaltenen Suspension.

**13.** Arzneimittel, umfassend eine Verbindung der Formel (I) oder ein pharmazeutisch verträgliches Derivat davon, zusammen mit einem weiteren therapeutischen Mittel oder Mitteln.

## Revendications

**1.** Composé de formule (I) :

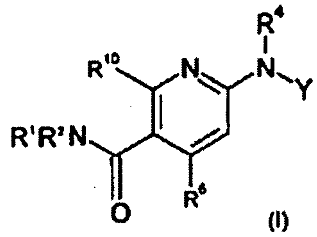

(I)

dans laquelle :

Y représente un groupe phényle substitué avec un, deux ou trois substituants ;

$R^1$ est choisi entre un atome d'hydrogène, des groupes alkyle en $C_1$ à $C_6$, cycloalkyle en $C_3$ à $C_7$ et alkyle en $C_1$ à $C_6$ halogénosubstitué ;

$R^2$ représente un groupe $(CH_2)_mR^3$ ;

$R^3$ représente un groupe hétérocyclyle penta- ou hexagonal aromatique, non substitué ou substitué, ou un groupe A :

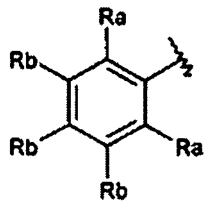

(A)

$R^4$ est choisi entre un atome d'hydrogène, des groupes alkyle en $C_1$ à $C_6$, cycloalkyle en $C_3$ à $C_7$ et alkyle en $C_1$ à $C_6$ halogénosubstitué, $COCH_3$ et $SO_2Me$ ;

$R^6$ représente un groupe alkyle en $C_1$ à $C_6$ non substitué ou substitué ou un groupe chloro et $R^{10}$ représente un atome d'hydrogène ou bien $R^{10}$ représente un groupe alkyle en $C_1$ à $C_6$ non substitué ou substitué ou un groupe chloro et $R^6$ représente un atome d'hydrogène ;

Ra peut être choisi indépendamment entre un atome d'hydrogène, des groupes fluoro, chloro et trifluorométhyle ;.

Rb peut être choisi indépendamment entre un atome d'hydrogène, des groupes alkyle en $C_1$ à $C_6$, alkoxy en $C_1$ à $C_6$, alkoxy en $C_1$ à $C_6$ halogénosubstitué, hydroxy, cyano, halogéno, sulfonyle, $CONH_2$, COOH, $SO_2CH_3$, $NHCOCH_3$, $NHSO_2CH_3$ et $CONHCH_3$ ;

m est égal à 1 ou 2 ;

ou un de ses dérivés pharmaceutiquement acceptables.

**2.** Composé suivant la revendication 1, dans lequel le composé répond à la formule (Ia) :

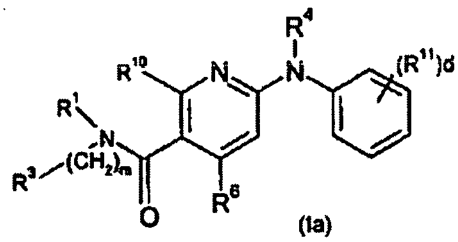

(Ia)

dans laquelle

$R^1$ est choisi entre un atome d'hydrogène, des groupes alkyle en $C_1$ à $C_6$, cycloalkyle en $C_3$ à $C_7$ ou alkyle en

$C_1$ à $C_6$ halogénosubstitué ;
$R^3$ représente un groupe furannyle, dioxalannyle, pyrrolyle, oxazolyle, thiazolyle, imidazolyle, oxadiazolyle, thiadiazolyle, triazolyle, triazinyle, isothiazolyle, isoxazolyle, thiényle, pyrazolyle, tétrazolyle, pyridyle, pyrizinyle, pyrimidinyle, pyrazinyle, triazinyle ou tétrazinyle qui peut être non substitué ou substitué avec 1, 2 ou 3 substituants choisis entre des substituants alkyle en $C_1$ à $C_6$, alkoxy en $C_1$ à $C_6$, alkoxy en $C_1$ à $C_6$ halogénosubstitué, alkyle en $C_1$ à $C_6$ halogénosubstitué, hydroxy, cyano, halogéno, sulfonyle, $CONH_2$ ou COOH, ou bien $R^3$ représente un groupe A :

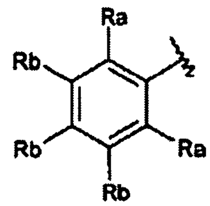

(A)

$R^4$ est choisi entre un atome d'hydrogène, des groupes alkyle en $C_1$ à $C_6$, cycloalkyle en $C_3$ à $C_7$ ou alkyle en $C_1$ à $C_6$ halogénosubstitué, $COCH_3$ ou $SO_2Me$ ;
$R^6$ représente un groupe alkyle en $C_1$ à $C_6$ non substitué ou substitué ou un groupe chloro et $R^{10}$ représente un atome d'hydrogène, ou bien $R^{10}$ représente un groupe alkyle en $C_1$ à $C_6$ non substitué ou substitué ou un groupe chloro et $R^6$ représente un atome d'hydrogène ;
Ra peut être choisi indépendamment entre un atome d'hydrogène, des groupes fluoro, chloro ou trifluorométhyle ;
Rb peut être choisi indépendamment entre un atome d'hydrogène, des groupes alkyle en $C_1$ à $C_6$, alkoxy en $C_1$ à $C_6$, alkoxy en $C_1$ à $C_6$ halogénosubstitué, hydroxy, cyano, halogéno, sulfonyle, $CONH_2$, COOH, $SO_2CH_3$, $NHCOCH_3$, $NHSO_2CH_3$ ou $CONHCH_3$
$R^{11}$ représente un groupe alkyle en $C_1$ à $C_6$, alkyle en $C_1$ à $C_6$ halogénosubstitué, alkoxy en $C_1$ à $C_6$, hydroxy, cyano, halogéno, alkylsulfonyle en $C_1$ à $C_6$, $CONH_2$, $NHCOCH_3$, COOH, alkoxy en $C_1$ à $C_6$ halogénosubstitué, alcynyle en $C_1$ à $C_6$, alcynyle en $C_1$ à $C_6$, $SO_2NR^{8a}R^{8b}$ ;
d est égal à 1, 2 ou 3 ;
m est égal à 1 ou 2 ;
$R^{8a}$ et $R^{8b}$ sont choisis indépendamment entre un atome d'hydrogène ou un groupe alkyle en $C_1$ à $C_6$ ; ou un de ses dérivés pharmaceutiquement acceptables.

**3.** Composé suivant la revendication 1 ou 2, dans lequel $R^1$ représente un atome d'hydrogène ou un groupe alkyle en $C_1$ à $C_6$.

**4.** Composé suivant l'une quelconque des revendications 1 à 3, dans lequel $R^4$ représente un atome d'hydrogène ou un groupe méthyle.

**5.** Composé suivant l'une quelconque des revendications précédentes, dans lequel $R^3$ est choisi entre des groupes A, pyridinyle, pyrimidinyle, imidazoyle, oxadiazoyle, triazolyle ou pyrazinyle.

**6.** Composé suivant l'une quelconque des revendications précédentes, choisi entre le (pyridine-4-ylméthyl)amide d'acide 2-(3-chlorophénylamino)-4-trifluorométhylpyridine-5-carboxylique,
le benzylamide d'acide 2-(3-chlorophénylamino)-4-trifluorométhylpyridine-5-carboxylique,
le 6-(3-chlorophénylamino) -N- (4-fluorobenzyl)-4-isopropyl-nicotinamide,
le N-benzyl-6- (3-chloro-phénylamino) -4-isopropyl-nicotinamide,
le 6- (3-chloro-phénylamino) -N- (4-cyano-benzyl) -4-isopropyl-nicotinamide,
le 6-(3-chloro-phénylamino)-4-isopropyl-N-(4-méthoxy-benzyl)-nicotinamide,
le 6-(3-chloro-phénylamino)-N-(3,4-difluoro-benzyl)-4-isopropyl-nicotinamide,
le N-(4-carbamoyl-benzyl)-6-(3-chloro-phénylamino)-4-isopropyl-nicotinamide,
le 6-(3-chloro-phénylamino)-N-(2,4-difluoro-benzyl)-4-isopropyl-nicotinamide,
le 6-(3-chloro-phénylamino)-4-isopropyl-N-(4-méthanesulfonyl-benzyl)-nicotinamide,
le N-(4-acétylamino-benzyl)-6-(3-chloro-phénylamino)-4-isopropyl-nicotinamide,

le 6-(3-chloro-phénylamino)-4-isopropyl-N-(4-méthane-sulfonylamino-benzyl)-nicotinamide,
le 6-(3-chloro-phénylamino)-4-isopropyl-N-(4-méthylcarbamoyl-benzyl)-nicotinamide,
le N- (4-fluoro-benzyl) -4-isopropyl-6-(3-trifluorométhyl-phénylamino)-nicotinamide,
le 6- (3-chloro-4-fluoro-phénylamino) -N- (4-fluoro-benzyl) -4-isopropyl-nicotinamide,
le 6- (2-cyano-3-méthyl-phénylamino) -N- (4-fluoro-benzyl) -4-isopropyl-nicotinamide,
le N- (4-fluoro-benzyl) -6- (3-fluoro-phénylamino) -4-isopropyl-nicotinamide,
le 6- (4-cyano-phénylamino) -N- (4-fluoro-benzyl) -4-isopropyl-nicotinamide,
le 6- (3-cyano-phénylamino)-N-(4-fluoro-benzyl)-4-isopropyl-nicotinamide,
le 6- (4-chloro-2-fluoro-phénylamino) -N- (4-fluoro-benzyl)-4-isopropyl-nicotinamide,
le 6- (4-bromo-2-chloro-phénylamino) -N- (4-fluoro-benzyl) -4-isopropyl-nicotinamide,
le 6-(2,4-dichloro-phénylamino)-N-(4-fluoro-benzyl)-4-isopropyl-nicotinamide,
le 6- (3-chloro-4-cyano-phénylamino) -N- (4-fluoro-benzyl) -4-isopropyl-nicotinamide,
le 6- (4-bromo-3-fluoro-phénylamino) -N- (4-fluoro-benzyl) -4-isopropyl-nicotinamide,
le N- (4-fluoro-benzyl) -4-isopropyl-6- (3-trifluorométhoxy-phénylamino)-nicotinamide,
le 6- (3-chloro-2-fluoro-phénylamino) -N- (4-fluoro-benzyl) -4-isopropyl-nicotinamide,
le 6- (3-bromo-2-méthyl-phénylamino)-N-(4-fluoro-benzyl)-4-isopropyl-nicotinamide,
le 6- (3-chloro-2-méthyl-phénylamino) -N- (4-fluoro-benzyl) -4-isopropyl-nicotinamide,
le N- (4-fluoro-benzyl) -4-isopropyl-6-m-tolylamino-nicotinamide,
le N-(4-fluoro-benzyl)-4-isopropyl-6-(3-méthoxy-phénylamino)-nicotinamide,
le 6- (4-bromo-2-fluoro-phénylamino) -N- (4-fluoro-benzyl) -4-isopropyl-nicotinamide,
le 6-(3,4-dichloro-phénylamino)-N-(4-fluoro-benzyl)-4-isopropyl-nicotinamide,
le N- (4-fluoro-benzyl) -4-isopropyl-6- (2-méthyl-3-trifluorométhyl-phénylamino)-nicotinamide,
le N-(4-fluoro-benzyl)-6-(2-fluoro-3-trifluorométhyl-phénylamino)-4-isopropyl-nicotinamide,
le 6-(2,3-dichloro-phénylamino)-N-(4-fluoro-benzyl)-4-isopropyl-nicotinamide,
le N-(4-fluoro-benzyl)-6-(3-fluoro-2-méthyl-phénylamino)-4-isopropyl-nicotinamide,
le 6- (2-bromo-3-méthyl-phénylamino) -N- (4-fluoro-benzyl) -4-isopropyl-nicotinamide,
le 6- (3 -bromo-phénylamino) -N- (4-fluoro-benzyl) -4-isopropyl-nicotinamide,
le 6- (3-chloro-2-cyano-phénylamino)-N-(4-fluoro-benzyl)-4-isopropyl-nicotinamide,
le N-(4-fluoro-benzyl)-6-(4-fluoro-3-trifluorométhyl-phénylamino)-4-isopropyl-nicotinamide,
le 6-(3,4-dibromo-phénylamino)-N-(4-fluoro-benzyl)-4-isopropyl-nicotinamide,
le 6- (3-chloro-4-méthyl-phénylamino) -N- (4-fluoro-benzyl) -4-isopropyl-nicotinamide,
le 6- (3-chloro-phénylamino) -N- (1H-imidazole-2-ylméthyl) -4-trifluorométhyl-nicotinamide,
le N- (4-fluoro-benzyl) -6- (3-chloro-phénylamino) -4-trifluorométhyl-nicotinamide,
le N- (2-fluoro-pyridine-4 -ylméthyl) -6- (3-chloro-phénylamino)-4-trifluorométhyl-nicotinamide,
le N- (4-fluoro-benzyl)-6-(3-bromo-phénylamino) -4-trifluorométhyl-nicotinamide,
le N-(2-fluoro-pyridine-4-ylméthyl)-6-(3-bromo-phénylamino)-4-trifluorométhyl-nicotinamide,
le N-(2-fluoro-pyridine-4-ylméthyl)-6-(3-fluoro-phénylamino)-4-trifluorométhyl-nicotinamide,
le N-(4-fluoro-benzyl) -6- (3-fluoro-phénylamino)-4-trifluorométhyl-nicotinamide,
le N-(2-fluoro-pyridine-4-ylméthyl)-6-(2,3-dichloro-phénylamino)-4-trifluorométhyl-nicotinamide,
le N- (2-fluoro-pyridine-4-ylméthyl)-6-(2,4-dichloro-phénylamino)-4-trifluorométhyl-nicotinamide,
le N-(4-fluoro-benzyl)-6-(2,4-dichloro-phénylamino)-4-trifluorométhyl-nicotinamide,
le N-(4-fluoro-benzyl)-6-(3,5-dichloro-phénylamino)-4-trifluorométhyl-nicotinamide,
le N-(2-fluoro-pyridine-4-ylméthyl)-6-(3,5-dichloro-phénylamino)-4-trifluorométhyl-nicotinamide,
le N-(4-fluoro-benzyl)-6-(3,4-dichloro-phénylamino)-4-trifluorométhyl-nicotinamide,
le N-(2-fluoro-pyridine-4-ylméthyl)-6-(3,4-dichloro-phénylamino)-4-trifluorométhyl-nicotinamide,
le N- (pyridine-4-ylméthyl) -6- (2-méthyl-5-chloro-phénylamino)-4-trifluorométhyl-nicotinamide,
le N- (pyridine-4-ylméthyl) -6- (2-méthyl-4-chloro-phénylamino)-4-trifluorométhyl-nicotinamide,
le N-phénéthyl-6- (3-chloro-phénylamino) -4-trifluorométhyl-nicotinamide,
le N-(5-méthyl-[1,3,4]oxadiazole-2-ylméthyl)-6-(3-chloro-phénylamino)-4-trifluorométhyl-nicotinamide,
le N-phénéthyl-6-(3-bromo-phénylamino)-4-trifluoron-éthyl-nicotinamide,
le N-phénéthyl-6- (3-fluoro-phénylamino)-4-trifluorométhyl-nicotinamide,
le N-(5-méthyl-[1,3,4]oxadiazole-2-ylméthyl)-6-(2,4-dichloro-phénylamino)-4-trifluorométhyl-nicotinamide,
le N-(5-méthyl-4H-[1,2,4]triazole-3-ylméthyl)-6-(2,4-dichloro-phénylamino)-4-trifluorométhyl-nicotinamide,
le N-phénéthyl-6-(3,4-dichloro-phénylamino)-4-trifluorométhyl-nicotinamide,
le N-phénéthyl-6-(3,5-dichloro-phénylamino)-4-trifluorométhyl-nicotinamide,
le 6-(3-chloro-phénylamino)-4-isopropyl-N-pyrimidine-4-ylméthyl-nicotinamide,
le 6- (3-chloro-phénylamino)-4-isopropyl-N-pyrazine-2-ylméthyl-nicotinamide,
le 6- (3-chloro-phénylamino)-4-isopropyl-N-(6-méthyl-pyridine-3-ylméthyl)-nicotinamide,

le 6- (2-bromo-4-chloro-phénylamino) -N- (4-fluoro-benzyl) -4-isopropyl-nicotinamide,
le 6- (2-chloro-4-fluoro-phénylamino) -N-(4-fluoro-benzyl)-4-isopropyl-nicotinamide,
le 6- (5-chloro-2-fluoro-phénylamino) -N- (4-fluoro-benzyl) -4-isopropyl-nicotinamide,
le 6- (4-cyano-2-méthyl-phénylamino)-N-(4-fluoro-benzyl)-4-isopropyl-nicotinamide,
le 6-(2,5-dichloro-phénylamino)-N-(4-fluoro-benzyl)-4-isopropyl-nicotinamide,
le 6- (4-bromo-3-chloro-phénylamino)-N-(4-fluoro-benzyl)-4-isopropyl-nicotinamide,
le N-(4-fluoro-benzyl)-6-(3-fluoro-4-trifluorométhyl-phénylamino)-4-isopropyl-nicotinamide,
le N- (4-fluoro-benzyl)-4-isopropyl-6-(2-méthyl-5-trifluorométhyl-phénylamino)-nicotinamide,
le 6-(3-chloro-phénylamino)-N-(pyridine-4-ylméthyl)-2-trifluorométhyl-nicotinamide, et
le 6-(3-chloro-phénylamino)-N-benzyl-2-trifluorométhyl-nicotinamide ;
ou un de ses dérivés pharmaceutiquement acceptables.

**7.** Composition pharmaceutique comprenant un composé suivant l'une quelconque des revendications 1 à 6 ou un de ses dérivés pharmaceutiquement acceptables.

**8.** Composition pharmaceutique suivant la revendication 7, comprenant en outre un support ou diluant pharmaceutique.

**9.** Utilisation d'une quantité thérapeutiquement efficace d'un composé de formule (I) suivant l'une quelconque des revendications 1 à 6 ou d'un de ses dérivés pharmaceutiquement acceptables, pour la production d'un médicament destiné au traitement d'un trouble immunitaire, d'un trouble inflammatoire, de la douleur, de la polyarthrite rhumatoïde, de la sclérose en plaques, de l'arthrose ou de l'ostéoporose.

**10.** Utilisation suivant la revendication 9, dans laquelle la douleur est choisie entre la douleur inflammatoire, la douleur viscérale, la douleur provoquée par le cancer, la douleur neuropathique, la douleur dans le bas du dos, la douleur squelettique, la douleur postopératoire, la douleur aiguë et la migraine.

**11.** Composition pharmaceutique comprenant un composé de formule (I) ou un de ses dérivés pharmaceutiquement acceptables, dans laquelle le composé de formule (I) ou un de ses dérivés pharmaceutiquement acceptables est présent dans des particules solides sous forme de nanoparticules, en mélange avec un ou plusieurs supports ou excipients pharmaceutiquement acceptables.

**12.** Composition pharmaceutique suivant la revendication 11, qui est une poudre séchée pouvant être obtenue en broyant par voie humide des particules solides du composé de formule (I), puis en séchant par pulvérisation la suspension résultante.

**13.** Composition pharmaceutique comprenant un composé de formule (I) ou un de ses dérivés pharmaceutiquement acceptables conjointement avec un ou plusieurs agents thérapeutiques supplémentaires.